(19) Europäisches Patentamt
European Patent Office
Office européen des brevets

(11) EP 0 401 790 B1

(12) **EUROPÄISCHE PATENTSCHRIFT**

(45) Veröffentlichungstag und Bekanntmachung des Hinweises auf die Patenterteilung:
**22.04.1998 Patentblatt 1998/17**

(51) Int. Cl.$^6$: **B01F 17/00**, C09C 3/00, D21H 17/67, D21H 19/44

(21) Anmeldenummer: **90110703.7**

(22) Anmeldetag: **06.06.1990**

(54) **Hochkonzentrierte wässrige Suspension aus Mineralien und/oder Füllstoffen und/oder Pigmenten**

Highly concentrated aqueous slurry of minerals and/or fillers and/or pigments

Suspension aqueuse très concentrée de minéraux et/ou charges et/ou pigments

(84) Benannte Vertragsstaaten:
**AT BE CH DE DK ES FR GB GR IT LI LU NL SE**

(30) Priorität: **06.06.1989 DE 3918461**
**19.12.1989 DE 3941936**

(43) Veröffentlichungstag der Anmeldung:
**12.12.1990 Patentblatt 1990/50**

(73) Patentinhaber: **Plüss-Staufer AG**
**4665 Oftringen (CH)**

(72) Erfinder:
- **Buri, Matthias**
**CH-4852 Rothrist (CH)**
- **Frey, Daniel**
**CH-4663 Aarburg (CH)**

(74) Vertreter:
**Reinhard - Skuhra - Weise & Partner**
**Postfach 44 01 51**
**80750 München (DE)**

(56) Entgegenhaltungen:
**EP-A- 0 256 312** **EP-A- 0 307 795**
**US-A- 4 533 434**

- **PATENT ABSTRACTS OF JAPAN, unexamined applications, C. Sektion, Band 6, Nr. 122, 7. Juli 1982 THE PATENT OFFICE JAPANESE GOVERNMENT Seite 32 C 112**

EP 0 401 790 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.1/3.4

## Beschreibung

Die vorliegende Erfindung betrifft eine wäßrige Suspension aus Mineralien und/oder Füllstoffen und/oder Pigmenten mit einem Feststoffgehalt ≥ 60 Gew.%, bezogen auf das trockene Mineral bzw. den trockenen Füllstoff bzw. das trockene Pigment, wobei das Mineral bzw. der Füllstoff bzw. das Pigment mit einem oder mehreren Dispergiermitteln dispergiert ist.

Unter "positive Ladungen" ist nachfolgend zu verstehen, daß die Partikeln auf ihrer Oberfläche ein positives Zeta-Potential aufweisen (vgl. P. Ney "Zeta-Potentiale und Flotierbarkeit von Mineralen", Applied Minerology 6, Springer Verlag, Wien, New York 1973, insbesondere Seite 22 ff.). Analoges gilt für die "negativen Ladungen", wie sie z. B. bei der Zellulosefaser und anionisch stabilisierten Suspensionen auftreten. Für die neutralen "Ladungen", bezogen auf die Teilchen, gilt, daß sich gegen außen die negativen und positiven Ladungen gegenseitig aufheben. Der isoelektrische Punkt muß nicht bei pH = 7 liegen. Der isoelektrische Punkt von Teilchenoberflächen und amphoteren Polyelektrolyten und/oder deren Salzen, Teilsalzen und/oder Vollsalzen liegt bei dem pH-Wert, bei welchem sich die positiven und negativen Ladungen gegen außen gegenseitig neutralisieren.

Unter neutralen Monomereinheiten sind im Rahmen der Erfindung Monomereinheiten zu verstehen, die keine dissoziierbaren Gruppen (wie z.B. die -COOH Gruppe) enthalten, z. B. Ethylengruppen.

Die nach außen geladenen und nach außen neutralen Polyelektrolyten der Erfindung werden bei der vorstehenden Anmeldung durch die Anzahl der positiven bzw. negativen Gruppen im Polymer definiert. Bei den amphoteren gegen außen neutralen Polyelektrolyten ist demnach die Anzahl positiven Ladungen in den kationischen Monomereinheiten gleich der Anzahl der negativen Ladungen in den anionischen Monomereinheiten. Bei den amphoteren kationischen Polyelektrolyten tragen die nicht neutralen Monomereinheiten überwiegend positive Ladungen. Bei den amphoteren anionischen Polyelektrolyten tragen die nicht neutralen Monomereinheiten überwiegend negative Ladungen.

Dies bedeutet jedoch nicht, daß beispielsweise bei einem Überschuß an positiven Ladungen der Polyelektrolyt automatisch elektrisch positiv ist. Dies deshalb, weil die "Säurestärke" und die "Basenstärke" je unterschiedlich sein können. So kann z.B. ein amphoterer Polyelektrolyt mit gleich vielen positiven und negativen Gruppen elektrisch entweder positiv oder negativ oder neutral sein. Dies gilt entsprechend auch für die amphoteren kationischen Polyelektrolyten und die amphoteren anionischen Polyelektrolyten. Durch Verschiebung des pH-Werts ist die Dissoziation der "Säure- respektive Basegruppen" beeinflußbar. Insbesondere bei pH-Werten zwischen 5 und 10 können die erfindungsgemäßen Polyelektrolyten folgende Ladungszustände nach außen aufweisen :

| pH 5-10 | amphoterer Polyelektr. | kationischer Polyelektr. | amph.leicht kati.Polyele. | amph.leicht anio.Polyel |
|---|---|---|---|---|
| A | + neutral - | + | + neutral - | + neutral - |
| B | + = - | + | + überwiegt etwas - | -überwiegt etwas + |
| C | + oder neutral | + | + | + oder neutral |

A = Möglichkeit der elektrischen Ladung nach außen
B = Anzahl der geladenen Monomereinheiten
C = Ladung der Teilchen

Auch die Neutralisation der negativen Gruppen mit ein- und/oder zwei- und/oder dreiwertigen Kationen beeinflußt deren Dissoziationsgrad und somit den Ladungszustand nach außen.

Anionisch stabilisierte, calciumhaltige Mineralien wie Calciumcarbonat, Dolomit etc. werden üblicherweise durch Mahlen mit anionischen Polyacrylaten, wie z.B. in den Patenten EP 0 100 947 oder FR 820806 beschrieben, hergestellt. Bei diesem Patent wird offenbart, daß bei anionisch stabilisierten Suspensionen teilneutralisierte Polyacrylsäuren bessere Viskositätsstabilitäten ergeben als vollneutralisierte. Der offenbarte Bereich der Neutralisation liegt zwischen 40 und 96 % Neutralisation, dies führt in der erfindungsgemäßen kationischen Suspension nicht zu befriedigenden Resultaten.

Aus den offenbarten Beispielen in FR 820 806 geht hervor, daß eine Neutralisation von < 50 % nicht zum Ziel führt, sondern daß 60 - 70 % Neutralisationsgrad das Optimum darstellt. Die Mineralien können auch, wie in der EP 0 256 312 beschrieben, mit amphoteren Dispergiermitteln in Suspension gebracht werden. Bei den in dieser Vorveröffentlichung offenbarten amphoteren Polyelektrolyten liegt der isoelektrische Punkt stark im sauren pH-Bereich, so daß sie für die erfindungsgemäßen Pigment- und/oder Füllstoff- und/oder Mineralsuspensionen nicht geeignet sind. Zudem sind nur amphotere Polyelektrolyten erwähnt, welche in ihrer molaren Monomerzusammensetzung überwiegend anionische Monomere enthalten. Die Partikeln gemäß diesem Stand der Technik weisen eine negative Ladung auf ihrer

Oberfläche auf.

Für manche Anwendungen ist die anionische Stabilisierung jedoch nicht erwünscht. Es wäre vielmehr sinnvoll, eine Aufschlämmung mit neutral oder positiv geladenen Partikeln einzusetzen. Wenn Calciumcarbonat, beschichtet mit dem anionischen Dispergiermittel, als Füllstoff in der Papierindustrie eingesetzt wird, ist es notwendig, den negativ geladenen Füllstoff mit kationischen Retentionsmitteln an die, durch Carboxylgruppen von Natur her negativ geladene, Papierfaser zu binden.

Bei der Neutralisierung und Flockung der negativ geladenen Mineral- und/oder Füllstoff- und/oder Pigmentteilchen mit dem Ziel, möglichst hohe Füllgrade und gute Füllstoffretentionen im Papier zu erzielen, kann zugleich auch die negativ geladene Papierfaser mit geflockt werden, was zu einer schlechteren Papierformation und dadurch zu einer unregelmäßigeren Durchsicht des Papiers führen kann. Bei dem heutigen Stand der Technik ist dieser negative Effekt kaum zu umgehen. Daher werden heute noch mehrheitlich trockengemahlene, pulverförmige Produkte in der Papiererzeugung eingesetzt, die nur schwach negative oder gegen außen neutrale oder schwach positive Oberflächenladungen aufweisen.

Bei den trocken gemahlenen Produkten sind jedoch die notwendigen Feinheiten nur sehr schwer herstellbar. Zudem ergeben Pulver das Problem der Staubentwicklung.

Durch Dispergieren hergestellte kationisch stabilisierte Mineral-und/oder Füllstoff- und/oder Pigment-Suspensionen:

Kationisch stabilisierte, d.h. auf der Oberfläche positiv geladene, partiell calciumhaltige Minerale wie Calciumcarbonat, Dolomit usw. werden üblicherweise durch Dispergieren in Wasser mit neutralen und/oder kationischen Schutzkolloiden und/oder kationischen Dispergiermitteln (vgl. die Offenlegungsschriften DE 37 07 221 und DE 37 30 833) oder durch Dispergieren mit einer Kombination aus einem voll neutralisierten anionischen und einem kationischen Dispergiermittel, wie im Europapatent 0 278 602 A 1 beschrieben, hergestellt, wobei beim zuletztgenannten soviel kationisches Polymer verwendet wird, daß die Teilchen in der Suspension eine positive Ladung besitzen.

In der EP 0 278 602 wird ebenfalls Polyacrylsäure offenbart. Reine, nicht neutralisierte Polyacrylsäure ist ungeeignet, da sie bei + 20°C bereits zu kristallisieren beginnt und somit nicht mehr dosierbar ist.
Wenn Kristallisation einmal eingetreten ist, muß die Polymerlösung auf 100°C aufgeheizt werden, damit die Kristalle wieder aufgelöst werden.
Im Winter und in kälteren Regionen ist eine Produktion mit nicht neutralisierten Polyacrylsäuren undenkbar.

Diese Verfahren haben den Nachteil, daß der Zerkleinerungsprozeß, also das Mahlen, und das Dispergieren in getrennten Schritten vorgenommen werden müssen. Nach dem Stand der Technik bestehen folgende Möglichkeiten:

a) - das calciumhaltige Gestein wird auf trockenem Wege zerkleinert, um auf die notwendige Feinheit zu gelangen. Die Feinheit, die auf diesem Wege erreicht werden kann, ist limitiert. Reagglomeration durch van-der-Waals-Kräfte verhindern weitgehend ein Aufmahlen auf hohe Feinheiten. In einem separaten Schritt wird anschließend mit den vorstehend genannnten Dispergiermitteln dispergiert.

b) - das calciumhaltige Gestein wird auf nassem Wege bei tiefem Feststoffgehalt (ca. 30 Gew.%) ohne Mahl- und Dispergiermittel gemahlen und muß über Filterpressen, durch Zusatz von Flockungsmitteln oder über Zentrifugen auf die gewünschte Konzentration gebracht werden. In einem separaten Schritt wird anschließend mit den vorstehend genannten Dispergiermitteln dispergiert.

c) - das calciumhaltige Mineral wird auf nassem Wege mit anionischen Dispergiermitteln auf die gewünschte Feinheit gemahlen, getrocknet und anschließend mit den vorstehend genannten kationischen Polyelektrolyten und/oder Schutzkolloiden wieder redispergiert. Bei der Trocknung entstehen Aggregate, die sich nicht wieder voll desaggregieren lassen, d.h. es resultiert eine geringere Feinheit als ursprünglich. Das beim Trocknen nicht zerstörte anionische Dispergiermittel kann zudem den anschließenden Dispergierprozeß stören und einen Mehrverbrauch an kationischem Polyelektrolyt bewirken.

Die Viskositätsstabilität über längere Zeit ist bei den genannten Herstellungsverfahren a - c nicht gegeben.

Die Herstellung der Mineral- und/oder Füllstoff- und/oder Pigment-Suspension muß dadurch zwangsläufig beim Verbraucher oder in der näheren Umgebung des Verbrauchers stattfinden und verdirbt in kurzer Zeit durch starken Viskositätsanstieg oder Sedimentation. Eine Viskositätssenkung durch Verdünnen ist in vielen Fällen nicht möglich, da die hohe Konzentration für die Weiterverarbeitung, z.B. in Streichfarben in der Papierindustrie, von ausschlaggebender Bedeutung ist.

Durch Mahlen erzeugte kationisch stabilisierte Mineral- und/oder Füllstoff- und/oder Pigment-Suspensionen:

In jüngster Zeit sind Bemühungen im Gange, kationisch stabilisierte, partiell calciumhaltige Füllstoffe durch Mahlen bei tiefen Feststoffgehalten herzustellen, wie dies im Vortrag von Loreen Goodwin, Columbia River Carbonates, gehalten am TAPPI Papermaker, April 89 in Washington DC., erläutert wurde.

Dieses Verfahren hat den Nachteil, daß der Feststoffgehalt auf 45 - 50 Gew.% limitiert ist. Bei höheren Konzentration sind die Viskositäten derart hoch, daß die Suspensionen nicht mehr verarbeitbar sind.

Die Viskositätsstabilität über längere Zeit ist nicht gegeben.

Bedingt durch den tiefen Feststoffgehalt neigt die Suspension stark zum Absetzen, und sie ist dadurch nicht lagerstabil. Die Transportkosten, bezogen auf das Trockenprodukt, sind bei 45 Gew.%-igen Suspensionen ca. 50 % teurer als bei 70 Gew.%-igen Suspensionen. Zudem sind beim Produzenten und beim Verbraucher je ca. 50% mehr Lagerkapazität notwendig.

In der EP 0 104 904 wird eine wäßrige Aufschlämmung mineralischer Partikel mit einem Feststoffgehalt von wenigstens 40 Gew.% beschrieben. Diese Aufschlämmung enhält kationische und amphotere Polyelektrolyte mit Stickstoffenthaltenden Gruppen, wobei aus der hier gemachten Offenbarung für den Durchschnittsfachmann nicht hervorgeht, was "amphoterer Polyelektrolyt" bedeutet. Eher wird man durch die einzige erwähnte amphotere Verbindung irregeführt, da diese keinen ersichtlichen amphoteren Charakter aufweist. Sowohl DMDAAC (Dimethyldiallylammoniumchlorid) wie Acrylamid, welche bei dem als amphoter bezeichneten Copolymer verwendet wurden, sind von ihrer Struktur her ausschließlich kationisch.

Bei den wäßrigen Aufschlämmungen wird eine Sedimentation der dispergierten Mineralpartikel über 3 - 7 Tage in Kauf genommen, was bei einem Schiffstransport von z.B. Skandinavien nach England über 4 - 7 Tage undenkbar ist, und ein Entleeren großer Schiffe, wie sie heute eingesetzt werden für Transporte dieser Art unmöglich machen würde.

Ein Rühren so großer Schiffsladungen ist praktisch ausgeschlossen. Auch ein Bahntransport von 4 - 7 Tagen im 56 t Kesselwagen von Österreich nach Norddeutschland ist aus denselben Gründen ausgeschlossen.
Sowohl Bahn- wie Schiffstransporte sind aus ökologischen Überlegungen heute sehr sinnvoll.

Folgende Anforderungen (Eigenschaften) an eine Suspension sind vom Verbraucher her gesehen wünschenswert:

- gute Lagerstabilität über Wochen bei tiefen Viskositäten
- Um die notwendigen Eigenschaften, wie z. B. eine geringe Abrasion der Papiermaschinensiebe bei der Papierherstellung und der Streichrakel in der Streichlage zu erhalten, ist es notwendig, sehr feinteilige Füllstoffe zu erzeugen. Ebenfalls neigen grobe Füllstoffe in der Papiermasse zum Stauben in Photokopierern etc.
- Papieropazität, Papierglanz und Papierweiße hängen stark von der Feinheit und den Füllgraden der Füllstoffe im und auf dem Papier ab. Opazität und Weiße sind heute für die Papierindustrie von ausschlaggebender Bedeutung.
- Für die Papiermasse sind heute üblicherweise Mineralien und/oder Füllstoffe und/oder Pigmente mit einem äquivalent sphärischen Durchmesser der Teilchen von 50 - 90 Gew.% < 2 µm (gemessen mit dem Sedigraph 5100) notwendig.
- Für Streichrezepturen werden heute üblicherweise Mineralien und/oder Füllstoffe und/oder Pigmente mit einem äquivalent sphärischen Durchmesser der Teilchen bis 99 Gew.% < 2 µm (gemessen auf dem Sedigraph 5100) eingesetzt.
- Die Viskositätsstabilät über einige Wochen muß garantiert sein, damit beim Transport und bei der Lagerung kein Verderb der Suspension durch Sedimentation oder Viskositätserhöhung eintritt und keine unnötig hohen Rührkosten entstehen. Um die Produktionssicherheit in der Papierindustrie heute zu gewährleisten, sind Lagerkapazitäten von tausenden von Kubikmetern solcher Suspensionen notwendig.
- Die Mineral- und/oder Füllstoff- und/oder Pigmentteilchen sollen sich ohne Einsatz hoher Mengen an Retentionshilfsmittel bei der Papierherstellung retendieren lassen. Die Festigkeitswerte des gefertigten Papiers sollen durch hohe Füllgrade an Mineralien und/oder Pigmenten und/oder Füllstoffen nicht stark beeinträchtigt werden.

  Durch hohe Füllgrade kann Zellulose eingespart werden, was einen enormen ökonomischen Vorteil für die Papierindustrie bedeutet.
- Pigment- und/oder Füllstoff- und/oder Mineralstreichfarben sollen bei der Applikation auf das Papier möglichst wenig ins Papier eindringen, sondern auf der Papieroberfläche bleiben und so eine optimale Faserabdeckung bewirken. Ein kationischer Strich auf anionischer Zellulose bleibt wesentlich besser an der Oberfläche.
- Möglichst hohe Feststoffkonzentrationen sollen erreicht werden.

EP-A-0 256 312 offenbart amphotere wasserlösliche Polymere, die

a) 90 bis 30 Gew.-% Acrylsäure und/oder Methacrylsäure
b) 10 bis 60 Gew.-% einer Verbindung der Formel

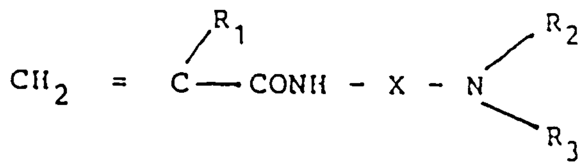

worin $R_1$ Wasserstoff oder den Methylrest, $R_2$ und $R_3$, die gleich oder verschieden sind, den Methyl- oder Ethylrest bedeuten und für X ein gegebenenfalls verzweigter Alkylenrest mit 1 bis 5 C-Atomen steht sowie gegebenenfalls bis 50 Gew.-% Acrylamidomethylpropansulfonsäure und bis 10 Gew.-% einer weiteren ethylenisch ungesättigten Verbindung enthalten, wobei das Molekulargewicht dieser Polymere, gemessen bei einem pH-Wert von 8,0 kleiner als 100 000 ist. Diese Druckschrift offenbart ferner ein Verfahren zur Herstellung dieser Polymeren und ihre Verwendung als Mahlhilfs- bzw. Dispergiermittel zur Herstellung von hochkonzentrierten wässrigen Pigmentaufschlämmungen.

Die EP-A-307 795 offenbart eine kationisch eingestellte Pigmentdispersion, die insbesondere zur Herstellung von Streichfarben zur Papierbeschichtung geeignet ist. Sie enthält

a) ein Calciumsulfat, Talkum und/oder Aluminiumhydroxid als Hauptbestandteil oder Verschnittkomponente enthaltende oder daraus bestehende Pigmentkomponente;
b) ein kationisches Polymer oder eine quaternäre Ammoniumverbindung als Dispergiermittel; und/oder
c) ein die Teilchen der Pigmentkomponente (a) als Schutzkolloid umhüllendes kationisiertes Polymer, das aus hydrophilen Polyacrylaten oder -methacrylaten, abgebauten Stärken oder abgebauten modifizierten Stärken, abgebauten Galaktomannanen oder abgebauten modifizierten Galaktomannanen, Methylcellulosen, Hydroxymethylcellulosen, Carboxymethylcellulosen, abgebauten Alginaten, Proteinen und/oder Polyvinylalkohol erhalten worden ist.

Eine Aufgabe dieser Erfindung ist es, lagerstabile Füllstoffund/oder Mineral- und/oder Pigment-Suspensionen mit hohem Feststoffgehalt bei tiefen Viskositäten zu schaffen.

Diese Aufgabe wird erfindungsgemäß dadurch gelöst, daß eine wäßrige Suspension gemäß den Merkmalen des Patentanspruchs 1 bereitgestellt wird.

Überraschend und nicht voraussehbar war die Tatsache, daß die erfindungsgemäßen amphoteren Polyelektrolyten im Gegensatz zum Stand der Technik, bei welchem die Füllstoff- und/oder Pigment- und/oder Mineralteilchen ebenfalls gegen außen neutrale oder positive Ladungen tragen, eine sehr gute Viskositätsstabilität über lange Zeit bei tiefen Viskositäten ergeben und trotzdem keine Sedimentation der Mineralteilchen eintritt, auch wenn nicht gerührt wird.

Im folgenden werden die amphoteren Polyelektrolyten, bei welchen die Anzahl der negativen Ladungen in den anionischen Monomereinheiten gleich der Anzahl der positiven Ladungen in den kationischen Monomereinheiten ist und die amphoteren kationischen Polyelektrolyten und die amphoteren anionischen Polyelektrolyten kurz als erfindungsgemäße amphotere Polyelektrolyten bezeichnet. Die amphoteren Polyelektrolyte, bei welchen die Anzahl der negativen Ladungen in den anionischen Monomereinheiten gleich der Anzahl der positiven Ladungen in den kationischen Monomereinheiten ist, werden kurz als "amphoter" bezeichnet.

Im Rahmen der Erfindung kann es vorteilhaft sein, wenn einige oder mehrere der amphoteren Polyelektrolyten teilneutralisiert sind.

Vorteilhafterweise trägt der amphotere anionische und der amphotere kationische Polyelektrolyt und der amphotere Polyelektrolyt, bei welchen die Anzahl der negativen Ladungen in den anionischen Monomereinheiten gleich der Anzahl der positiven Ladungen in den kationischen Monomereinheiten ist, die die positive Ladung erzeugende funktionelle Gruppe in einem Substituenten der ethylenischen Hauptkette.

Weiterhin vorteilhaft ist, daß der die kationische Ladung tragende Substituent über

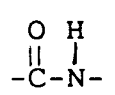

oder

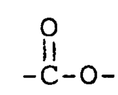

an der Hauptkette gebunden ist. Vorzüglich geeignet ist die erstgenannte Gruppe.
Weiterhin vorteilhaft ist, wenn die erfindungsgemäßen amphoteren Polyelektrolyten quaternäre Ammoniumgruppen, Carboxylgruppen und/oder Sulfonsäuregruppen und/oder saure phosphorsäureesterhaltige Gruppen enthalten.

Vorteilhafterweise sind die erfindungsgemäßen amphoteren Polyelektrolyten eine oder mehrere Verbindungen aus der Gruppe der folgenden Verbindungen gemäß der folgenden allgemeinen Formel

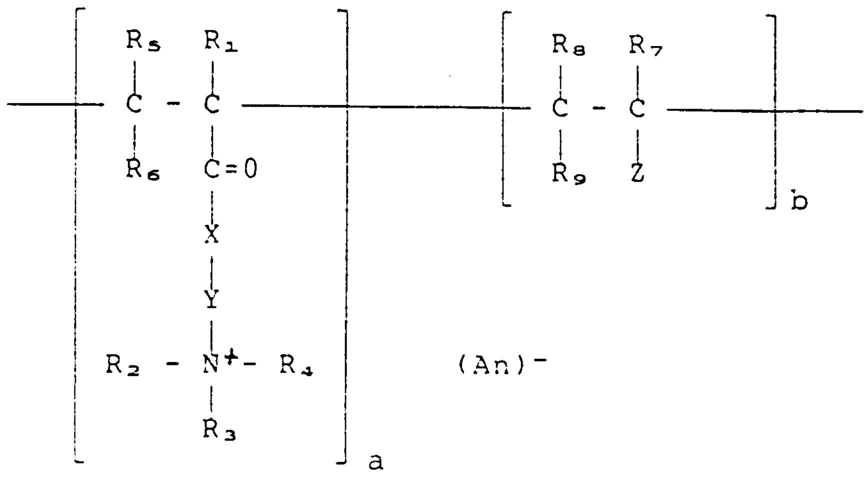

und $(An)^-$ = Chlorid und/oder Bromid und/oder Jodid und/oder $HSO_4^-$ und/oder $CH_3SO_4^-$ und/oder Nitrit sein kann.

wobei $R_1$, $R_5$, $R_6$ und $R_7$ bevorzugt H
und/oder

- $R_1$ bis $R_7$ Alkyl, bevorzugt ein $C_1$ - $C_{18}$-Alkyl, insbeson dere bevorzugt $C_1$ - $C_6$, optimal $-CH_3$ und/oder
- Aryl, bevorzugt ein 6-Ring, insbesondere ein nicht-substituierter 6-Ring
  sein kann,

$R_8$ und $R_9$ =

- H und/oder
- Alkyl, bevorzugt ein $C_1$ - $C_{18}$-Alkyl, insbesondere bevor zugt $C_1$ - $C_6$, optimal $-CH_3$ oder H und/oder
- Aryl, bevorzugt ein 6-Ring, insbesondere ein nicht-substituierter 6-Ring,
  $R_8$ oder $R_9$ auch

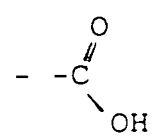

sein kann,
wenn

Z =

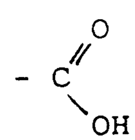

ist,

X = O und/oder N-H
Y = $-CH_2-$ bis $-C_5H_{10}-$

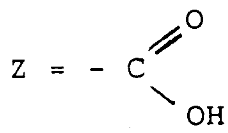

und/oder

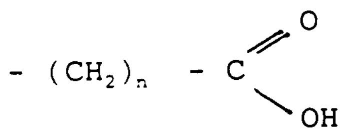

und/oder

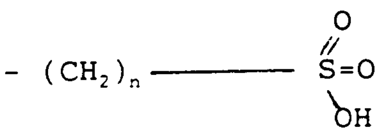

und/oder

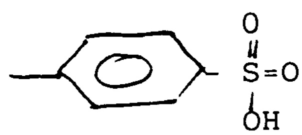

und/oder

eine saure Phosphorsäureester-Gruppe
und n = 1-18 sein kann.

Z kann teilweise neutralisiert sein durch 1-, 2- und/oder 3-wertige Kationen.

Erfindungsgemäß vorteilhaft sind Alkali und/oder Erdalkali-und/oder Erdmetallkationen einsetzbar, wobei Erdalkalikationen bevorzugt sind. Insbesondere bevorzugt sind $Ca^{++}$ und/oder $Mg^{++}$ und/oder $Sr^{++}$, insbesondere bevorzugt $Ca^{++}$ und/oder $Mg^{++}$.

Der Neutralisationsgrad von Z liegt bei 1 bis 99 Mol.%, vorteilhafterweise bei 50 bis 98 Mol.%, bevorzugt bei 70 bis 97 Mol.% und insbesondere bevorzugt bei 95 Mol.%, je bezogen auf Z in b.

Bei der Neutralisation mit einwertigen Kationen wie $K^+$ und/oder $Na^+$ und/oder $Li^+$ beträgt der Neutralisationsgrad von Z 1 bis 99 Mol.%, vorteilhaft 1 bis 50 Mol.%, bevorzugt 1 bis 25 Mol.% und insbesondere bevorzugt < 5 Mol.%, je bezogen auf Z in b.

Z kann auch vollneutralisiert sein, wenn das Kation 2- und/oder 3-wertig ist oder es sich um $NH_4^+$, prim., sec., tert. Amine und/oder quart. Ammoniumionen handelt, wobei $NH_4^+$ zu sehr unangenehmer Geruchsbelästigung führt und gesundheitsschädlich sein kann.

Z kann auch nichtneutralisiert vorliegen.

Wenn $R_8$ oder $R_9$ nicht

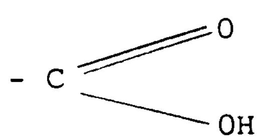

ist, und wenn die amphoteren anionischen Polyelektrolyten in Kombination mit der amphoteren kationischen Polyelektrolyten verwendet werden und die Teilchen dadurch neutral sind oder positive Oberflächenladungen aufweisen, liegen

a und b in den folgenden Verhältnissen vor:

| amphoter anionisch | amphoter | amphoter kationisch |
|---|---|---|
| a = 5-49 Mol.% | a = 50 Mol.% | a = 51-99 Mol.% |
| b = 51-95 Mol.% | b = 50 Mol.% | b = 49- 1 Mol.% |

wobei n = 1 - 18

und $(An)^-$ = Chlorid und/oder Bromid und/oder Jodid und/oder $HSO_4^-$ und/oder $CH_3SO_4^-$ und/oder Nitrit sein kann.

Weiterhin vorteilhaft sind folgende Mischungen :

| amphoter anionisch | amphoter | amphoter kationisch |
|---|---|---|
| a = 47-49 Mol.% | a = 50 Mol.% | a = 51-80 Mol.% |
| b = 51-53 Mol.% | b = 50 Mol.% | b = 49-20 Mol.% |

wobei n = 1 - 18

und $(An)^-$ = Chlorid und/oder Bromid und/oder Jodid und/oder $HSO_4^-$ und/oder $CH_3SO_4^-$ und/oder Nitrit sein kann.

Wenn $R_8$ oder $R_9$ =

$R_9$ =

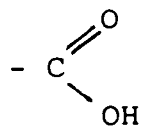

ist, und wenn die amphoteren anionischen Polyelektrolyten in Kombination mit der amphoteren kationischen Polyelektrolyten verwendet werden und die Teilchen dadurch neutral sind oder positive Oberflächenladungen aufweisen, liegen a und b in den folgenden Verhältnissen vor:

| amphoter anionisch | amphoter | amphoter kationisch |
|---|---|---|
| a = 10-66 Mol.% | a = 66,66 Mol.% | a = 67-99 Mol.% |
| b = 34-90 Mol.% | b = 33,33 Mol.% | b = 1-33 Mol.% |

wobei n = 1 - 18

und $(An)^-$ = Chlorid und/oder Bromid und/oder Jodid und/oder $HSO_4^-$ und/oder $CH_3SO_4^-$ und/oder Nitrit sein kann.

Weiterhin vorteilhaft sind folgende Mischungen :

| amphoter anionisch | amphoter | amphoter kationisch |
|---|---|---|
| a = 64-66 Mol.% | a = 66,66 Mol.% | a = 67-90 Mol.% |
| b = 34-36 Mol.% | b = 33,33 Mol.% | b = 10-33 Mol.% |

wobei n = 1 - 18

und $(An)^-$ = Chlorid und/oder Bromid und/oder Jodid und/oder $HSO_4^-$ und/oder $CH_3SO_4^-$ und/oder Nitrit sein kann.

Insbesondere vorteilhaft ist, daß die erfindungsgemäßen amphoteren Polyelektrolyten eine Verbindung gemäß dieser allgemeinen Formel sind, wobei

$R_1$ = H oder $-CH_3$
$R_2 = -CH_3$ oder $-C_2H_5$
$R_3 = -CH_3$ oder $-C_2H_5$
$R_4 = -CH_3$ bis $-C_4H_9$ und Isomere
X = O oder N - H
Y = $-CH_2$-bis - $C_5H_{10}$-
$R_5$ und $R_6$ = H
$R_7$ = H oder $-CH_3$
$R_8$ und $R_9$ = H.

Ganz besonders vorteilhaft ist, wenn $(An)^- = Cl^-$ und
Y = $-(CH_2)_3$- ist.

Gemäß der Erfindung bedeutet amphoter anionisch, daß die anionischen Ladungen im amphoteren Polyelektrolyten gegenüber den kationischen Ladungen überwiegen.

Amphoter kationisch bedeutet, daß die kationischen Ladungen im amphoteren Polyelektrolyten gegenüber den anionischen Ladungen überwiegen.

Amphoter schwach anionisch bzw. kationisch bedeutet erfindungsgemäß, daß die entsprechenden negativen oder positiven Überschußladungen im amphoteren Polyelektrolyten nur gering sind. Amphoter schwach anionisch bedeutet, daß das Verhältnis der anionischen Ladung zur kationischen Ladung im Bereich von 55 : 45 bis 51 : 49 Mol.% liegt.

Amphoter schwach kationisch bedeutet, daß das Verhältnis der anionischen zu den kationischen Ladungen im Bereich von 45 : 55 bis 49 : 51 Mol.% liegt.

Es ist darauf hinzuweisen, daß die Begriffe "schwach" und "leicht" in dieser Anmeldung synonym verwendet werden.

Besonders günstig sind erfindungsgemäß Polyelektrolyten gemäß der folgenden Formel

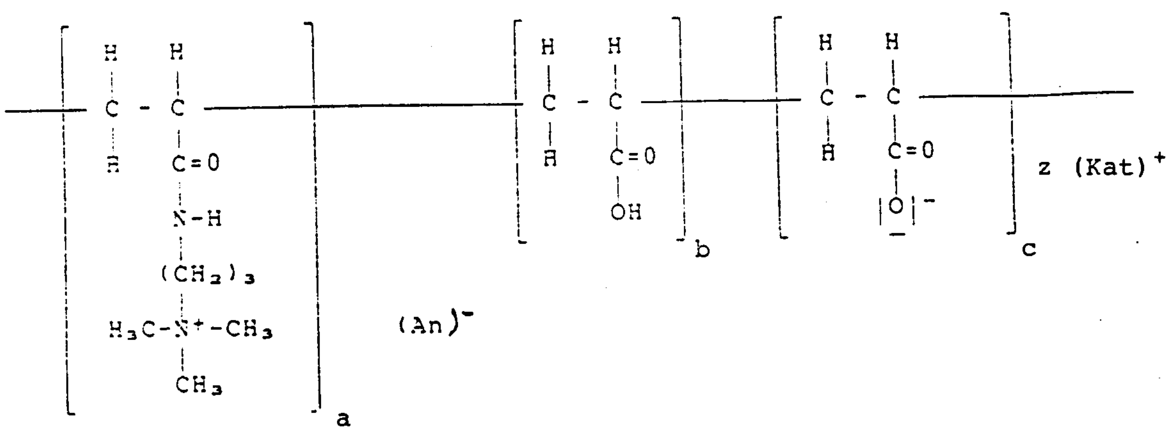

$$z = \frac{c}{\text{Wertigkeit von } (Kat)^+}$$

Wenn c = 0, dann z = 0
wobei

$(Kat)^+$ = Alkali- und/oder
Erdalkali- und/oder Erdmetallkationen und/oder Amine und/oder Alkanolamine und/oder quaternäre

Ammonium-Kationen
$(An)^-$ = Chlorid, Bromid, Jodid, $HSO_4^-$, $CH_3SO_4^-$ und/oder Nitrit

und wobei in den erfindungsgemäßen Polyelektrolyten a und b und c in folgenden Verhältnissen vorliegen:

| amphoter leicht anionisch | amphoter | amphoter kationisch |
|---|---|---|
| a = 49-47 Mol.%<br>b+c = 51-53 Mol.% | a = 50 Mol.%<br>b+c = 50 Mol.% | a = 51-80 Mol.%<br>b+c = 49-20 Mol.% |

wobei n = 1 - 18

Besonders vorteilhaft sind Polyelektrolyten gemäß dieser allgemeinen Formel, wobei

$(Kat)^+$ = Alkali- und/oder Erdalkalikationen
$(An)^-$ = Chlorid, Bromid, Jodid, $HSO_4^-$, $CH_3SO_4^-$ und/oder Nitrit

und wobei in den erfindungsgemäßen Polyelektrolyten a und b und c in folgenden Verhältnissen vorliegen :

| amphoter leicht anionisch | amphoter | amphoter kationisch |
|---|---|---|
| a = 49-48 Mol.%<br>b+c = 51-52 Mol.% | a = 50 Mol.%<br>b+c = 50 Mol.% | a = 51-70 Mol.%<br>b+c = 49-30 Mol.% |

und wobei

$$z = \frac{c}{\text{Wertigkeit (Kat)}^+}$$

Weiterhin vorteilhaft liegen in den erfindungsgemäßen Polyelektrolyten a und b und c in folgenden Verhältnissen vor :

| amphoter leicht anionisch | amphoter | amphoter kationisch |
|---|---|---|
| a = 49-47 Mol.%<br><br>b+c = 51-53 Mol.% | a = 50 Mol.%<br>b = 0-50 Mol.%<br>c = 50-0 Mol.% | a = 51-80 Mol.%<br><br>b+c = 49-20 Mol.% |

Besonders vorteilhaft sind Polyelektrolyten gemäß dieser allgemeinen Formel, wobei

$(Kat)^+$ = Erdalkalikationen
$(An)^-$ = Chlorid, Bromid, Jodid, $HSO_4^-$, $CH_3SO_4^-$ und/oder Nitrit

und wobei in den erfindungsgemäßen Polyelektrolyten a und b und c in folgenden Verhältnissen vorliegen :

| amphoter leicht anionisch | amphoter | amphoter kationisch |
|---|---|---|
| a = 49-48 Mol.% | a = 50 Mol.% | a = 51-70 Mol.% |
| | b = 0-25 Mol.% | |
| b+c = 51-52 Mol.% | c = 25-50 Mol.% | b+c = 49-30 Mol.% |

und wobei

$$z = \frac{c}{\text{Wertigkeit (Kat)}^{+}}$$

Weiterhin vorteilhaft sind Polyelektrolyten gemäß dieser allgemeinen Formel, wobei

$(Kat)^+$ = $Na^+$, $K^+$, $Li^+$, $Ca^{2+}$, $Mg^{2+}$,
$(An)^-$ = Chlorid, Bromid, Jodid, $HSO_4^-$, $CH_3SO_4^-$ und/oder Nitrit

und wobei in den erfindungsgemäßen Polyelektrolyten a und b und c in folgenden Verhältnissen vorliegen :

| amphoter leicht anionisch | amphoter | amphoter kationisch |
|---|---|---|
| a = 49-48,5 Mol.% | a = 50 Mol.% | a = 51-60 Mol.% |
| b+c = 51-51,5 Mol.% | b+c = 50 Mol.% | b+c = 49-40 Mol.% |

und wobei

$$z = \frac{c}{\text{Wertigkeit (Kat)}^{+}}$$

Ganz besonders günstige Ergebnisse werden erzielt, wenn bei den erfindungsgemäßen Polyelektrolyten gemäß dieser allgemeinen Formeln

$(Kat)^+$ = Alkalikationen
$(An)^-$ = Halogenidionen

und wobei in den erfindungsgemäßen Polyelektrolyten a und b und c in folgenden Verhältnissen vorliegen :

| amphoter leicht anionisch | amphoter | amphoter kationisch |
|---|---|---|
| a = 49 Mol.% | a = 50 Mol.% | a = 51 Mol.% |
| b = 51 Mol.% | b = 50 Mol.% | b = 49 Mol.% |
| c = < 1 Mol.% | c = < 1 Mol.% | c = < 1 Mol.% |

$$z = \frac{c}{\text{Wertigkeit (Kat)}^{+}}$$

Weiterhin vorteilhaft sind Polyelektrolyten gemäß dieser allgemeinen Formel, wobei

| amphoter leicht anionisch | amphoter | amphoter kationisch |
|---|---|---|
| a = 49-48,5 Mol.% | a = 50 Mol.% | a = 51-60 Mol.% |
| | b = 0-10 Mol.% | |
| b+c = 51-51,5 Mol.% | c = 40-50 Mol.% | b+c = 49-40 Mol.% |

und wobei

$$z = \frac{c}{\text{Wertigkeit (Kat)}^{+}}$$

Ganz besonders günstige Ergebnisse werden erzielt, wenn bei den Polyelektrolyten gemäß dieser allgemeinen Formeln

$(Kat)^{+}$ = Erdalkalikationen
$(An)^{-}$ = Halogenidionen

und wobei in den erfindungsgemäßen Polyelektrolyten a und b und c in folgenden Verhältnissen vorliegen :

| amphoter leicht anionisch | amphoter | amphoter kationisch |
|---|---|---|
| a = 49 Mol.% | a = 50 Mol.% | a = 51 Mol.% |
| b = 2 Mol.% | b = 2 Mol.% | b = 2 Mol.% |
| c = 49 Mol.% | c = 48 Mol.% | c = 47 Mol.% |

$$z = \frac{c}{\text{Wertigkeit (Kat)}^{+}}$$

Weiterhin vorteilhaft sind Mischungen aus amphoteren kationischen Polyelektrolyten und amphoteren Polyelektrolyten, bei welchen die Anzahl der kationischen Monomereinheiten gleich der Anzahl der anionischen Monomereinheiten ist, gemäß der oben angegebenen allgemeinen Formel, wobei

$(Kat)^{+}$ = Alkali- und/oder Erdalkali- und/oder Erdmetallkationen und/oder Amine und/oder Alkanolamine und/oder quaternäre Ammonium-Hationen
$(An)^{-}$ = Chlorid, Bromid, Jodid, $HSO_4^-$, $CH_3SO_4^-$ und oder Nitrit

und wobei in den erfindungsgemäßen Polyelektrolyten a und b und c in folgenden Verhältnissen vorliegen:

| amphoter | amphoter kationisch |
|---|---|
| a = 50 Mol.% | a = 70-99 Mol.% |
| b+c = 50 Mol.% | b+c = 30- 1 Mol.% |

Weiterhin vorteilhaft sind in der erfindungsgemäßen Mischung folgende Verhältnisse der Polyelektrolyten a und b und c gemäß der oben angegebenen allgemeinen Formel :

| amphoter | amphoter kationisch |
|---|---|
| a = 50 Mol.% | a = 75-98 Mol.% |
| b+c = 50 Mol.% | b+c = 25- 2 Mol.% |

bevorzugt :

| amphoter | amphoter kationisch |
|---|---|
| a = 50 Mol.% | a = 80-97 Mol.% |
| b+c = 50 Mol.% | b+c = 20- 3 Mol.% |

weiterhin bevorzugt :

| amphoter | amphoter kationisch |
|---|---|
| a = 50 Mol.% | a = 90-96 Mol.% |
| b+c = 50 Mol.% | b+c = 10- 4 Mol.% |

insbesondere bevorzugt :

| amphoter | amphoter kationisch |
|---|---|
| a = 50 Mol.% | a = 95 Mol.% |
| b+c = 50 Mol.% | b+c = 5 Mol.% |

Weiterhin vorteilhaft sind Mischungen aus amphoter leicht anionischen und amphoter kationischen Polyelektrolyten gemäß der oben angegebenen allgemeinen Formel, wobei

$(Kat)^+$ = Alkali- und/oder Erdalkali- und/oder Erdmetallkationen und/oder Amine und/oder Alkanolamine und/oder quaternäre Ammonium-Kationen

$(An)^-$ = Chlorid, Bromid, Jodid, $HSO_4^-$, $CH_3SO_4^-$ und oder Nitrit

und wobei in den erfindungsgemäßen Polyelektrolyten a und b und c in folgenden Verhältnissen vorliegen :

| amphoter leicht anionisch | amphoter kationisch |
|---|---|
| a = 47-49 Mol.% | a = 70-99 Mol.% |
| b+c = 51-53 Mol.% | b+c = 30 -1 Mol.% |

besser :

| amphoter leicht anionisch | amphoter kationisch |
|---|---|
| a = 48-49 Mol.% | a = 75-98 Mol.% |
| b+c=51-52 Mol.% | b+c = 25- 2 Mol.% |

bevorzugt :

| amphoter leicht anionisch | amphoter kationisch |
|---|---|
| a = 48,5-49 Mol.% | a = 80-97 Mol.% |
| b+c = 51-51,5 Mol.% | b+c = 20- 3 Mol.% |

insbesodere bevorzugt :

| amphoter leicht anionisch | amphoter kationisch |
|---|---|
| a = 49 Mol.% | a = 95 Mol.% |
| b+c = 51 Mol.% | b+c = 5 Mol.% |

Weiterhin vorteilhaft sind Mischungen aus amphoter leicht kationischen und amphoter kationischen Polyelektrolyten gemäß der oben angegebenen allgemeinen Formel, wobei

$(Kat)^+$ = Alkali- und/oder Erdalkali- und/oder Erdmetallkationen und/oder Amine und/oder Alkanolamine und/oder quaternäre Ammonium-Kationen

$(An)^-$ = Chlorid, Bromid, Jodid, $HSO_4^-$, $CH_3SO_4^-$ und oder Nitrit

und wobei in den erfindungsgemäßen Polyelektrolyten a und b und c in folgenden Verhältnissen vorliegen :

| amphoter leicht kationisch | amphoter kationisch |
|---|---|
| a = 51-53 Mol.% | a = 80-97 Mol.% |
| b+c = 49-47 Mol.% | b+c = 20- 3 Mol.% |

bevorzugt :

| amphoter leicht kationisch | amphoter kationisch |
|---|---|
| a = 51-52 Mol.%<br>b+c = 49-48 Mo.% | a = 90-96 Mol.%<br>b+c = 10- 4 Mol.% |

insbesondere bevorzugt :

| amphoter leicht kationisch | amphoter kationisch |
|---|---|
| a = 51 Mol.%<br>b+c = 49 Mol.% | a = 95 Mol.%<br>b+c = 5 Mol.% |

Vorteilhafterweise ist der Neutralisationsgrad der anionischen Komponente im amphoteren kationischen und amphoteren schwach anionischen und amphoteren schwach kationischen und amphoteren Polyelektrolyten mit Erdalkalikationen, besonders mit $Ca^{++}$ und/oder $Mg^{++}$, 0,1-100 Mol.%, besser 50-100 Mol.% und bevorzugterweise 70-99 Mol.%, am besten 98 Mol.% oder die anionische Komponente ist nicht-neutralisiert.

Vorteilhafterweise ist der Neutralisationsgrad der anionischen Komponente im amphoteren kationischen und amphoteren schwach anionischen und amphoteren schwach kationischen und amphoteren Polyelektrolyten mit einwertigen Kationen 0,1-100 Mol.%, besser 0,1-50 Mol.% und bevorzugterweise 0,1-39 Mol.% oder 0,1-30 Mol.%, weiterhin bevorzugt 0,1-35 Mol.% oder 0,1-25 Mol.% oder 0,1-15 Mol.%, am besten < 1 Mol.% oder die anionische Komponente ist nicht-neutralisiert. Wenn zweiwertige Kationen wie $Ca^{++}$ und $Mg^{++}$ verwendet werden, dann ist ein Neutralisationsgrad von > 90 % bevorzugt. Ein Neutralisationsgrad von > 90 % mit $Ca^{++}$ ist dabei erfindungsgemäß besser als ein Neutralisationsgrad < 1 % mit $Na^{+}$.

Es ist vorteilhaft, daß der Polymerisationsgrad der erfindungsgemäßen Polyelektrolyten, gemessen über deren Viskosität in einer wäßrigen Lösung bei 32% Konzentration, im Bereich von 5 mPa.s bis 150 mPa.s liegt. Ganz besonders vorteilhaft ist die Viskosität im Bereich von 15 mPa.s bis 100 mPa.s, wobei ein Bereich von 25 mPa.s bis 70 mPa.s insbesondere bevorzugt wird.

Weiterhin vorteilhaft ist bei der Mischung aus amphoteren kationischen Polyelektrolyten und amphoteren leicht kationischen Polyelektrolyten und/oder amphoteren Polyelektrolyten und/oder amphoteren leicht anionischen Polyelektrolyten, daß der Polymerisationsgrad der amphoteren kationischen Polyelektrolyten, gemesen über die Grenzviskosität, im Bereich von 5 ml/g bis 50 ml/g, bevorzugt im Bereich von 15 ml/g bis 40 ml/g, insbesondere bevorzugt von 25 ml/g bis 35 ml/g, und der Polymerisationsgrad der amphoteren leicht kationischen Polyelektrolyten und der amphoteren Polyelektrolyten und der amphoteren leicht anionischen Polyelektrolyten, gemessen über deren Viskosität in einer wäßrigen Lösung bei 32 Gew.% Konz. im Bereich von 5 bis 150 ml/g, bevorzugt von 15 bis 100 ml/g, insbesondere bevorzugt von 25 bis 70 ml/g liegen.

Weiterhin vorteilhaft enthält das Dispergiermittel einen oder mehrere amphotere Polyelektrolyten, bei welchen die Anzahl der negativen Ladungen in den anionischen Monomereinheiten gleich der Anzahl der positiven Ladungen in den kationischen Monomereinheiten ist.

Weiterhin vorteilhaft enthält das Dispergiermittel eine Mischung aus einem oder mehreren amphoteren Polyelektrolyten, und einem oder mehreren amphoteren kationischen Polyelektrolyten, bei welchen die nicht neutralen Monomereinheiten überwiegend positive Ladungen tragen.

Weiterhin vorteilhaft enthält das Dispergiermittel eine Mischung aus einem oder mehreren amphoteren Polyelektrolyten, und einem oder mehreren amphoteren, leicht kationischen Polyelektrolyten, bei welchen die nicht neutralen Monomereinheiten überwiegend positive Ladungen tragen.

Weiterhin vorteilhaft enthält das Dispergiermittel eine Mischung aus einem oder mehreren amphoteren Polyelektrolyten, und einem oder mehreren amphoteren kationischen Polyelektrolyten, bei welchen die nicht neutralen Monomereinheiten überwiegend positive Ladungen tragen und einem oder mehreren amphoteren anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren Polyelektrolyten, und einem oder mehreren amphoteren, leicht kationischen Polyelektrolyten, bei welchen die nicht neutralen Monomereinheiten überwiegend positive Ladungen tragen und einem oder mehreren amphoteren anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren Polyelektrolyten, und einem oder mehreren amphoteren kationischen Polyelektrolyten, bei welchen die nicht neutralen Monomereinheiten überwiegend positive Ladungen tragen und einem oder mehreren amphoteren, leicht anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren Polyelektrolyten, und einem oder mehreren amphoteren, leicht kationischen Polyelektrolyten, bei welchen die nicht neutralen Monomereinheiten überwiegend positive Ladungen tragen und einem oder mehreren amphoteren, leicht anionischen Polyelektrolyten, bei welchen die nicht neutralen Monomereinheiten überwiegend negative Ladungen tragen.

Weiterhin vorteilhaft enthält das Dispergiermittel einen oder mehrere amphotere kationische Polyelektrolyten, bei welchen die nicht neutralen Monomereinheiten überwiegend positive Ladungen tragen.

Weiterhin vorteilhaft enthält das Dispergiermittel einen oder mehrere amphotere kationische Polyelektrolyten und einen oder mehrere amphotere, leicht kationische Polyelektrolyten, bei welchen die nicht neutralen Monomereinheiten überwiegend positive Ladungen tragen.

Weiterhin vorteilhaft enthält das Dispergiermittel einen oder mehrere amphotere, leicht kationische Polyelektrolyten, bei welchen die nicht neutralen Monomereinheiten überwiegend positive Ladungen tragen.

Weiterhin vorteilhaft enthält das Dispergiermittel eine Mischung aus einem oder mehreren amphoteren kationischen Polyelektrolyten, bei welchen die nicht neutralen Monomereinheiten überwiegend positive Ladungen tragen und einem oder mehreren amphoteren anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren, leicht kationischen Polyelektrolyten, bei welchen die nicht neutralen Monomereinheiten überwiegend positive Ladungen tragen und einem oder mehreren amphoteren anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren kationischen Polyelektrolyten, bei welchen die nicht neutralen Monomereinheiten überwiegend positive Ladungen tragen und einem oder mehreren amphoteren, leicht anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren, leicht kationischen Polyelektrolyten, bei welchen die nicht neutralen Monomereinheiten überwiegend positive Ladungen tragen und einem oder mehreren amphoteren, leicht anionischen Polyelektrolyten, bei welchen die nicht neutralen Monomereinheiten überwiegend negative Ladungen tragen.

Weiterhin vorteilhaft enthält das Dispergiermittel eine Mischung aus einem oder mehreren amphoteren Polyelektrolyten, und einem oder mehreren amphoteren, leicht anionischen Polyelektrolyten, bei welchen die nichtneutralen Monomereinheiten überwiegend negative Ladungen tragen.

Weiterhin vorteilhaft enthält das Dispergiermittel eine oder mehrere amphotere, leicht anionische Polyelektrolyte, bei welchen die nichtneutralen Monomereinheiten überwiegend negative Ladungen tragen.

Die Füllstoffteilchen weisen bei Verwendung der amphoteren, leicht anionischen Polyelektrolyten trotz des leichten Überschusses an negativer Ladung (Carboxylgruppen) eine neutrale bzw. leicht positive Oberflächenladung auf. Dies rührt wahrscheinlich daher, daß ein Teil der Carboxylgruppen durch $Ca^{++}$ - Ionen derart stark neutralisiert ist, daß diese nicht mehr dissoziiert vorliegen und deshalb gegen außen neutral wirken. Dadurch überwiegen die dissoziierten kationischen Gruppen, und die Füllstoffteilchen weisen, trotz Carboxylgruppenüberschuß im eigentlichen Dispergiermittel, keine negative Ladung auf.

Dies ist insbesondere der Fall bei einem Verhältnis von 51 - 53 Mol.% Carboxylgruppen zu 47-49 Mol.% quaternären Ammoniumgruppen im Polymermolekül.

Weiterhin bevorzugt ist ein Verhältnis von 51 bis 52 Mol.% Carboxylgruppen zu 49 - 48 Mol.% quaternären Ammoniumgruppen im Polymermolekül. Insbesondere bevorzugt ist ein Verhältnis von 51 Mol.% Carboxylgruppen zu 49 Mol.% quaternären Ammoniumgruppen im Polymermolekül.

Vorzugsweise enthält das Dispergiermittel 0 bis 100 Gew.% eines ersten amphoteren Polyelektrolyten und 100 - 0 Gew.% eines zweiten amphoteren Polyelektolyten.

Weiterhin bevorzugt enthält das Dispergiermittel eine Mischung aus 0,1 bis 99,9 Gew.% eines oder mehrerer amphoterer Polyelektrolyten und 99,9 bis 0,1 Gew.% eines oder mehrerer amphoterer kationischer Polyelektrolyten.

Insbesondere bevorzugt enthält das Dispergiermittel eine Mischung aus 50 - 99,9 Gew.% bzw. 80 bis 99,9 Gew.% bzw. 10 - 50 Gew.% bzw. 10 - 30 Gew.% eines oder mehrerer amphoteren Polyelektrolyten und 0,1 bis 50 Gew.% bzw. 0,1 bis 20 Gew.% bzw. 50 - 90 Gew.% bzw. 70 - 90 Gew.% eines oder mehrerer amphoterer kationischer Polyelektrolyten.

Weiterhin bevorzugt enthält das Dispergiermittel 0,1 bis 99,9 Gew.% eines oder mehrerer amphoteren Polyelektrolyten und 99,9 bis 0,1 Gew.% eines oder mehrerer amphoterer leicht kationischer Polyelektrolyten.

Weiterhin bevorzugt enthält das Dispergiermittel eine Mischung aus 0,1 bis 99,8 Gew.% eines oder mehrerer amphoterer Polyelektrolyten und 0,1 bis 99,8 Gew.%, bevorzugt 0,1 bis 49,8 Gew.% eines oder mehrerer amphoterer kationischer Polyelektrolyten und 0,1 bis 99,8 Gew.%, bevorzugt 0,1 bis 49,8 Gew.% eines oder mehrerer amphoterer anionischer Polyelektrolyten.

Weiterhin bevorzugt enthält das Dispergiermittel eine Mischung aus 0,1 bis 20 Gew.% eines oder mehrerer amphoterer Polyelektrolyten und 60 bis 79,9 Gew.% eines oder mehrerer amphoteren kationischer Polyelektrolyten und 0,1 bis 20 Gew.% eines oder mehrerer amphoterer anionischer Polyelektrolyten.

Weiterhin bevorzugt enthält das Dispergiemittel eine Mischung aus 0,1 bis 99,8 Gew.% eines oder mehrerer amphoterer Polyelektrolyten und 0,1 bis 99,8 Gew.%, bevorzugt 0,1 bis 49,8 Gew.% eines oder mehrerer amphoterer leicht kationischer Polyelektrolyten und 0,1 bis 99,8 Gew.%, bevorzugt 0,1 bis 49,8 Gew.% eines oder mehrerer amphoterer anionischen Polyelektrolyten.

Weiterhin bevorzugt enthält das Dispergiermittel eine Mischung aus 0,1 bis 99,8 Gew.% eines oder mehrerer amphoterer Polyelektrolyten und 0,1 bis 99,8 Gew.%, bevorzugt 0,1 bis 49,8 Gew.% eines oder mehrerer amphoterer kationischer Polyelektrolyten und 0,1 bis 99,8 Gew.%, bevorzugt 0,1 bis 49,8 Gew.% eines oder mehrerer amphoterer leicht anionischer Polyelektrolyten.

Weiterhin bevorzugt enthält das Dispergiermittel eine Mischung aus 0,1 bis 99,8 Gew.% eines oder mehrerer amphoterer Polyelektrolyten und 0,1 bis 99,8 Gew.% eines oder mehrerer amphoterer leicht kationischer Polyelektrolyten und 0,1 bis 99,8 Gew.% eines oder mehrerer amphoterer leicht anionischer Polyelektrolyten.

Weiterhin bevorzugt enthält das Dispergiermittel 0 bis 100 Gew.% eines ersten amphoteren kationischen Polyelektrolyten und 0 bis 100 Gew.% eines zweiten amphoteren kationischen Polyelektrolyten.

Weiterhin bevorzugt enthält das Dispergiermittel 0,1 bis 99,9 Gew.% eines ersten amphoteren leicht kationischen Polyelektrolyten und 0,1 bis 99,9 Gew.% eines zweiten amphoteren leicht kationischen Polyelektrolyten.

Weiterhin bevorzugt enthält das Dispergiermittel 50 bis 99,9 Gew.% bzw. 70 bis 99,9 Gew.% eines oder mehrerer amphoterer kationischer Polyelektroylten und 0,1 bis 50 Gew.% bzw. 0,1 bis 30 Gew.% eines oder mehrerer amphoterer anionischer Polyelektrolyten.

Weiterhin bevorzugt enthält das Dispergiermittel eine Mischung aus 90 bis 99,9 Gew.% bzw. 75 bis 90 Gew.% bzw. 80 Gew.% eines oder mehrerer amphoterer kationischer Polyelektrolyten und 0,1 bis 10 Gew.% bzw. 25 bis 10 Gew.% bzw. 20 Gew.% eines oder mehrerer amphoterer anionischer Polyelektrolyten enthält.

Weiterhin bevorzugt enthält das Dispergiermittel eine Mischung aus 80 bis 99,9 Gew.% eines oder mehrerer amphoterer leicht kationischer Polyelektrolyten und 0,1 bis 20 Gew.% eines oder mehrerer amphoterer anionischer Polyelektrolyten.

Weiterhin bevorzugt enthält das Dispergiermittel eine Mischung aus 0,1 bis 99,9 Gew.% eines oder mehrerer amphoterer kationischer Polyelektrolyten und 99,9 bis 0,1 Gew.% eines oder mehrer amphoterer leicht anionischer Polyelektrolyten.

Weiterhin bevorzugt enthält das Dispergiermittel 50 bis 99,9 Gew.% bzw. 70 bis 90 Gew.% bzw. 75 Gew.% eines oder mehrerer amphoterer kationischer Polyelektrolyten und 0,1 bis 50 Gew.% bzw. 10 bis 30 Gew.% bzw. 25 Gew.% eines oder mehrerer amphoterer leicht anionischer Polyelektrolyten enthält.

Weiterhin bevorzugt enthält das Dispergiermittel 0,1 bis 99,9 Gew.% eines oder mehrerer amphoterer leicht kationischer Polyelektrolyten und 99,9 bis 0,1 Gew.% eines oder mehrerer amphoterer leicht anionischer Polyelektrolyten.

Weiterhin bevorzugt enthält das Dispergiermittel eine Mischung aus 0,1 bis 99,9 Gew.% bzw. 50 bis 99,9 Gew.% eines oder mehrerer amphoterer Polyelektrolyten und 0,1 bis 99,9 Gew.% bzw. 0,1 bis 50 Gew.% eines oder mehrerer amphoterer leicht anionischer Polyelektrolyten.

Weiterhin bevorzugt enthält das Dispergiermittel 0 bis 100 Gew.% eines ersten und 0 bis 100 Gew.% eines zweiten amphoteren leicht anionischen Polyelektrolyten.

Erfindungsgemäß umfassen die Mineralien bzw. Füllstoffe bzw. Pigmente insbesondere Elemente aus der zweiten und/oder dritten Hauptgruppe und/oder aus der vierten Nebengruppe des Periodensystems der Elemente. Günstigerweise werden calcium- und/oder siliziumhaltige und/oder aluminiumhaltige und/oder titanhaltige Mineralien und/oder Füllstoffe und/oder Pigmente verwendet, wobei calciumcarbonathaltige Mineralien und/oder Füllstoffe und/oder Pigmente bevorzugt sind. Ganz besonders bevorzugt sind natürliches Calciumcarbonat und/oder präzipitiertes Calciumcarbonat und/oder Marmor und/oder Kreide und/oder Dolomit und/oder dolomithaltiges Calciumcarbonat.

Die wäßrige Suspension besteht bevorzugt aus 97,0 Gew.% bis 99,97 Gew.% Mineralien und/oder Füllstoffen und/oder Pigmenten und Wasser und 0,03 Gew.% - 3,0 Gew.% der erfindungsgemäßen amphoteren Polyelektrolyten bei einem Feststoffgehalt von 60 - 80 Gew.%, bezogen auf das trockene Mineral bzw. den trockenen Füllstoff bzw. das trockene Pigment.

Weiterhin günstig ist es, daß die wäßrige Suspension aus 98,5 Gew.% bis 99,95 Gew.% Mineralien und/oder Füllstoffen und/oder Pigmenten und Wasser und 0,05 Gew.% bis 1,5 Gew.% der erfindungsgemäßen amphoteren Polyelektrolyten bei einem Feststoffgehalt von 65 - 77 Gew.%, bezogen auf das trockene Mineral bzw. den trockenen Füllstoff bzw. das trockene Pigment, besteht.

Weiterhin gute Ergebnisse werden erzielt, wenn die wäßrige Suspension aus 98,8 Gew.% bis 99,90 Gew.% Mineralien und/oder Füllstoffen und/oder Pigmenten und Wasser und 0,1 Gew.% bis 1,2 Gew.% der erfindungsgemäßen amphoteren Polyelektrolyten bei einem Feststoffgehalt von 67 - 76 Gew.%, bezogen auf das trockene Mineral bzw. den trockenen Füllstoff bzw. das trockene Pigment besteht.

Hervorragende Ergebnisse werden erzielt, wenn die wäßrige Suspension besteht aus 99,5 Gew.% bzw. 98,8 Gew.% bzw. 99,6 Gew.% Mineralien und/oder Füllstoffen und/oder Pigmenten und Wasser und 0,5 Gew.% bzw. 1,2 Gew.% bzw. 0,4 Gew.% eines amphoteren, gegen außen neutralen Polyelektrolyten mit einer Viskosität von 37 mPa.s bei einem Feststoffgehalt von 72 Gew.% bzw. 72 Gew.% bzw. 67 Gew.%, bezogen auf das trockene Mineral bzw. den trockenen Füllstoff bzw. das trockene Pigment, bei einer Kornverteilung, so daß 70 Gew.% bzw. 90 Gew.% bzw. 60 Gew.% der Teilchen einen äquivalent sphärischen Durchmesser < 2 µm aufweisen.

Weiterhin ist es vorteilhaft, wenn die wäßrige Suspension aus 97 bis 99,89 Gew.% besser 98,5 bis 99,8 Gew.% besser 99,2 bis 99,65 Gew.% Mineralien und/oder Füllstoffen und/oder Pigmenten und Wasser und einer Dispergiermittelmischung aus amphoteren kationischen und amphoter leicht anionischen und/oder amphoteren und/oder amphoter leicht kationischen Polyelektrolyten im Bereich von 0,11 bis 3,00 Gew.% besser 0,2 bis 1,5 Gew.% besser 0,35 bis 0,8 Gew.% besteht, je bezogen auf einen Feststoffgehalt im Bereich von 60 - 80 Gew.% bevorzugt 62 - 75 Gew.%, insbesondere 65 - 72 Gew.% bezogen auf das trockene Mineral bzw. den trockenen Füllstoff bzw. das trockene Pigment.

Hervorragende Ergebnisse werden erzielt, wenn die wäßrige Suspension aus 99,6 Gew.% Mineralien und/oder Füllstoffen und/oder Pigmenten und Wasser und 0,4 Gew.% der oben ausgeführten erfindungsgemäßen Dispergiermittelmischung besteht.

Insbesonders gute Ergebnisse werden erzielt, wenn die wäßrige Suspension besteht aus 99,6 Gew.% Mineralien und/oder Füllstoffen und/oder Pigmenten und Wasser und 0,4 Gew.% einer Dispergiermittelmischung, bestehend aus 0,35 Gew.% eines amphoteren kationischen Polyelektrolyten gemäß der allgemeinen Formel von Seite 17, wobei a = 95 Mol.% und b = 5 Mol.% und c = 0 Mol.% bei einer Grenzviskosität von 27,3 ml/g und 0,1 Gew.% eines amphoteren Polyelektrolyten gemäß der allgemeinen Formel von Seite 17, bei welcher a = 50 Mol.% und b = 50 Mol.% und c = 0 Mol.%, mit einer Viskosität, gemessen in einer wäßrigen Lösung von 32 Gew.%, von 37 mPas bei einem Feststoffgehalt von 67 Gew.%, wobei 60 Gew.% der Teilchen einen äquivalent sphärischen Durchmesser < 2 µm aufweisen.

Eine weitere Aufgabe dieser Erfindung ist es, ein Verfahren zu schaffen, mit dem eine lagerstabile, hochkonzentrierte Mineral- und/oder Füllstoff- und/oder Pigment-Suspension durch Mahlen bei hohen Feststoffgehalten hergestellt werden kann, wobei Mahlen und Dispergieren bei hohen Feststoffgehalten in einem Arbeitsgang erfolgen.

Diese Aufgabe wird erfindungsgemäß dadurch gelöst, daß ein Verfahren zur Herstellung einer wäßrigen Suspension bereitgestellt wird, welches durch folgende Verfahrensschritte gekennzeichnet ist:

a) eine wäßrige Suspension von Mineralien und/oder Füllstoffen und/oder Pigmenten wird zusammen mit der erfindungsgemäßen Dispergier- und Mahlhilfsmittelmischung naßvermahlen, wobei
b) die erfindungsgemäßen amphoteren Polyelektrolyten vollständig vor der Vermahlung oder
c) ein Teil der erfindungsgemäßen amphoteren Polyelektrolyten vor der Vermahlung und
d) ein Teil der erfindungsgemäßen amphoteren Polyelektkrolyten während der Vermahlung und/oder
e) einen Teil der erfindungsgemäßen amphoteren Polyelektrolyten nach der Vermahlung

hinzugegeben werden.

Besonders vorteilhaft ist ein Verfahren, wobei

a) die amphoteren, leicht anionischen und/oder die amphoteren Polyelektrolyten vollständig vor der Vermahlung oder
b) ein Teil der amphoteren , leicht anionischen und/oder der amphoteren Polyelektrolyten vor der Vermahlung und
c) ein Teil der amphoteren, leicht anionischen und/oder der amphoteren Polyelektrolyten während der Vermahlung und/oder
d) ein Teil der amphoteren, leicht anionischen und/oder der amphoteren Polyelektrolyten nach der Vermahlung

hinzugegeben werden.

Weiterhin vorteilhaft ist ein Verfahren, in welchem

a) die amphoteren und/oder die amphoteren kationischen Polyelektrolyten vollständig vor der Vermahlung oder
b) ein Teil der amphoteren und/oder der amphoteren kationischen Polyelektrolyten vor der Vermahlung und
c) ein Teil der amphoteren und/oder der amphoteren kationischen Polyelektrolyten während der Vermahlung und/oder
d) ein Teil der amphoteren und/oder der amphoteren kationischen Polyelektrolyten nach der Vermahlung

hinzugegeben werden.

Besonders vorteilhaft ist ein Verfahren, in welchem

a) 50 - 100 Gew.% der amphoteren, leicht anionischen und/oder der amphoteren Polyelektrolyten vor der Vermahlung und
b) 0 - 50 Gew.% der amphoteren, leicht anionischen und/oder der amphoteren Polyelektrolyten während der Vermahlung und/oder
c) 0 - 50 Gew.% der amphoteren, leicht anionischen und/oder der amphoteren Polyelektrolyten nach der Vermahlung

zugegeben werden.

Weiterhin vorteilhaft ist ein Verfahren, in welchem

a) 50 - 100 Gew.% der amphoteren und/oder der amphoteren kationischen Polyelektrolyten vor der Vermahlung und
b) 0 - 50 Gew.% der amphoteren und/oder der amphoteren kationischen Polyelektrolyten während der Vermahlung und/oder
c) 0 - 50 Gew.% der amphoteren und/oder der amphoteren kationischen Polyelektrolyten nach der Vermahlung

zugegeben werden.

Sehr gute Resultate werden erzielt, wenn ein Verfahren angewandt wird, bei welchem

a) 70 - 100 Gew.% der amphoteren, leicht anionischen und/oder der amphoteren Polyelektrolyten vor der Vermahlung und/oder
b) 0 - 30 Gew.% der amphoteren, leicht anionischen und/oder der amphoteren Polyelektrolyten während der Vermahlung und/oder
c) 0 - 30 Gew.% der amphoteren, leicht anionischen und/oder der amphoteren Polyelektrolyten nach der Vermahlung

zugegeben werden.

Weiterhin vorteilhaft ist ein Verfahren, bei welchem

a) 70 - 100 Gew.% der amphoteren und/oder der amphoteren kationischen Polyelektrolyten vor der Vermahlung und
b) 0 - 30 Gew.% der amphoteren und/oder der amphoteren kationischen Polyelektrolyten während der Vermahlung und/oder
c) 0 - 30 Gew.% der amphoteren und/oder der amphoteren kationischen Polyelektrolyten nach der Vermahlung

zugegeben werden.

Weiterhin vorteilhaft ist ein Verfahren, bei welchem

a) ein Teil der amphoteren, leicht kationischen und/oder der amphoteren und /oder der amphoteren, leicht anionischen Polyelektrolyten vor der Vermahlung und
b) ein Teil der amphoteren, leicht kationischen und/oder der amphoteren und /oder der amphoteren, leicht anionischen Polyelektrolyten während der Vermahlung und/oder
c) ein Teil der amphoteren, leicht kationischen und/oder der amphoteren und /oder der amphoteren, leicht anionischen Polyelektrolyten nach der Vermahlung

zugegeben werden.

Weiterhin vorteilhaft ist ein Verfahren, bei welchem

a) die amphoteren kationischen Polyelektrolyten vollständig vor der Vermahlung oder
b) ein Teil der amphoteren kationischen Polyelektrolyten vor der Vermahlung und
c) ein Teil der amphoteren kationischen Polyelektrolyten während der Vermahlung und/oder
d) ein Teil der amphoteren kationischen Polyelektrolyten nach der Vermahlung

zugegeben werden.

Insbesondere vorteilhaft ist ein Verfahren, bei welchem

a) 10 - 90 Gew.% bzw. 20 - 40 Gew.% bzw. 30 Gew.% der amphoteren, leicht kationischen und/oder amphoteren und /oder amphoteren, leicht anionischen Polyelektrolyten vor der Vermahlung und

b) 10 - 90 Gew.% bzw. 60 - 80 Gew.% bzw. 70 Gew.% der amphoteren, leicht kationischen und/oder amphoteren und /oder amphoteren, leicht anionischen Polyelektrolyten während der Vermahlung und/oder

c) 0 - 80 Gew.% bzw. 0 - 20 Gew.% der amphoteren, leicht kationischen und/oder amphoteren und /oder amphoteren, leicht anionischen Polyelektrolyten nach der Vermahlung

zugegeben werden.

Insbesondere bevorzugt ist ein Verfahren, bei welchem

a) 50 - 100 Gew.% bzw. 70 - 100 Gew.% der amphoteren kationischen Polyelektrolyten vor der Vermahlung und

b) 0 - 50 Gew.% bzw. 0 - 30 Gew.% der amphoteren kationischen Polyelektrolyten während der Vermahlung und/oder

c) 0 - 50 Gew.% bzw. 0 - 30 Gew.% der amphoteren kationischen Polyelektrolyten nach der Vermahlung

zugegeben werden.

Hervorragende Ergebnisse werden bei dem Verfahren erzielt, bei welchem einerseits 100 Gew.% der amphoteren, leicht anionischen und/oder der amphoteren Polyelektrolyten oder andererseits 100 Gew.% der amphoteren und/oder der amphoteren kationischen Polyelektrolyten vor der Vermahlung zugegeben werden, wenn die gewünschte Endfeinheit in einem Mühlendurchgang erreicht werden soll.

Bei mehreren Mühlendurchgängen zur Erreichung der Endfeinheit ergeben sich hervorragende Ergebnisse, wenn die notwendige Dispergiermittelmenge entsprechend der erreichten Zwischenfeinheit aufgeteilt wird.

Erfindungsgemäß erfolgt die Verwendung der wäßrigen Suspension von Mineralien und/oder Füllstoffen und/oder Pigmenten bei der Papierherstellung bzw. in der Papiererzeugung. Weitere Verwendungen betreffen die Oberflächenbehandlung (Pigmentierung) der Papieroberfläche in der Leimpresse der Papiermaschine, die Verwendung in der Papierstreicherei, im Vorstrich bzw. im Deckstrich bei der Papierstreicherei, im Holzschliff zur Störstoffbekämpfung, im Streichereiausschuß zur Störstoffbekämpfung (Pitchkontrolle), im Kreislaufwasser der Papiermaschine zur CSB-Erniedrigung (chemischer Sauerstoffbedarf-Erniedrigung), in der Kläranlage zur Abwasserbehandlung, zur Vorflokkung anionisch stabilisierter Pigment- und/oder Mineral- und/oder Füllstoff-Suspensionen in der Papiererzeugung bzw. zur Vorflockung (Immobilisierung) von Streichfarben in der Streicherei.

Es ist erfindungsgemäß gelungen, eine Mineral- und/oder Füllstoff- und/oder Pigment-Suspension durch Mahlen bei hohen Feststoffgehalten von $\geq 60$ Gew.% herzustellen, bei welcher die Mineral- und/oder Füllstoff- und/oder Pigmentteilchen vermutlich sowohl elektrostatisch positiv wie auch sterisch stabilisiert sind und die Suspension über Wochen genügend viskositätsstabil bleibt ,über lange Strecken sehr gut transportfähig ist , nicht absetzt und z. B. die Retention bei der Papierherstellung vorzüglich ist.

Überraschend und nicht voraussehbar war die Tatsache, daß bei der geeigneten Kombination eines oder mehrerer kationischer Monomere und eines oder mehreren anionischer Monomere sowie der geeigneten Zugabestelle der daraus polymerisierten amphoteren Polyelektrolyten vor, und/oder während und/oder nach dem Mahlprozess bei den hohen Scherkräften und Temperaturen, wie sie beim Naßvermahlen auftreten, keine gegenseitige Neutralisation der entgegengesetzt geladenen Monomereinheiten und somit Koagulation der Polymere eintritt. Im Gegensatz dazu wird eine optimale Mahlung und Stabilisierung über längere Zeit der Suspension erreicht.

Die Zetapotentiale der Füllstoff- und/oder Pigment- und/oder Mineralteilchen weisen positive Vorzeichen auf oder sind gegen außen neutral, d.h. bei den neutralen Füllstoff- und/oder Pigment- und/oder Mineralteilchen heben sich die Summe der positiven und negativen Ladungen auf der Oberfläche der Teilchen gegen außen gegenseitig auf.

Eine gute Lagerstabilität in Bezug auf die Viskosität und das Absetzverhalten ist vor allem beim Transport und bei großen Lagertanks von ausschlaggebender Bedeutung, um den Verderb der Ware zu verhindern. Mit der erfindungsgemäß hergestellten Mineral- und/oder Füllstoff- und/oder Pigment-Suspension ist es möglich, den Produktionsort (Herstellungsort der Mineral- und/oder Füllstoff- und/oder Pigment-Suspension) sowie den Verbraucherort (z. B. Papierfabrik) frei zu wählen. Der Produktionsort kann so den geologischen Vorkommen der Mineral- und/oder Füllstoff- und/oder Pigment-Vorkommen angepaßt werden, und es muß nicht aus rein logistischen Gründen der Standort des Kunden mitberücksichtigt werden. Man ist dadurch auch völlig frei in der Wahl der Transportmittel und kann die ökologisch sinnvollste Variante wählen.

Eine wäßrige Suspension von Mineralien und/oder Füllstoffen und/oder Pigmenten mit einem Feststoffgehalt $\geq 60$ Gew.%, bezogen auf die trockenen Mineralien und/oder Füllstoffe und/oder Pigmente, wird erfindungsgemäß durch Mahlen eines grob gebrochenen Rohgesteins hergestellt, wobei die erfindungsgemäßen amphoteren Polyelektrolyten zu Beginn der Vermahlung zugesetzt werden und/oder weitere Teile der erfindungsgemäßen amphoteren Polyelektrolyten während der Mahlung und/oder nach der Mahlung je in der erfindungsgemäßen Zusammensetzung zur Viskositätssenkung zugegeben werden.

Die für die Anwender, vornehmlich die Papierindustrie, ideale Kornverteilung, Konzentration und Lagerstabilität bei tiefer Viskosität der Mineral- und/oder Füllstoff- und/oder Pigment-Suspensionen können nach dem erfindungsgemäßen Verfahren in einem Arbeitsgang hergestellt werden, was einen enormen ökonomischen und qualitativen Fortschritt darstellt.

- Vorzugsweise beträgt die Konzentration der wäßrigen Aufschlämmung 60 - 78 Gew.%, bezogen auf das trockene Mineral.
- Vorzugsweise hat das Rohmaterial vor dem Mahlprozeß erfindungsgemäß einen mittleren äquivalent sphärischen Teilchendurchmeser von 10 -50 μm (gemessen auf dem Sedigraph 5100).

Vorbemerkungen zu den Beispielen :

a) Viskositätsmessung der amphoteren Polyelektrolyten

Die Viskositätsmessung erfolgte auf einen Brookfield Viskosimeter Typ PVF-100 bei 100 U/min. Für die einzelnen Messungen wurde die Spindel 1 verwendet:

Die Konzentration betrug bei allen Proben 32 Gew.% Polymer in Wasser. Der pH-Wert bei welchem die Viskosität gemessen wurde, entspricht dem angegebenen Wert bei den entsprechenden Beispielen. Die anionischen Gruppen lagen nicht neutralisiert vor.

Die Messung erfolgte in einem 400 ml-Becherglas von niedriger Form.

Die Temperatur während der Messung betrug 20°C. die Messung erfolgte nach 1 Min Rührzeit.

Diese Art der Viskositätsmessung wurde für alle folgenden Beispiele verwendet, mit Ausnahme der amphoteren kationischen Polyelektrolyten in der Mischung mit den amphoteren, leicht kationischen und/oder amphoteren und/oder amphoteren, leicht anionischen Polyelektrolyten.

b) Feinheit der Mineral- und/oder Füllstoff- und/oder Pigment-Suspension:

Die Feinheitsmerkmale der erfindungsgemäß hergestellten Suspensionen wurden durch Sedimentationsanalyse im Schwerefeld mit dem SEDIGRAPH 5100 der Firma Micromeritics, U.S.A., bestimmt.

Die Messung der kationisch stabilisierten Suspensionen erfolgte in destilliertem Wasser. Die Dispergierung der Proben wurde mittels Schnellrührer und Ultraschall vorgenommen.

Die Messung der Pulver erfolgte in 0,1 % $Na_4P_2O_7$-Lösung.

Die gemessene Teilchenverteilung wurde auf einem X-Y-Schreiber als Durchgangs-Summenkurve dargestellt (siehe z.B. Belger, P., Schweizerische Vereinigung der Lack- und Farben-Chemiker, XVII. FATIPEC-Kongreß, Lugano, 23. bis 28. September 1984), wobei auf der X-Achse der Teilchendurchmesser eines entsprechenden sphärischen Durchmessers und auf der Y-Achse der Anteil an Teilchen in Gew.% aufgetragen wurde.

c) Viskositätsmessung der Mineral- und/oder Füllstoff- und/oder Pigment-Suspension:

Die Viskositätsmessung erfolgte auf einen Brookfield Viskosimeter Typ PVF-100 bei 100 U/min. Für die einzelnen Messungen wurden die folgenden Spindeln verwendet:

| | |
|---|---|
| Spindel | RV 2 40 - 320 mPas |
| | RV 3 320 - 800 mPas |
| | RV 4 800 - 1600 mPas |
| | RV 5 1600 - 3200 mPas |
| | RV 6 3200 - 8000 mPas |

Die Messung erfolgte in einem 400 ml-Becherglas von niedriger Form.

Die Temperatur während der Messung betrug 20°C. Die Messung erfolgte nach 1 min. Rührzeit.

Vor den eigentlichen Messungen wurden alle Proben 2 Min. intensiv gerührt (5000 U/min, Rührscheibendurchmesser 50 mm).

Diese Art der Viskositätsmessung wurde für alle folgenden Beispiele verwendet.

d) Die spezifische Viskosität für die anionischen Dispergiermittel in den Anwendungsbeispielen, welche als Symbol den griechischen Buchstaben "Eta" hat, wurde wie folgt bestimmt:

Man stellt eine Lösung des Polymers/Copolymers, zur Messung 100% neutralisiert mit Natronlauge (pH 9), her,

indem man 50 g, bezogen auf trockenes Polymer/Copolymer, in 1 lt, 60 g NaCl enthaltendes, destilliertes Wasser auflöst.

Danach mißt man mit einem Kapillarviskosimeter mit einer Baumekonstante von 0.000105 in einem auf 25°C thermostabiliserten Heizbad die Zeit, die ein genau definiertes Volumen der alkalischen Polymer/Copolymerlösung zum Durchströmen der Kapillare braucht und vergleicht mit der Zeit, die dasselbe Volumen der Blindlösung mit 60 g NaCl/1 zum Durchströmen der Kapillare braucht.

Es ist somit möglich, die spezifische Viskosität "Eta" wie folgt zu definieren:

$$\text{Eta} = \frac{\text{Zeit des Durchströmens der Polymerlösung - Zeit des Durchströmens der NaCl-Lösung}}{\text{Zeit des Durchströmens der NaCl-Lösung}}$$

Die besten Resultate werden erreicht, wenn der Kapillardurchmesser so gewählt wird, daß die Zeit, welche die Polymer/Copolymer enthaltende NaCl-Lösung benötigt, zwischen 90 und 100 sec. beträgt.

e) Die Grenzviskosität der amphoteren kationischen Polyelektrolyten in der Mischung mit den amphoteren, leicht kationischen und/oder amphoteren und/oder amphoteren, leicht anionischen Polyelektrolyten sowie von Poly-DADMAC der Beispiele 1a) bis 1c) wurde nach folgender Literatur bestimmt:

B. Vollmert "Grundriß der Makromolekularen Chemie" Band III
E. Vollmert-Verlag, Karlsruhe 1985.

f) Ladungsmessung der Pigment-, Füllstoff- und Mineralsuspension mit SCD

Zur Bestimmung der Oberflächenladungen wurde der "Streaming Current Detector" der Fa. Mütek, Hersching b. München, verwendet. (Typ PCD-02)

Die Titrationen erfolgten nach den Ausführungen in der Dissertation "Untersuchungen zur Anwendung der Polyelektrolyttitration auf dem Gebiet der Papierherstellung" von Peter Heß, Darmstadt, 1983, insbesondere gemäß den Seiten 33 ff. dieser Dissertation.

Als Titrierlösung wurde 0,01 M Kaliumpolyvinylsulfatlösung (KPVS) der Fa. SERVA verwendet.

Herstellungsbeispiele

I. Beispiele zum Stand der Technik

Beispiel 1a:

Eine 60 Gew.%-ige wäßrige Aufschlämmung von natürlichem Marmor mit einer Kornverteilung, so daß 60 Gew.% der Teilchen einen äquivalent sphärischen Durchmesser < 2 μm aufweisen (gemessen auf dem Sedigraph 5100), wurden mit 0,1 Gew.% eines Poly(diallyldimethylammoniumchlorid), Grenz-Viskosität 25 ml/g, und 0,02 Gew.% eines Natriumpolyacrylats (spez. Viskosität 0,35; 100 % der Carboxylgruppen mit NaOH neutralisiert), je bezogen auf den trockenen Marmor, unter starken Scherkräften dispergiert (8000 U/min, Rührscheiben ∅ 50 mm).

| Viskosität in mPas | | | |
|---|---|---|---|
| nach 1 Std. | 2 Tage | 6 Tage | 12 Tage |
| 204 | 420 | 640 | 1560 |

Beispiel 1 a zeigt, daß die Viskosität beim Stand der Technik nicht stabil ist und daß die Suspension nach zwei Wochen bereits unbrauchbar ist.

Beispiel 1 b:

Es wurde versucht, eine 67 Gew.%-ige wäßrige Aufschlämmung von natürlichem Marmor mit einem äquivalent sphärischen mittleren Teilchendurchmesser von 12 μm (gemessen auf dem Sedigraph 5100) nach folgender Rezeptur auf einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 µm (gemessen auf dem Sedigraph 5100) aufweisen, aufzumahlen.

Rezeptur:

| | |
|---|---|
| 5000 g | Marmor |
| 15 g | Poly(diallyldimethylammoniumchlorid) Grenz-Viskosität 25 ml/g |
| 4,5 g | Natriumpolyacrylat (spez. Viskosität 0,35, 100 % der Carboxylgruppen mit NaOH neutralisiert) |
| 2472 g | Wasser |

Die Mahlung mußte abgebrochen werden, da der Viskositätsanstieg derart hoch war, daß eine Weitervermahlung nicht mehr möglich war, weil die Mühle blockierte. Die gewünschte Endfeinheit konnte nicht erreicht werden.

Beispiel 1 c:

Eine 60 Gew.%-ige wäßrige Aufschlämmung von natürlchem Marmor mit einem äquivalent spärischen mittleren Teilchendurchmesser von 12 µm (gemessen auf dem Sedigraph 5100) wurde in der folgenden Rezeptur auf einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 µm (gemessen auf dem Sedigraph 5100) aufwiesen, aufgemahlen.

Rezeptur:

| | |
|---|---|
| 5000 g | Marmor |
| 15 g | Poly(diallyldimethylammoniumchlorid) Grenz-Viskosität 25 ml/g |
| 4,5 g | Natriumpolyacrylat (spez. Viskosität 0,35; 100 % der Carboxylgruppen mit NaOH neutralisiert) |
| 3346 g | Wasser |

Auch bei einer Konzentration von 60 Gew.% ergab sich keine Verbesserung der Mahleigenschaften mit einem Poly(diallyldime-thylammoniumchlorid) gegenüber dem Versuch 1b.

Eine Vermahlung auf die gewünschte Feinheit bei Viskositäten < 2000 mPas war mit dem Stand der Technik nicht möglich.

II. Erfindungsgemäße Beispiele

Beispiel 2:

Eine 67 Gew.%-ige wäßrige Aufschlämmung von natürlichem Mamor mit einer Kornverteilung, so daß 60 Gew.% der Teilchen einen äquivalent sphärischen Durchmesser < 2µm aufweisen (gemessen auf dem Sedigraph 5100), wurde mit unterschiedlichen Mengen, bezogen auf den trockenen Marmor, des folgenden Copolymers hergestellt,

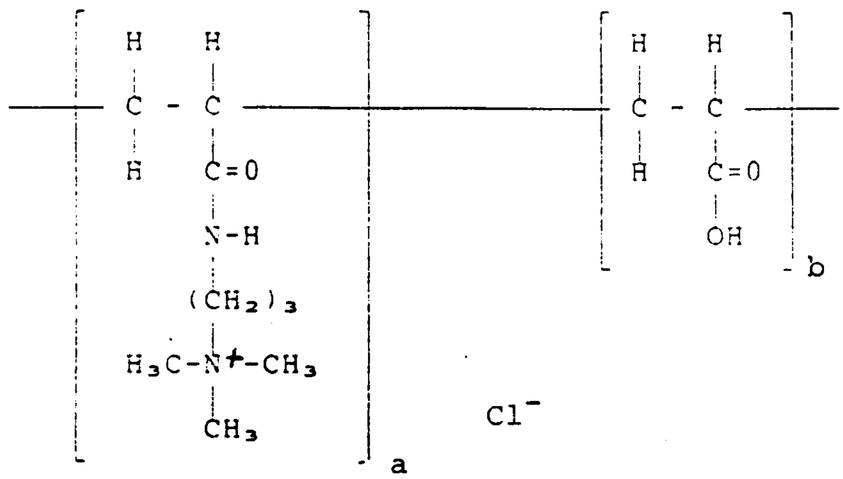

a=50 Mol.%
b=50 Mol.%

wobei das Copolymer zusätzlich im Molekulargewicht resp. Eigenviskosität der 32 Gew.%-igen wäßrigen Lösung variiert wurde. Es wurde unter starkem Rühren dispergiert (8000 U/min, Rührscheibendurchmesser 50 mm).
Das Ziel dieser Versuchsserie war es, die optimale Viskosität bzw. das Molekulargewicht der amphoteren Polyelektrolyten sowie die optimale Dispergiermittelmenge festzustellen.

TABELLE 1

| Viskosität und pH-Wert des amphoteren Polyelektrolyten (32 Gew.%ig in $H_2O$) | | Zugabemenge in Gew.% bez. auf trockenen Marmor | Viskosität der Suspension | |
|---|---|---|---|---|
| Viskosität | pH-Wert | | sofort | nach 20 Tg. |
| 95 mPas | 3,3 | 0,1 | 1440 | |
| | | 0,15 | 840 | |
| | | 0,2 | 610 | |
| | | 0,25 | 420 | |
| | | 0,3 | 340 | |
| | | 0,35 | 275 | |
| | | 0,4 | 215 | |
| | | 0,5 | 165 | 155 |
| 61 mPas | 3,3 | 0,1 | 1430 | |
| | | 0,2 | 420 | |
| | | 0,3 | 265 | |
| | | 0,4 | 190 | 180 |
| 37 mPas | 3,3 | 0,1 | 950 | |
| | | 0,2 | 250 | |
| | | 0,3 | 145 | 230 |
| 24 mPas | 3,5 | 0,1 | 1910 | |
| | | 0,2 | 1180 | |
| | | 0,3 | 670 | |
| | | 0,4 | 455 | |
| | | 0,5 | 360 | |
| | | 0,6 | 275 | |
| | | 0,7 | 200 | 280 |

Die optimale Viskosität der amphoteren Polyelektrolyten liegt bei 30 - 50 mPas.

Beispiel 3 :

Eine 67 Gew.%-ige wäßrige Aufschlämmung von natürlichem Marmor mit einer Kornverteilung, so daß 60 Gew.% der Teilchen einen äquivalent sphärischen Durchmesser < 2 µm aufweisen (gemessen auf dem Sedigraph 5100), wurden mit unterschiedlichen Mengen, bezogen auf den trockenen Marmor, des folgenden Copolymers hergestellt,

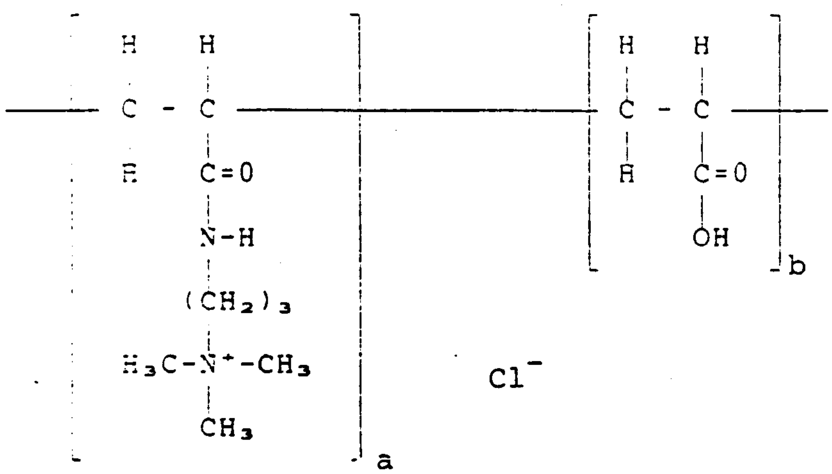

a= 70 Mol.%
b= 30 Mol.%

wobei das Copolymer zusätzlich im Molekulargewicht resp. Eigenviskosität der 32 Gew.%-igen wäßrigen Lösung variiert wurde. Es wurde unter starkem Rühren dispergiert (8000 U/min Rührscheibendurchmesser 50 mm).

Das Ziel dieser Versuchsserie war es, die optimale Viskosität bzw. das Molekulargewicht der amphoteren kationischen Polyelektrolyten festzustellen, sowie die optimale Dispergiermittelmenge festzustellen.

TABELLE 2

| Viskosität und pH-Wert des amphoteren kationischen Polyelektrolyten (32 Gew.%ig in $H_2O$) | | Zugabemenge in Gew.% bez. auf trockenen Marmor | Viskosität der Suspension | |
|---|---|---|---|---|
| Viskosität | pH-Wert | | sofort | nach 10 Tg. |
| 106 mPas | 3,7 | 0,1 | 465 | |
| | | 0,2 | 260 | |
| | | 0,3 | 200 | 345 |
| 44 mPas | 3,7 | 0,1 | 535 | |
| | | 0,2 | 220 | |
| | | 0,3 | 140 | 375 |
| 33 mPas | 3,7 | 0,1 | 1090 | |
| | | 0,2 | 750 | |
| | | 0,3 | 570 | |
| | | 0,4 | 430 | |
| | | 0,5 | 290 | |
| | | 0,6 | 200 | 530 |

<u>Beispiel 4 :</u>

Eine 67 Gew.%-ige wäßrige Aufschlämmung von natürlichem Marmor mit einer Kornverteilung, so daß 60 Gew.% der Teilchen einen äquivalent sphärischen Durchmesser < 2 µm aufweisen (gemessen auf dem Sedigraph 5100), wurde mit unterschiedlichen Mengen, bezogen auf den trockenen Marmor, des folgenden Copolymers hergestellt,

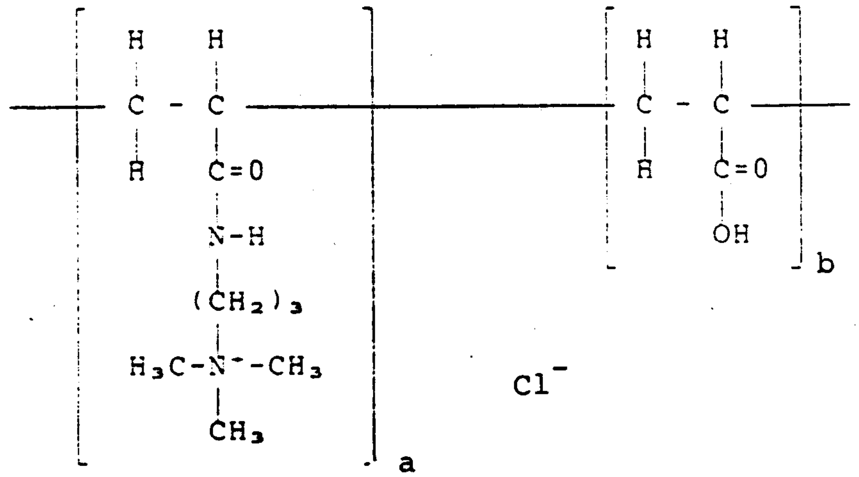

a= 47 Mol.%
b= 53 Mol.%

wobei das Copolymer zusätzlich im Molekulargewicht resp. Eigenviskosität der 32 Gew.%-igen wäßrigen Lösung variiert wurde. Es wurde unter starkem Rühren dispergiert (8000 U/min Rührscheibendurchmesser 50 mm).

Das Ziel dieser Versuchsserie war es, die optimale Viskosität bzw. das Molekulargewicht der amphoteren, leicht anionischen Polyelektrolyten festzustellen, sowie die optimale Dispergiermittelmenge festzustellen.

TABELLE 3

| Viskosität und pH-Wert des amphoteren leicht anionischen Polyelektrolyten (32 Gew.%ig in $H_2O$) | | Zugabemenge in Gew.% bez. auf trockenen Marmor | Viskosität der Suspension | |
|---|---|---|---|---|
| Viskosität | pH-Wert | | sofort | nach 10 Tg. |
| 84 mPas | 3,1 | 0,1 | 1500 | |
| | | 0,2 | 840 | |
| | | 0,3 | 420 | |
| | | 0,4 | 275 | |
| | | 0,5 | 185 | 165 |
| 40 mPas | 3,0 | 0,1 | 1180 | |
| | | 0,2 | 265 | |
| | | 0,3 | 165 | 190 |

Beispiel 5 :

Eine 72 Gew.%-ige wäßrige Aufschlämmung von natürlichem Marmor mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas ( ∅ 1 mm) auf eine Kornverteilungskurve, so daß 70 Gew.% der Teilchen einen äquivalenten sphärischen Durchmesser < 2 µm (gemessen auf dem Sedigraph 5100) aufwiesen, aufgemahlen.

Rezeptur :

5000 g Marmor
25 g amphoteres Copolymer
entsprechend der Formel des Beispiels 2

| | |
|---|---|
| | (Viskosität 37 mPas) |
| 1925 g | Wasser zugeben |

| Viskosität : | nach 2 Std. | 1 Tag | 5 Tagen | 8 Tagen | 16 Tagen | nach 30 Tagen |
|---|---|---|---|---|---|---|
| | 215 | 255 | 300 | 365 | 430 | 515 mPas |
| Oberflächenladung nach 7 Tagen + 7,9 µVal/g Feststoff | | | | | | |

In Beispiel 5 ist deutlich zu erkennen, daß beim erfindungsgemäßen Einsatz der erfindungsgemäßen amphoteren Polyelektrolyten bei hohen Konzentrationen eine sehr tiefe und über Wochen ausreichend stabile Viskosität auch bei durch Mahlen erzeugten feinteiligen Mineral- und/oder Füllstoff- und/oder Pigment Suspensionen herzustellen ist.

Beispiel 6 :

Eine 72 Gew.%-ige wäßrige Aufschlämmung von Champagnekreide mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 90 Gew.% der Teilchen einen äquivalenten sphärischen Durchmesser < 2 µm (gemessen auf dem Sedigraph 5100) aufwiesen, aufgemahlen.

Rezeptur :

| | |
|---|---|
| 5000 g | Champagnekreide |
| 60 g | amphoteres Copolymer aus Beispiel 2 (Viskosität 37 mPas) |
| 1715 g | Wasser zugeben |

| Viskosität : in mPas: | nach 1 Std. | 1 Tag | 10 Tagen | 20 Tage |
|---|---|---|---|---|
| | 600 | 650 | 710 | 900 |

Beispiel 7 :

Eine 72 Gew.%-ige wäßrige Aufschlämmung von natürlichem Marmor mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 90 Gew.% der Teilchen einen äquivalenten sphärischen Durchmesser < 2 µm (gemessen auf dem Sedigraph 5100) aufwiesen, aufgemahlen.

Rezeptur :

| | |
|---|---|
| 5000 g | Marmor |
| 55 g | amphoteres Polymer aus Beispiel 2 |
| 1418 g | Wasser zugeben |

| Viskosität in mPas: | nach 1 Std. | 1 Tag | 10 Tagen | 20 Tage |
|---|---|---|---|---|
| | 740 | 780 | 870 | 980 |
| Oberflächenladung nach 7 Tagen + 10,1 µVal/g Feststoff | | | | |

Beispiel 7a :

Im Pilot-plant-Maßstab wurde der im Beispiel 7 verwendete Marmor in einer vertikal angeordneten Permill (Süssmeier mit 180 Liter Inhalt) mit Mahlkörpern aus Glas (∅ 1-2 mm) auf eine Kornverteilungskurve, so daß 90 Gew.-% der Teilchen einen äquivalent sphärischen Durchmesser < 2 µm (gemessen auf dem Sedigraph 5100) aufwiesen, bei einer Konzentration von 74,5 Gew.-% vermahlen. Es wurden ca. 2 to dieser Slurry hergestellt.

Rezeptur:

| | |
|---|---|
| 1480 kg | Marmor |
| 10,4 kg | amphoteres Polymer aus Beispiel 2 |
| 510 kg | Wasser zugeben |

| Viskosität in mPas: | nach 1 Std. | 1 Tag | 10 Tagen | 20 Tage |
|---|---|---|---|---|
| | 600 | 560 | | 680 |
| Oberflächenladung nach 7 Tagen + 11,9 µVal/g Feststoff | | | | |

Beispiele 6 und 7 und 7a zeigen, daß auch sehr hohe Feinheiten, wie sie in Streichrezepturen Verwendung finden, problemlos durch Mahlen von grob gebrochenem Rohgestein bei hohen Konzentrationen herstellbar sind.

Beispiel 8 :

Eine 67 Gew.%-ige wäßrige Aufschlämmung von natürlichem Marmor mit einem äquivalent sphärischen mittleren Teilchendurchmesser von 12 µm (gemessen auf dem Sedigraph 5100) wurden mit der folgenden Rezeptur in einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 µm (gemessen auf dem Sedigraph 5100) aufwiesen, aufgemahlen.

Rezeptur :

| | |
|---|---|
| 5000 g | Marmor |
| 20 g | amphoteres Copolymer entsprechend der Formel des Beispiel 2 (Viskosität 37 mPas) |
| 2472 g | Wasser zugeben |

| Viskosität in mPas: | nach 2 Std. | 1 Tag | 5 Tagen | 8 Tagen | 16 Tagen | nach 30 Tagen |
|---|---|---|---|---|---|---|
| | 120 | 130 | 140 | 212 | 208 | 520 |
| Oberflächenladung nach 7 Tagen + 4,8 µVal/g Feststoff. | | | | | | |

In Beispiel 8 ist deutlich zu erkennen, daß beim erfindungsgemäßen Einsatz von amphoteren, gegen außen neutrale Polyelektrolyten bei hohen Konzentrationen eine sehr tiefe und über Wochen ausreichend stabile Viskosität auch bei durch Mahlen erzeugten feinteiligen Mineral- und/oder Füllstoff- und/oder Pigment Suspensionen herzustellen ist, wie sie als Füllstoff für die Papiererzeugung eingesetzt werden.

Beispiel 9 :

Eine 72 Gew.%-ige wäßrige Aufschlämmung von natürlichem Marmor mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 µm (gemessen auf dem Sedigraph 5100) aufwiesen, aufgemahlen.

Rezeptur :

5000 g Marmor
25 g amphoteres Polymer
aus Beispiel 5 wobei bei a) 95 Mol.% der Carboxylgruppen mit $Ca(OH)_2$ neutralisiert und bei b) 95 Mol.% mit $Mg(OH)_2$ neutralisiert wurden.
2460 g Wasser zugeben

| Viskosität in mPas: | | nach 1 Std. | 1 Tag | 4 Tagen | 8 Tagen | 16 Tagen |
|---|---|---|---|---|---|---|
| | a) | 96 | 110 | 130 | 140 | 160 |
| | b) | 104 | | | | 155 |

Beispiel 9 zeigt, daß die erfindungsgemäße Calcium- und/oder Magnesiumneutralisation der Carboxylgruppen im amphoteren Polyelektkrolyten trotz erheblich höherem Feststoffgehalt und nur gering mehr Dispergiermittel gegenüber dem Beispiel 8 nochmals bessere Viskosität bringt als derselbe nicht neutralisierte amphotere Polyelektrolyt.

Beispiel 10 :

Eine 67 Gew.%-ige wäßrige Aufschlämmung von natürlichem Marmor mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 µm (gemessen auf dem Sedigraph 5100) aufwiesen, aufgemahlen.

Rezeptur :

5000 g Marmor
17,5 g amphoteres kationisches Copolymer der folgenden Verbindung

2,5 g amphoteres Copolymer, wobei die anionischen und kationischen Gruppen im Verhältnis 1:1 vorliegen analog Beispiel 2 mit einer Viskosität von 37 mPas vor der Mahlung zugegeben
2,5 g "wie die ersten 2,5 g" während der Vermahlung zugegeben
2472 g Wasser

| Viskosität : | nach 2 Std. | 1 Tag | 4 Tagen | 8 Tagen | 16 Tagen | 30 Tagen |
|---|---|---|---|---|---|---|
| | 450 cP | 450cP | 520cP | 615cP | 730cP | 830cP |

Das Beispiel 10 zeigt, daß mit der erfindungsgemäßen Kombination von den erfindungsgemäßen amphoteren Polyelektrolyten eine transportfähige, nicht sedimentierende Aufschlämmung von Calciumcarbonat durch Mahlen von

grob gebrochenem Stein möglich ist. Die Viskosität bei hoher Konzentration ist gut.

Die in den Herstellungsbeispielen 5 + 8 hergestellten Marmorslurries wurden im Vergleich zu einem, heute üblicherweise mit anionischen Dispergiermitteln hergestellten Marmorslurry auf ihre Retention bei der Papierherstellung untersucht.

Testbedingungen:

| | | |
|---|---|---|
| Stoff: | 80 % Birkensulfat | Mahlgrad 23° SR |
| | 20 % Kiefersulfat | |
| Retentionshilfsmittel: | | 0,05 % Polyacrylamid (Grenzviskosität 700 ml/g) |

Durchführung der Retentionsuntersuchung nach Britt-Jar Firma Paper Research Material, SYRACUSE, U.S.A.:

1. 275 mlg 2%-ige Fasersuspension (atro 3,63 g Fasern) sowie 275 ml des. Wasser ins Britt-Jar Gefäß geben;
2. Britt-Jar Rührer auf 700 U/min;
3. 25,4 ml 5%-ige Mineral- und/oder Füllstoff- und/oder Pigmentsuspension zugeben;
4. nach 20 sec. die entsprechende Menge Retentionsmittel zugeben;
5. nach weiteren 25 sec. Ablaufhahn öffnen und 100 ml Siebwasser ablaufen lassen.
6. Im Siebwasser wird der $CaCO_3$-Anteil komplexometrisch nach aufschließen mit HCl oder mit Flammen-AAS bestimmt. Bei anderen Mineralien und/oder Füllstoffen und/oder Pigmenten wird das Siebwaser über Membranfilter filtriert, verascht bei 600 °C, über einen alkalischen Schmelzaufschluß, z. B. mit NaOH/KOH im Zirkontiegel, in eine wasserlösliche Form gebracht und im angesäuerten Zustand mittels AAS bestimmt. Unter Berücksichtigung der entsprechenden Umrechnungsfaktoren kann auf die jeweiligen Mineralien und/oder Füllstoffe und/oder Pigmente geschlossen werden.
7. Über den eingetragenen Anteil an Mineralien und/oder Füllstoffen und/oder Pigmenten pro 100 ml und den im Siebwasser bestimmten Anteil an Mineralien und/oder Füllstoffen und/oder Pigmenten pro 100 ml läßt sich die Füllstoffretention berechnen.

| Resultate: | |
|---|---|
| Produkte: | Füllstoff-First-Pass-Retention |
| Anionisch stabilisierte $CaC0_3$-Suspension mit 60 % < 2 μm (0,15 % Natriumpolyacrylat spez. Visk. 0.35) | 41,1 % |
| Anionisch stabilisierte $CaC0_3$-Suspension mit 70 % < 2 μm (0,3% Natriumpolyacrylat spez. Visk. 0.54) | 35,3 % |
| $CaC0_3$-Suspension aus Herstellungsbeispiel 8 | 62,4 % |
| $CaC0_3$-Suspension aus Herstellungsbespiel 5 | 65,8 % |

Durch den Einsatz eines mit dem erfindungsgemäßen, neuen Herstellungsverfahren produzierten Marmorsuspension ist eine Steigerung der Füllstoffretention möglich ohne die Papierformation und Papierfestigkeit negativ zu beeinträchtigen, was einen enormen Sprung in der Entwicklung darstellt.

Die erfindungsgemäßen wäßrigen Suspensionen sowie das erfindungsgemäße Verfahren zu ihrer Herstellung weisen u.a. folgende Vorteile auf:

- Im Gegensatz zu den bis heute bekannten Verfahren ist es möglich, hochkonzentrierte (≥ 60 Gew.%) Mineral- und/oder Füllstoff- und/oder Pigment-Suspension durch Naßvermahlung aus grob gebrochenem Rohstein herzustellen.
- Ein Füllstofferhöhung ohne nennenswerten Abfall der Reißfestigkeit des Papiers ist möglich, wodurch sich ein enormer ökonomischer Vorteil in der Papierfabrikation ergibt. Weiterhin ergab sich mit der erfindungsgemäßen Zusammensetzung, daß eine Füllgraderhöhung von 15 Gew.% auf 17 Gew.% ohne nennenswerte Einbuße an

Papierfestigkeit, speziell Reißfestigkeit, möglich ist. Neueste Praxisversuche haben gezeigt, daß eine Füllstofferhöhung von 16 % auf 26 % erreichbar ist, ohne die Papiereigenschaften negativ zu beeinflussen.

- Die Suspensionen weisen eine ausgezeichnete Lagerstabilität bei tiefen Viskositäten ohne Sedimentationsproblem auf.
- In der Anwendung ergeben sich z.B. große Vorteile bezüglich der Füllstoffretention in der Papiererzeugung.
- Die Mahlung und Dispergierung ist unter hohen Mahlkräften und bei Siedetemperatur des Wassers möglich.
- Die ökologisch günstigste Transportmöglichkeit kann gewählt werden.

Eine vorzugsweise Ausführungsform der Erfindung ist dadurch gekennzeichnet, daß das Dispergiermittel eine Mischung aus einem oder mehreren kationischen Polyelektrolyten und/oder einem oder mehreren amphoteren kationischen Polyelektrolyten, bei welchen die nichtneutralen Monomereinheiten überwiegend positive Ladungen tragen, und einem oder mehreren teilneutralisierten anionischen Polyelektrolyten und/oder einem oder mehreren teilneutralisierten amphoteren anionischen Polyelektrolyten, bei welchen die nichtneutralen Monomereinheiten überwiegend negative Ladungen tragen, ist.

Im folgenden werden die teilneutralisierten anionischen bzw. kationischen Polyelektrolyte und die teilneutralisierten amphoteren anionischen bzw. kationischen Polyelektrolyte kurz als erfindungsgemäße anionische bzw. erfindungsgemäße kationische Polyelektrolyte bezeichnet.

Vorteilhafterweise ist das Dispergiermittel eine Mischung aus einem oder mehreren homopolymeren kationischen Polyelektrolyten und/oder einem oder mehreren copolymeren amphoteren kationischen Polyelektrolyten, bei welchen die nichtneutralen Monomereinheiten überwiegend positive Ladungen tragen, und einem oder mehreren homo- und/oder copolymeren teilneutralisierten anionischen Polyelektrolyten und/oder einem oder mehreren amphoteren anionischen teilneutralisierten Polyelektrolyten, bei welchen die nichtneutralen Monomereinheiten überwiegend negative Ladungen tragen.

Vorteilhafterweise trägt der kationische Polyelektrolyt und/oder amphotere kationische Polyelektrolyt, bei welchen die nichtneutralen Monomareinheiten überwiegend positive Ladungen tragen, die die positive Ladung erzeugende funktionelle Gruppe in einem Substituenten der ethylenischen Hauptkette.

Weiterhin vorteilhaft ist, daß der Substituent über

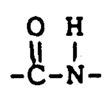

oder

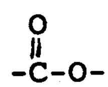

an der Hauptkette gebunden ist.
Weiterhin vorteilhaft ist, daß der kationische Polyelektrolyt quaternäre Ammoniumgruppen enthält und daß der amphotere kationische Polyelektrolyt, bei welchem die nichtneutralen Monomereinheiten überwiegend positive Ladungen tragen, quaternäre Ammoniumgruppen und Carboxylgruppen und/oder Sulfonsäuregruppen und/oder saure phosphorsäureesterhaltige Gruppen enthält.

Insbesondere vorteilhaft ist, daß der kationische Polyelektrolyt eine oder mehrere Verbindungen aus der Gruppe der folgenden Verbindungen gemäß der folgenden allgemeinen Formel ist

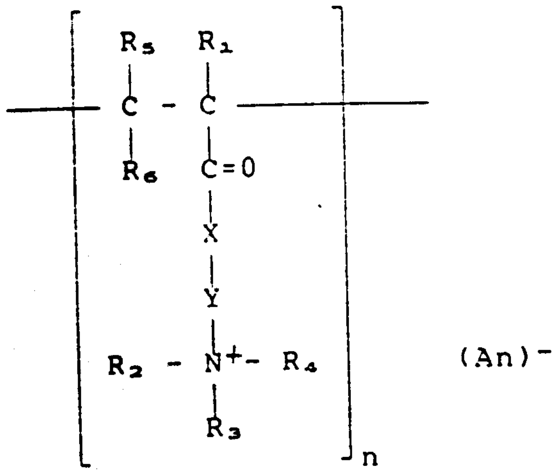

wobei $R_1$, $R_5$ und $R_6$ = H

- und/oder $R_1$ bis $R_6$
- Alkyl und/oder
- Aryl,

wobei $R_5$ auch

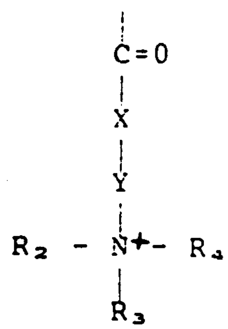

X = O und/oder N-H
Y = $-CH_2-$ bis $-C_5H_{10}-$
n = 20 bis 3000

und $(An)^-$ = Chlorid und/oder Bromid und/oder Jodid und/oder $HSO_4^-$ und/oder $CH_3SO_4^-$ und/oder Nitrit sein kann.

Ganz besonders vorteilhaft ist, wenn gemäß dieser allgemeinen Formel

$R_1$ = H oder $-CH_3$
$R_2 = -CH_3$ oder $-C_2H_5$
$R_3 = -CH_3$ oder $-C_2H_5$
$R_4 = -CH_3$ bis $-C_4H_9$ und Isomere
X = O oder N - H
Y = $-CH_2-$bis $-C_5H_{10}-$

$R_5$ und $R_6$ = H,

insbesondere, wenn Y = $-(CH_2)_3-$ und

X = -NH ist.

Vorteilhafterweise ist der amphotere kationische Polyelektrolyt, bei welchem die nichtneutralen Monomereinheiten überwiegend positive Ladungen tragen, eine oder mehrere Verbindungen aus der Gruppe der folgenden Verbindungen gemäß der folgenden allgemeinen Formel

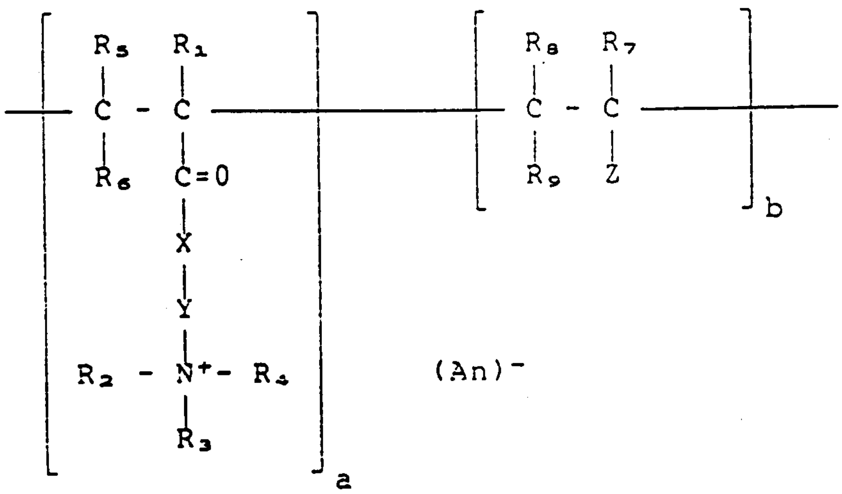

wobei $R_1$,$R_5$, $R_6$ und $R_7$ = H

- und/oder $R_1$ bis $R_7$
- Alkyl und/oder
- Aryl,

wobei $R_5$ auch

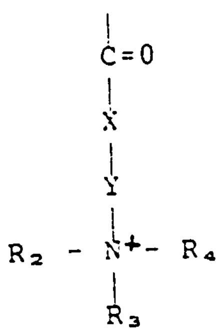

sein kann,
$R_8$ und $R_9$ =

- H und/oder
- Alkyl und/oder
- Aryl sein kann;

$R_8$ oder $R_9$ auch

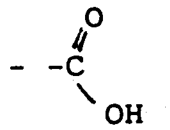

sein kann,
wenn

Z =

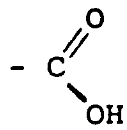

ist,

X = O und/oder N-H
Y = $-CH_2-$ bis $-C_5H_{10}-$

Z =

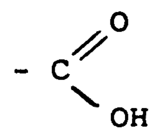

und/oder

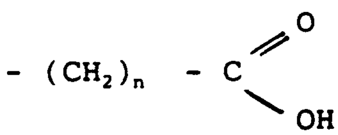

und/oder

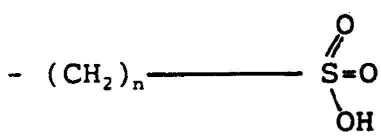

und/oder

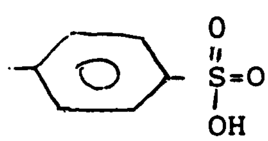

und/oder

eine saure Phosphorsäureester-Gruppe
sein kann.
a = 70 - 99 Mol.%
b = 1 - 30 Mol.%
n = 1 - 18

und $(An)^-$ = Chlorid und/oder Bromid und/oder Jodid und/oder $HSO_4^-$ und/oder $CH_3SO_4^-$ und/oder Nitrit sein kann.

Insbesondere vorteilhaft ist, daß der amphotere kationische Polyelektrolyt eine Verbindung gemäß dieser allgemeinen Formel ist, wobei

$R_1$ = H oder $-CH_3$
$R_2 = -CH_3$ oder $-C_2H_5$
$R_3 = -CH_3$ oder $-C_2H_5$
$R_4 = -CH_3$ bis $-C_4H_9$ und Isomere
X = O oder N - H
Y = $-CH_2$-bis - $C_5H_{10}$-
$R_5$ und $R_6$ = H
$R_7$ = H oder $-CH_3$
$R_8$ und $R_9$ = H.

Ganz besonders vorteilhaft ist, wenn $(An)^-$ = $Cl^-$ und Y = $-(CH_2)_3-$ ist.

Weiterhin vorteilhaft ist, wenn der anionische teilneutralisierte Polyelektrolyt eine oder mehrere Verbindungen aus der Gruppe der folgenden Verbindungen gemäß der folgenden allgemeinen Formel ist

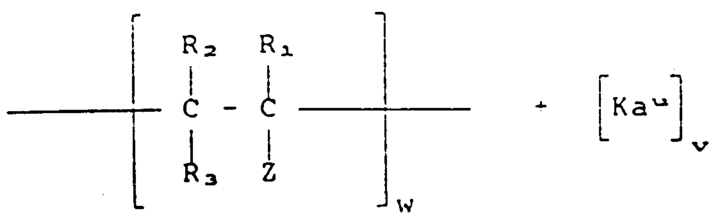

Z = 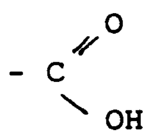 oder 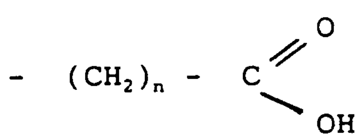

und/oder

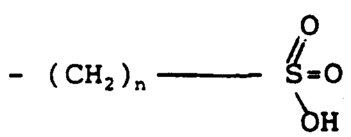 und/oder

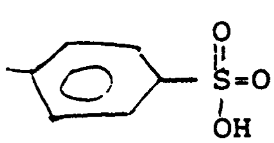 und/oder

eine saure phosphorsäureester-Gruppe

$R_1$ = - H oder - $CH_3$

$R_2$ und $R_3$ =

- H und/oder
- Alkyl und/oder
- Aryl

wobei $R_2$ oder $R_3$ auch Z sein kann, wenn

Z = 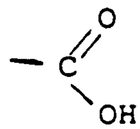

u = +I und/oder +II und/oder +III
Ka = Alkali- und/oder Erdalkali und/oder Erdmetallion
w = 59 bis 95 Mol.% pro Anzahl Z im Monomer
v = 5 bis 41 Mol.% geteilt durch u
n = 1 - 12.

Weiterhin ist vorteilhaft, daß der teilneutralisierte anionische Polyelektrolyt eine Mischung aus einem oder mehreren der Homo- und/oder Copolymerisate von Verbindungen gemäß dieser allgemeinen Formel ist.

Weiterhin vorteilhaft ist, daß der amphotere anionische teilneutralisierte Polyelektrolyt, bei welchem die nicht neutralen Monomereinheiten überwiegend negative Ladungen tragen, eine oder mehrere Verbindungen aus der Gruppe der Verbindungen gemäß der folgenden allgemeinen Formel ist:

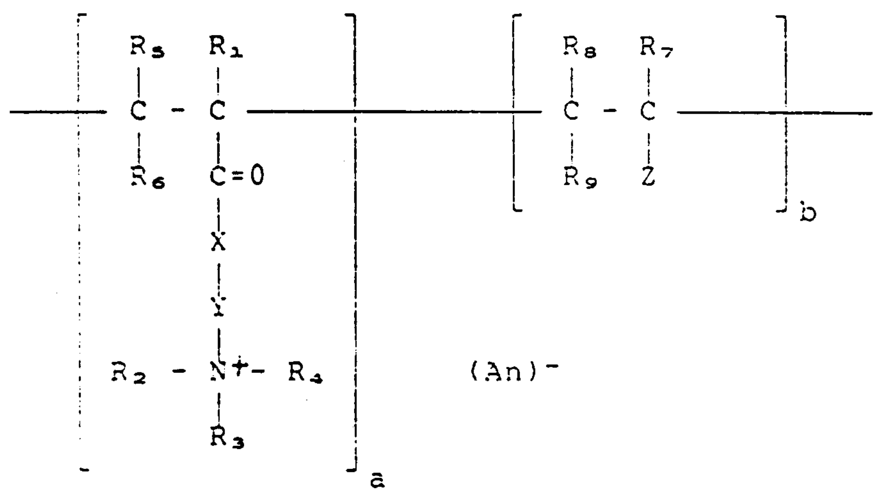

wobei $R_1$, $R_5$, $R_6$ und $R_7$ = H

- und/oder $R_1$ bis $R_7$
- Alkyl und/oder
- Aryl,

wobei $R_5$ auch

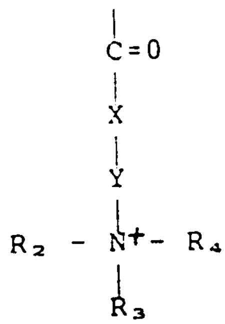

sein kann,
$R_8$ und $R_9$ =

- H und/oder
- Alkyl und/oder
- Aryl sein kann;

$R_8$ oder $R_9$ auch

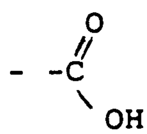

sein kann,
wenn

Z =

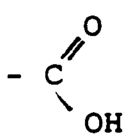

ist,

X = O und/oder N-H
Y = $-CH_2-$ bis $-C_5H_{10}-$

Z = 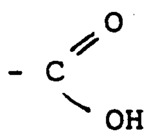 und/oder

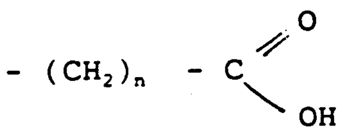 und/oder

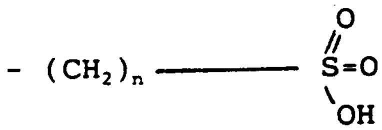 und/oder

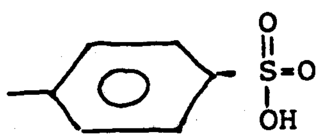 und/oder

eine saure Phosphorsäureester-Gruppe sein kann.

a = 1 - 30 Mol.%
b = 70 - 99 Mol.%
n = 1 - 18

und $(An)^-$ = Chlorid und/oder Bromid und/oder Jodid und/oder $HSO_4^-$ und/oder $CH_3SO_4^-$ und/oder Nitrit sein kann.

Insbesondere vorteilhaft ist, daß der amphotere anionische teilneutralisierte Polyelektrolyt eine oder mehrere Verbindungen gemäß dieser allgemeinen Formel ist, wobei

$R_1$ = H oder $-CH_3$
$R_2$ = $-CH_3$ oder $-C_2H_5$
$R_3$ = $-CH_3$ oder $-C_2H_5$
$R_4$ = $-CH_3$ bis $-C_4H_9$ und Isomere
X = O oder N - H
Y = $-CH_2$-bis - $C_5H_{10}$-
$R_5$ und $R_6$ = H
$R_7$ = H oder $-CH_3$
$R_8$ und $R_9$ = H,
sind.

Ganz besonders vorteilhaft ist, wenn $(An)^-$ = $Cl^-$ und Y = $-(CH_2)_3-$ ist.

Eine weitere günstige Ausführungsform der Erfindung ist es, daß der anionische teilneutralisierte Polyelektrolyt ein homo-und/oder copolymerer, und daß der amphotere anionische teilneutralisierte Polyelektrolyt, bei welchem die nichtneutralen Monomereinheiten überwiegend negative Ladungen tragen, je ein Carboxylgruppen- und/oder Sulfonsäuregruppen- und/oder saure Phosphorsäureestergruppenenthaltender teilneutralisierter anionischer Polyelektrolyt ist.

Insbesondere ist es günstig, daß der teilneutralisierte anionische Polyelektrolyt eine teilneutralisierte Polyacrylsäure und/oder eine teilneutralisierte Polymethacrylsäure und/oder ein teilneutralisiertes Copolymer aus diesen ist.

Vorteilhafterweise sind beim anionischen teilneutralisierten Polyelektrolyt und beim amphoteren anionischen teilneutralisierten Polyelektrolyt nur ein statistischer Teil der Säuregruppen mit einem ein- und/oder mehrwertigen Kation neutralisiert.

Günstigerweise werden als Kationen Alkali- und/oder Erdalkali-und/oder Erdmetallkationen und/oder Amine und/oder Alkanolamine und/oder quaternäre Ammonium-Kationen verwendet, wobei insbesondere vorteilhaft als Kationen $Na^+$ und/oder $K^+$ und/oder $Li^+$ und/oder $NH_4^+$ und/oder $Ca^{2+}$ und/oder $Mg^{2+}$ und/oder $Sr^{2+}$ verwendet werden. Ganz besonders gute Ergebnisse werden erhalten, wenn als Kationen Alkali- und/oder Erdalkalikationen verwendet werden, insbesondere Alkalikationen und hier insbesondere $Na^+$. $NH_4^+$ ist insbesondere ungeeignet, da es zu starker Geruchsbelästigung und Gesundheitsschädigung führt.

Dispergiermittel, die sich erfindungsgmäß besonders eignen, sind Mischungen gemäß den allgemeinen Formeln des Anspruchs 14 und/oder der amphoteren kationischen Polyelektrolyten des Anspruchs 15 und des Anspruchs 16 und/oder der amphoteren anionischen teilneutralisierten Polyelektrolyten des Anspruchs 15.

Besonders günstig ist eine Dispergiermittelmischung gemäß der folgenden allgemeinen Formel

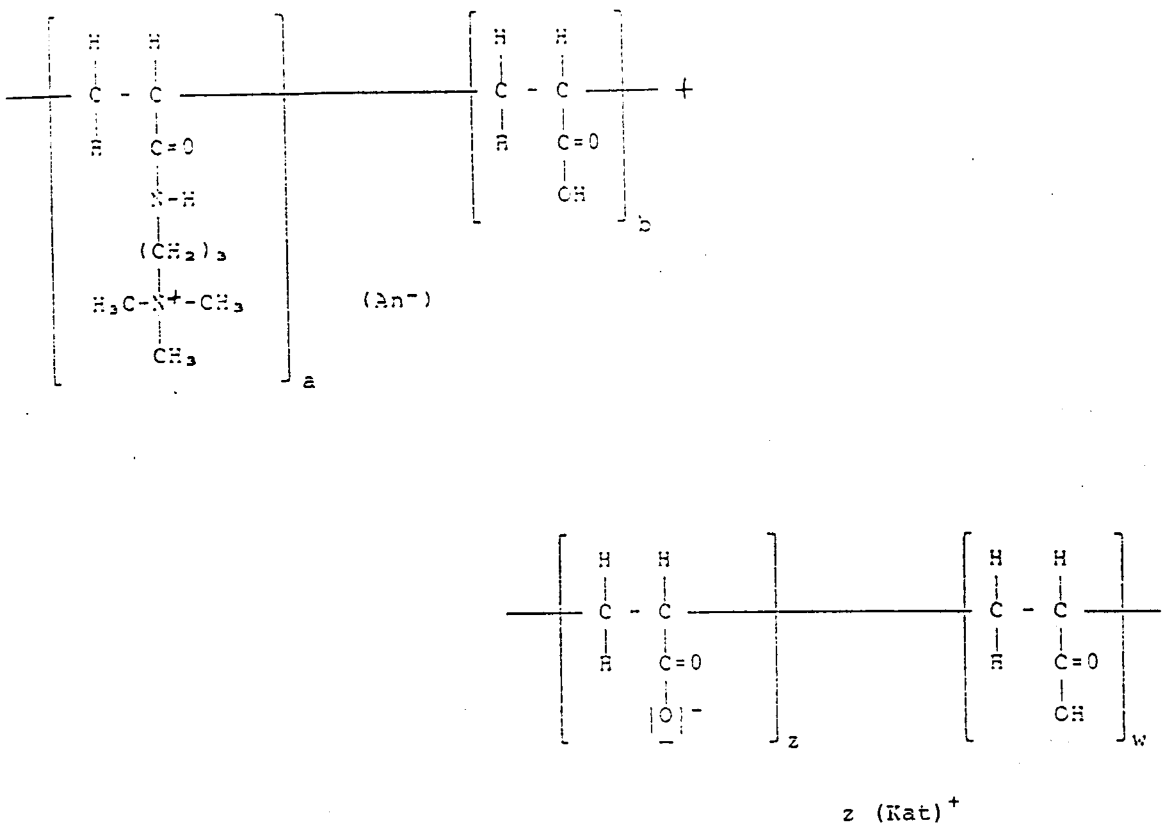

wobei

$(Kat)^+$ = Alkali- und/oder Erdalkali- und/oder Erdmetallkationen und/oder Amine und/oder Alkanolamine und/oder quaternäre Ammonium-Kationen

$(An)^-$ = Chlorid, Bromid, Jodid, $HSO_4^-$, $CH_3SO_4^-$ und/oder Nitrit

a = 60 - 99 Mol.%
b = 1 - 40 Mol.%
z = 1 - 70 Mol.%
w = 30 - 99 Mol.%.

Besonders vorteilhaft sind Dispergiermittelmischungen gemäß dieser allgemeinen Formel, wobei

$(Kat)^+$ = Alkali- und/oder Erdalkalikationen

$(An)^-$ = Chlorid, Bromid, Jodid, $HSO_4^-$, $CH_3SO_4^-$ und/oder Nitrit

a = 80 - 98 Mol.%
b = 2 - 20 Mol.%
z = 2 - 50 Mol.%
w = 50 - 98 Mol.%.

Weiterhin vorteilhaft sind Dispergiermittelmischungen gemäß dieser allgemeinen Formel, wobei

$(Kat)^+$ = $Na^+$, $K^+$, $Li^+$, $Ca^{2+}$, $Mg^{2+}$, und/oder $Sr^{2+}$
$(An)^-$ = Chlorid, Bromid, Jodid, $HSO_4^-$, $CH_3SO_4^-$ und/oder Nitrit

a = 85 - 97 Mol.%
b = 3 - 15 Mol.%
z = 3 - 30 Mol.%
w = 70 - 97 Mol.%.

Ganz besonders günstige Ergebnisse werden erzielt, wenn die Dispergiermittelmischung eine Mischung dieser allgemeinen Formel ist, wobei

$(Kat)^+$ = Alkaliion
$(An)^-$ = Halogenion

a = 90 - 96 Mol.%
b = 4 - 10 Mol.%
z = 4 - 20 Mol.%
w = 80 - 96 Mol.%.

Hervorragende Ergebnisse werden erzielt, wenn die Dispergiermittelmischung eine Mischung gemäß dieser allgemeinen Formel ist, wobei

$(Kat)^+$ = $Na^+$
$(An)^-$ = $Cl^-$

a = 95 Mol.%
b = 5 Mol.%
z = 5 Mol.%
w = 95 Mol.%.

Vorteilhafterweise sind der anionische Polyelektrolyt und/oder der amphotere anionische Polyelektrolyt mit Alkali-, und/oder Erdalkali und/oder Erdmetall-Kationen und/oder Aminen und/oder Alkanolaminen und/oder quaternären Ammonium-Kationen teilneutralisiert, wobei insbesondere Alkali- und/oder Erdalkali-Kationen, ganz besonders Alkali-Kationen und hier insbesondere $Na^+$-Kationen geeignet sind.

Günstigerweise sind beim anionischen Polyelektrolyten und/oder beim amphoteren anionischen Polyelektrolyten 1 bis 70 Mol.% der Säuregruppen neutralisiert. Besonders günstige Ergebnisse werden erzielt, wenn 2 bis 60 Mol.%, insbesondere wenn 3 bis 30 Mol.% der Säuregruppen neutralisiert sind, wobei ein Neutralisationsgrad von 5 Mol.% bis 10 Mol.% beste Ergebnisse erzielt.

Nichtneutralisierte Polyacrylsäure ist ungeeignet, da sie bei + 20°C bereits zu kristallisieren beginnt und somit nicht mehr dosierbar ist.
Wenn die Kristallisation einmal eingetreten ist, muß die Polymerlösung auf 100°C aufgeheizt werden, damit die Kristalle wieder aufgelöst werden.
Im Winter und in kälteren Regionen ist eine Produktion mit nichtneutralisierten Polyacrylsäuren undenkbar.

Es ist vorteilhaft, daß die spezifische Viskosität "Eta" des teilneutralisierten anionischen Polyelektrolyten und/oder des amphoteren anionischen Polyelektrolyten in der Mischung mit dem kationischen und/oder dem amphoteren kationischen Polyelektrolyten, gemessen in der vollen Salzform, zwischen 0,2 und 1,0 liegt. Insbesondere vorteilhaft ist, daß "Eta" zwischen 0,35 und 0,6 liegt und es ist ganz besonders günstig, wenn "Eta" 0,55 beträgt.

Es ist vorteilhaft, daß der Polymerisationsgrad des kationischen Polyelektrolyten und/oder des amphoteren kationischen Polyelektrolyten in der Mischung mit dem teilneutralisierten anionischen Polyelektrolyten und/oder dem amphoteren anionischen teilneutralisierten Polyelektrolyten, gemessen über die Grenzviskosität, im Bereich von 5 ml/g bis 50 ml/g liegt. Ganz besonders vorteilhaft ist ein Polymerisationsgrad im Bereich von 15 ml/g bis 40 ml/g, wobei ein

Bereich von 25 ml/g bis 35 ml/g insbesondere bevorzugt wird.

Vorteilhafterweise besteht die Dispergiermittelmischung aus

70 - 98 Gew.% kationischem Polyelektrolyt und/oder amphoterem kationischen Polyelektrolyt und
2 - 30 Gew.% anionischem teilneutralisierten Polyelektrolyt und/oder amphoterem teilneutralisierten anionischen Polyelektrolyt.

Weiterhin vorteilhaft kann eine Dispergiermittelmischung aus

75 - 95 Gew.% der erfindungsgemäßen kationischen Polyelektrolyten und 5 - 25 Gew.% der erfindungsgemäßen anionischen Polyelektrolyten verwendet werden. Weiterhin günstig sind Dispergiermittelmischungen aus 80 - 90 Gew.% der erfindungsgemäßen kationischen Polyelektrolyten und 10 bis 20 % der erfindungsgemäßen anionischen Polyelektrolyten. Ganz besonders vorteilhaft geeignet sind Dispergiermittelmischungen aus 80 bzw. 90 Gew.% der erfindungsgemäßen kationischen Polyelektrolyten und 20 bzw. 10 Gew.% der erfindungsgemäßen anionischen Polyelektrolyten.

Vorteilhafterweise beträgt das Mischungsverhältnis von kationischem Polyelektrolyt zu amphoterem kationischen Polyelektrolyt in der Mischung mit dem teilneutralisierten anionischen und/oder dem teilneutralisierten amphoteren anionischen Polyelektrolyt 0 - 100 Gew.% kationischer Polyelektrolyt und 100 - 0 Gew.% amphoterer kationischer Polyelektrolyt. Weiterhin bevorzugt ist ein Mischungsverhältnis von 0 bis 30 Gew.% kationischem Polyelektrolyt und 70 bis 100 Gew.% amphoterer kationischer Polyelektrolyt, insbesondere ein Mischungsverhältnis von 0 bis 20 Gew.% kationischer Polyelektrolyt und 80 bis 100 Gew.% amphoterer kationischer Polyelektrolyt.

Vorzugsweise beträgt die molare Zusammensetzung der einzelnen Komponenten im teilneutralisierten anionischen Polyelektrolyten in der Mischung mit dem kationischen und/oder amphoteren kationischen Polyelektrolyten zwischen 0 Mol.% bis 100 Mol.% Acrylsäure und 100 Mol.% bis 0 Mol.% anderer Monomere. Günstigerweise sind die anderen Monomere carboxylgruppenhaltig und/oder sulfonsäuregruppenhaltig und/oder saure phosphorsäureestergruppenhaltig.

Insbesondere günstig ist, daß die molare Zusammensetzung der einzelnen Komponenten im teilneutralisierten anionischen amphoteren Polyelektrolyten in der Mischung mit dem kationischen und/oder amphoteren kationischen Polyelektrolyten zwischen 0 Mol.% bis 99 Mol.% Acrylsäure und 100 % bis 1 Mol.% anderer Monomere liegt.

Ganz besonders günstige Ergebnisse werden erzielt, wenn die anderen Monomere carboxylgruppenhaltig und/oder sulfonsäuregruppenhaltig und/oder saure phosphorsäureestergruppenhaltig und/oder eine oder mehrere Verbindungen aus der Gruppe der Verbindungen gemäß der allgemeinen Formel des Anspruchs 6 sind.

Ganz besonders vorteilhaft ist, daß der anionische Polyelektrolyt teilneutralisierte Acrylsäure ist. Vorteilhafterweise sind 2 - 80 Mol.% der Säuregruppen des anionischen Polyelektrolyten neutralisiert, insbesondere vorteilhaft 3 bis 70 Mol.% und ganz besonders vorteilhaft 3 bis 10 Mol.% der Säuregruppen.

Erfindungsgemäß enthalten die Mineralien bzw. Füllstoffe bzw. Pigmente insbesondere Elemente aus der zweiten und/oder dritten Hauptgruppe und/oder aus der vierten Nebengruppe des Periodensystems der Elemente enthalten. Günstigerweise werden calcium- und/oder siliziumhaltige und/oder aluminiumhaltige und/oder titanhaltige Mineralien und/oder Füllstoffe und/oder Pigmente verwendet, wobei calciumcarbonathaltige Mineralien und/oder Füllstoffe und/oder Pigmente bevorzugt sind. Ganz besonders bevorzugt sind natürliches Calciumcarbonat und/oder präzipitiertes Calciumcarbonat und/oder Marmor und/oder Kreide und/oder Dolomit und/oder dolomithaltiges Calciumcarbonat.

Die wäßrige Suspension besteht bevorzugt aus 97,0 Gew.% bis 99,89 Gew.% Mineralien und/oder Füllstoffen und/oder Pigmenten und Wasser und 0,11 Gew.% - 3,0 Gew.% einer Mischung aus kationischem und/oder amphoterem kationischen und teilneutralisiertem anionischen und/oder teilneutralisiertem amphoteren anionischen Polyelektrolyt, bei einem Feststoffgehalt von 60 - 80 Gew.%, bezogen auf daß trockene Mineral bzw. den trockenen Füllstoff bzw. das trockene Pigment.

Weiterhin günstig ist es, daß die wäßrige Suspension aus 98,5 Gew.% bis 99,8 Gew.% Mineralien und/oder Füllstoffen und/oder Pigmenten und Wasser und 0,2 Gew.% - 1,5 Gew.% einer Mischung aus kationischem und/oder amphoterem kationischen und teilneutralisiertem anionischen und/oder teilneutralisiertem amphoteren anionischen Polyelektrolyt, bei einem Feststoffgehalt von 60 - 75 Gew.%, bezogen auf daß trockene Mineral bzw. den trockenen Füllstoff bzw. das trockene Pigment, besteht.

Weiterhin gute Ergebnisse werden erzielt, wenn die wäßrige Suspension aus 99,2 Gew.% bis 99,65 Gew.% Mineralien und/oder Füllstoffen und/oder Pigmenten und Wasser und 0,35 Gew.% - 0,8 Gew.% einer Mischung aus kationischem und/oder amphoterem katonischen und teilneutralisiertem anionischen und/oder teilneutralisiertem amphoteren anionischen Polyelektrolyt, bei einem Feststoffgehalt von 60 - 70 Gew.%, bezogen auf daß trockene Mineral bzw. den trockenen Füllstoff bzw. das trockene Pigment besteht.

Hervorragende Ergebnisse werden erzielt, wenn die wäßrige Suspension besteht aus 99,6 Gew.% bzw. 99,05

Gew.% bzw. 99,1 Gew.% Mineralien und/oder Füllstoffen und/oder Pigmenten und Wasser und 0,4 Gew.% bzw. 0,95 Gew.% bzw. 0,9 Gew.% einer Mischung aus kationischem und/oder amphoterem kationischen und teilneutralisiertem anionischen und/oder teilneutralisiertem amphoteren anionischen Polyelektrolyt, bei einem Feststoffgehalt von 67 Gew.% bzw. 67 Gew.% bzw. 60 Gew.%, bezogen auf daß trockene Mineral bzw. den trockenen Füllstoff bzw. das trockene Pigment, bei einer Kornverteilung, so daß 60 Gew.% bzw. 70 Gew.% bzw. 90 Gew.% der Teilchen einen äquivalent sphärischen Durchmesser < 2 µm aufweisen.

Vorteilhafterweise ist der anionische Polyelektrolyt und/oder amphotere anionische Polyelektrolyt in der Mischung mit dem kationischen und/oder amphoteren kationischen Polyelektrolyten mit einem ein- und/oder mehrwertigen Kation teilneutralisiert. Besonders gute Ergebnisse werden erzielt, wenn der anionische Polyelektrolyt und/oder amphotere anionische Polyelektrolyt in der Mischung mit dem kationischen und/oder amphoteren kationischen Polyelektrolyten mit Alkalimetall-Kationen und/oder Aminen und/oder Alkanol-aminen und/oder quaternären Ammoniumverbindungen, insbesondere aber mit $Na^+$ und/oder $Ca^{2+}$ und/oder $Mg^{2+}$ teilneutralisiert ist.

Die Grenzviskosität der in der wäßrigen Suspension verwendeten kationischen und/oder amphoteren kationischen Polyelektrolyten liegt vorzugsweise im Bereich zwischen 9,2 ml/g und 48.5 ml/g, insbesondere bevorzugt aber im Bereich zwischen 16,2 ml/g und 31,2 ml/g.

Eine weitere vorzugsweise Ausführungsform des erfindungsgemäßen Verfahrens ist durch folgende Verfahrensschritte gekennzeichnet:

a) eine wäßrige Suspension von Mineralien und/oder Füllstoffen und/oder Pigmenten wird zusammen mit der erfindungsgemäßen Dispergier- und Mahlhilfsmittelmischung naßvermahlen, wobei
b) ein Teil des teilneutralisierten anionischen und/oder teilneutralisierten amphoteren anionischen Polyelektrolyts vor der Vermahlung und
c) ein Teil des teilneutralisierten anionischen und/oder teilneutralisierten amphoteren anionischen Polyelektrolyts während der Vermahlung und/oder
d) ein Teil des teilneutralisierten anionischen und/oder teilneutralisierten amphoteren anionischen Polyelektrolyts nach der Vermahlung,
e) und der kationische und/oder amphotere kationische Polyelektrolyt vollständig vor der Vermahlung oder nur
f) ein Teil des kationischen und/oder amphoteren kationischen Polyelektrolyts vor der Vermahlung und
g) ein Teil des kationischen und/oder amphoteren kationischen Polyelektrolyts während der Vermahlung und/oder oder
h) ein Teil des kationischen und/oder amphoteren kationischen Polyelektrolyts nach der Vermahlung

hinzugegeben werden.

Besonders vorteilhaft ist ein Verfahren, in welchem

a) 10 - 90 Gew.% des teilneutralisierten anionischen und/oder teilneutralisierten amphoteren anionischen Polyelektrolyts vor der Vermahlung und
b) 10 - 90 Gew.% des teilneutralisierten anionischen und/oder teilneutralisierten amphoteren anionischen Polyelektrolyts während der Vermahlung und/oder
c) 0 - 80 Gew.% des teilneutralisierten anionischen und/oder teilneutralisierten amphoteren anionischen Polyelektrolyts nach der Vermahlung,
d) 50 - 100 Gew.% des kationischen und/oder kationischen amphoteren Polyelektrolyts vor der Vermahlung und
e) 0 - 50 Gew.% des kationischen und/oder kationischen amphoteren Polyelektrolyts während der Vermahlung und/oder
f) 0 - 50 Gew.% des kationischen und/oder amphoteren kationischen Polyelektrolyts nach der Vermahlung

zugegeben werden.

Gute Resultate werden erzielt, wenn ein Verfahren angewandt wird, bei welchem

a) 20 - 40 Gew.% des teilneutralisierten anionischen und/oder teilneutralisierten amphoteren anionischen Polyelektrolyts vor der Vermahlung und
b) 60 - 80 Gew.% des teilneutralisierten anionischen und/oder teilneutralisierten amphoteren anionischen Polyelektrolyts während der Vermahlung und/oder
c) 0 - 20 Gew.% des teilneutralisierten anionischen und/oder teilneutralisierten amphoteren anionischen Polyelektrolyts nach der Vermahlung,
d) 50 - 100 Gew.% des kationischen und/oder kationischen amphoteren Polyelektrolyts vor der Vermahlung und
e) 0 - 50 Gew.% des kationischen und/oder kationischen amphoteren Polyelektrolyts während der Vermahlung und/oder

f) 0 - 50 Gew.% des kationischen und/oder amphoteren kationischen Polyelektrolyts nach der Vermahlung

zugegeben werden.

Sehr gute Resultate werden erzielt, wenn ein Verfahren angewandt wird, bei welchem

a) 25 - 35 Gew.% des teilneutralisierten anionischen und/oder teilneutralisierten amphoteren anionischen Polyelektrolyts vor der Vermahlung und
b) 65 - 75 Gew.% des teilneutralisierten anionischen und/oder teilneutralisierten amphoteren anionischen Polyelektrolyts während der Vermahlung und/oder
c) 0 - 10 Gew.% des teilneutralisierten anionischen und/oder teilneutralisierten amphoteren anionischen Polyelektrolyts nach der Vermahlung, und
d) 70 - 100 Gew.% des kationischen und/oder kationischen amphoteren Polyelektrolyts vor der Vermahlung und
e) 0 - 30 Gew.% des kationischen und/oder kationischen amphoteren Polyelektrolyts während der Vermahlung und/oder
f) 0 - 30 Gew.% des kationischen und/oder amphoteren kationischen Polyelektrolyts nach der Vermahlung zugegeben werden.

Hervorragende Ergebnisse werden bei einem Verfahren erzielt, wobei

a) 30 Gew.% des teilneutralisierten anionischen und/oder teilneutralisierten amphoteren anionischen Polyelektrolyts vor der Vermahlung und
b) 70 Gew.% des teilneutraliserten anionischen und/oder teilneutralisierten amphoteren anionischen Polyelektrolyts während der Vermahlung und
c) 100 Gew.% des kationischen und/oder kationischen amphoteren Polyelektrolyts vor der Vermahlung

zugegeben werden.

Erfindungsgemäß erfolgt die Verwendung der wäßrigen Suspension von Mineralien und/oder Füllstoffen und/oder Pigmenten bei der Papierherstellung bzw. in der Papiererzeugung. Weitere Verwendungen betreffen die Oberflächenbehandlung (Pigmentierung der Papieroberfläche in der Leimpresse der Papiermaschine, die Verwendung in der Papierstreicherei, im Vorstrich bzw. im Deckstrich bei der Papierstreicherei, im Holzschliff zur Störstoffbekämpfung, im Streichereiausschuß zur Störstoffbekämpfung (Pitchkontrolle), im Kreislaufwasser der Papiermaschine zur CSB-Erniedrigung (chemischer Sauerstoffbedarf-Erniedrigung), in der Kläranlage zur Abwasserbehandlung, zur Vorflokkung, anionisch stabilisierter Pigment- und/oder Mineral- und/oder Füllstoff-Suspensionen in der Papiererzeugung bzw. zur Vorflockung (Immobilisierung) von Streichfarben in der Streicherei.

Es ist erfindungsgemäß gelungen, eine Mineral- und/oder Füllstoff- und/oder Pigment-Suspension durch Mahlen bei hohen Feststoffgehalten von ≥ 60 Gew.% herzustellen, bei welcher die Mineral- und/oder Füllstoff- und/oder Pigmentteilchen sowohl elektrostatisch positiv wie wahrscheinlich auch sterisch stabilisiert sind und die Suspension über Wochen viskositätsstabil bleibt und z. B. die Retention bei der Papierherstellung vorzüglich ist.

Überraschenderweise und nicht voraussehbar ist die Tatsache, daß bei der geeigneten Kombination eines oder mehrerer kationischer Polyelektrolyte und/oder eines oder mehrerer amphoterer kationischer Polyelektrolyte und eines oder mehrerer teilneutralisierter anionischer Polyelektrolyte und/oder eines oder mehrerer amphoterer anionischer teilneutralisierter Polyelektrolyte sowie der geeigneten Zugabestelle der Polyelektrolyte vor, während und/oder nach dem Mahlprozeß, bei den hohen Scherkräften und Temperaturen, wie sie beim Naßvermahlen auftreten, keine gegenseitige Neutralisation der entgegengesetzt geladenen Polymere und somit Koagulation der Polymere eintritt. Im Gegensatz dazu wird eine optimale Mahlung und Stabilisierung der Suspension dadurch bewirkt, daß die erfindungsgemäßen anionischen Polyelektrolyte

a) vermutlich als Brückenbildner zwischen den Mineral- und/oder Füllstoff- und/oder Pigmentteilchen und den erfindungsgemäßen kationischen und/oder amphoteren kationischen Polyelektrolyten wirken, wobei der so auf der Mineral- und/oder Füllstoff- und/oder Pigmentoberfläche fixierte kationische und/oder amphotere kationische Polyelektrolyt dem Mineral- und/oder Füllstoff- und/oder Pigmentteilchen eine positive Ladung gibt und dadurch zu einer elektrostatisch positiven Stabilisierung des Systems führt, und
b) erfindungsgemäß durch weitere Zugaben der erfindungsgemäßen anionischen Polyelektrolyte während und/oder nach der Mahlung diese vermutlich als Brückenbildner zwischen den kationischen Polymerketten des kationischen und/oder amphoteren kationischen Polyelektrolyts wirken, wobei vermutlich eine Überstruktur entsteht, welche die Mineral-und/oder Füllstoff- und/oder Pigmentteilchen sterisch stabilisiert, was zu einer wesentlich tieferen, stabilen Viskosität bei hoher Konzentration führt, als wenn die gesamte Menge an erfindungsgemäßem anionischem Polyelektrolyt zu Beginn der Mahlung zugegeben wird.

Überraschend und nicht voraussehbar ist ebenfalls die Tatsache, daß der Neutralisationsgrad der erfindungsgemäßen anionischen Polyelektrolyte mit ein- und/oder mehrwertigen Kationen einen ausschlaggebenden Einfluß auf die Lagerstabilität der Mineral- und/oder Füllstoff- und/oder Pigment-Suspension hat, d. h. auf die Viskositätskonstanz der Suspension über die Zeit.

Beim Einsatz von mit 100 Mol.% natriumneutralisierten anionischen Polyelektrolyten, wie sie in den Beispielen der EP 0278602 A 1 verwendet werden, steigt die Viskosität über die Zeit stark an, so daß die Suspension unbrauchbar wird.

Bei Verwendung eines erfindungsgemäß mit ein- und/oder mehrwertigen Kationen teilneutralisierten anionischen Polyelektrolyten und/oder teilneutralisierten amphoteren anionischen Polyelektrolyten bleibt dagegen die Viskosität proportional zum Neutralisationsgrad über Tage bis Wochen stabil. Je tiefer der Neutralisationsgrad mit einwertigen Kationen, desto besser die Lagerstabilität. Am besten geeignet sind Neutralisationsgrade von 5 - 10 Mol.%. Mehrwertige Kationen, wie Calcium und/oder Magnesium, haben einen geringeren negativen Einfluß auf die Lagerstabilität.

Bei nichtneutralisierten anionischen Polyelektrolyten, im speziellen bei Polyacrylsäure, ergibt sich das Problem, daß eine üblicherweise 40 Gew.-%ige wässrige Polymerlösung sehr hochviskos ist sowie normalerweise eine Kristallisationstemperatur über 0°C aufweist. Polyacrylsäure kristallisiert bereits bei 20°C. Dies führt zu Problemen bei der Dosierung, vor allem in kalten Jahreszeiten und speziell in Skandinavien. Dadurch ergibt sich eine unregelmäßige Dosierung, die zu großen Viskositätsschwankungen bei den herzustellenden Mineral- und/oder Füllstoff- und/oder Pigmentsuspensionen führt.

Bei den erfindungsgemäßen, anionischen Polyelektrolyten ist dies jedoch nicht der Fall.

Eine gute Lagerstabilität in Bezug auf die Viskosität und das Absetzverhalten ist vor allem beim Transport und bei großen Lagertanks von ausschlaggebender Bedeutung, um den Verderb der Ware zu verhindern. Mit der erfindungsgemäß hergestellten Mineral- und/oder Füllstoff- und/oder Pigment-Suspension ist es möglich, den Produktionsort (Herstellungsort der Mineral-und/oder Füllstoff- und/oder Pigment-Suspension) sowie den Verbraucherort (z. B. Papierfabrik) frei zu wählen. Der Produktionsort kann so den geologischen Vorkommen der Mineral-und/oder Füllstoff- und/oder Pigment-Vorkommen angepaßt werden, und es muß nicht aus rein logistischen Gründen der Standort des Kunden mitberücksichtigt werden.

Eine wäßrige Suspension von Mineralien und/oder Füllstoffen und/oder Pigmenten mit einem Feststoffgehalt ≥ 60 Gew.%, bezogen auf die trockenen Mineralien und/oder Füllstoffe und/oder Pigmente, wird erfindungsgemäß durch Mahlen eines grob gebrochenen Rohgesteins hergestellt, wobei eine Kombination aus einem teilneutralisierten anionischen und/oder teilneutralisierten amphoteren anionischen Polyelektrolyten und einem kationischen und/oder amphoteren kationischen Polyelektrolyten in der Weise eingesetzt wird, so daß der gesamte bzw. ein Teil des kationischen und/oder des amphoteren kationischen Polyelektrolyten und nur ein Teil des teilneutralisierten anionischen Polyelektrolyten und/oder des teilneutralisierten amphoteren anionischen Polyelektrolyten zu Beginn der Mahlung zugesetzt wird und weitere Teile der erfindungsgemäßen anionischen Polyelektrolyten während der Mahlung und/oder nach der Mahlung zur Viskositätssenkung zugegeben werden.

Obwohl der kationische und/oder amphotere kationische Polyelektrolyt im Überschuß vorhanden ist und sich dadurch eine positive Ladung auf den Mineral- und/oder Füllstoff- und/oder Pigmentteilchen befindet, bewirkt eine weitere Zugabe des erfindungsgemäßen anionischen und/oder amphoteren anionischen Polyelektrolyten während dem Mahlen und/oder nach dem Mahlen eine nicht voraussehbare enorme Viskositätsabsenkung.

Bei der erfindungsgemäßen Teilneutralisation des anionischen und/oder amphoteren anionischen Polyelektrolyten mit ein- und/oder mehrwertigen Kationen ergibt sich zudem eine sehr stabile Viskosität über mehrere Wochen. Dieser Effekt konnte mit keinem, dem Stand der Technik entsprechenden System erreicht werden.

In den Beispielen nach dem Stand der Technik mußte die Mahlung wegen Blockierens der Mühle vor Erreichen der gewünschten Feinheit abgebrochen werden. Das Blockieren der Mühle kam durch einen enormen Viskositätsanstieg während dem Mahlen zustande.

Die Viskositätserhöhung rührt vermutlich daher, daß normalerweise kationische polymere Polyelektrolyte mit polymeren anionischen Polyelektrolyten unter Salzbildung miteinander reagieren und sich gegenseitig neutralisieren und ausfallen. Bei der erfindungsgemäßen Polyelektrolytkombination und den erfindungsgemäßen Zugabestellen tritt dies überraschenderweise nicht ein, vielmehr tritt eine nicht eindeutig erklärbare starke Viskositätserniedrigung ein. Der während der Mahlung und/oder nachträglich zugegebene erfindungsgemäße anionische und/oder amphotere anionische Polyelektrolyt wirkt auf den kationischen und/oder amphoteren kationischen Polyelektrolyten nicht ladungsneutralisierend, wie dies eigentlich zu erwarten wäre.

Die für die Anwender, vornehmlich die Papierindustrie, ideale Kornverteilung, Konzentration und tiefe Viskosität der Mineral- und/oder Füllstoff- und/oder Pigment-Suspensionen können nach dem erfindungsgemäßen Verfahren in einem Arbeitsgang hergestellt werden, was einen enormen ökonomischen und qualitativen Fortschritt darstellt.

- Vorzugsweise beträgt die Konzentration der wäßrigen Aufschlämmung 60 - 70 Gew.%, bezogen auf das trockene Mineral.

- Vorzugsweise hat das Rohmaterial vor dem Mahlprozeß erfindungsgemäß einen mittleren äquivalent sphärischen Teilchendurchmeser von 10 -50 µm (gemessen auf dem Sedigraph 5100).

Bei der Mahlung dient also vermutlich der erfindungsgemäße anionische und/oder amphotere anionische Polyelektrolyt, welcher von seinen chemischen Eigenschaften her auf die beim Mahlen neu gebildete Oberfäche des Minerals und/oder Füllstoffs und/oder Pigments aufzieht, als Brückenbildner zwischen dem Mineral und/oder Füllstoff und/oder Pigment und dem kationischen und/oder amphoteren kationischen Polyelektrolyten. Der dadurch in genügendem Maße fixierte kationische und/oder amphotere kationische Polyelektrolyt gibt dem Mineral- und/oder Füllstoff- und/oder Pigmentteilchen eine positive Ladung. Zudem wirkt vermutlich der in weiteren Schritten zur Mineral- und/oder Füllstoff- und/oder Pigment-Suspension zugegebene erfindungsgemäße anionische und/oder amphotere anionische Polyelektrolyt mit der erfindungsgemäßen Kettenlänge als Brückenbildner zwischen den Polymerketten des kationischen und/oder amphoteren kationischen Polyelektrolyten, was zu größeren Polymerkettenverbänden führt, welche die Mineralteilchen vermutlich zusätzlich sterisch stabilisieren.

Bei der Mahlung dient der kationische und/oder amphotere kationische Polyelektrolyt, welcher, unterstützt durch den erfindungsgemäßen anionischen und/oder amphoteren anionischen Polyelektrolyten, vermutlich auf die Mineral- und/oder Füllstoff-und/oder Pigmentoberfläche aufzieht, als positiver Ladungsträger und stabilisert so die Mineral- und/oder Füllstoff- und/oder Pigmentteilchen positiv.

Durch die vermutete Brückenbildung zwischen dem erfindungsgemäßen anionischen und/oder amphoteren anionischen Polyelektrolyten und dem kationischen und/oder amphoteren kationischen Polyelektrolyten wird zudem vermutlich eine sterische Stabilisierung der Mineral- und/oder Füllstoff- und/oder Pigmentteilchen bewirkt.

Geeignete Mischungen des erfindungsgemäßen anionischen und/oder amphoteren anionischen Polyelektrolyten und des kationischen und/oder amphoteren kationischen Polyelektrolyten vor dem Mahlen sind erfindungsgemäß:

teilneutralisierter anionischer und/oder teilneutralisierter amphoterer anionischer Polyelektrolyt zu kationischer und/oder amphoterer kationischer Polyelektrolyt = 1:10 bis 1:40

vorzugsweise für Marmor 1:12
für Champagnekreide 1:30

Während und/oder nach dem Mahlen wird je nach Konzentration und gewünschter Endviskosität nochmals teilneutralisierter anionischer und/oder teilneutralisierter amphoterer anionischer Polyelektrolyt zugegeben. Vorzugsweise ca. doppelt so viel wie vor der Mahlung.

Weitere erfindungsgemäße Beispiele

Beispiel 11:

Wie Beispiel 1a (Stand der Technik), jedoch teilneutralisierte Polyacrylsäure wie Beispiel 12.

| Viskosität in mPas | | |
|---|---|---|
| nach 1 Std. | 1 Tag | 15 Tage |
| 144 | 152 | 280 |

Das Beispiel 11 zeigt deutlich, daß im Gegensatz zum Beispiel 1a (Stand der Technik) mit einer teilneutralisierten Polyacrylsäure eine wesentlich bessere Lagerstabilität über mehrere Wochen erreicht wird.

Beispiel 12:

Eine 70 Gew.%-ige wäßrige Aufschlämmung von natürlichem Marmor mit einer Kornverteilung,so daß 60 Gew.% der Teilchen einen äquivalent sphärischen Durchmesser < 2 µm aufweisen (gemessen auf dem Sedigraph 5100), wurden mit 0,33 Gew.%, bez. auf den trockenen Marmor, des folgenden Copolymers (amphoteres, kationisches Polymer),

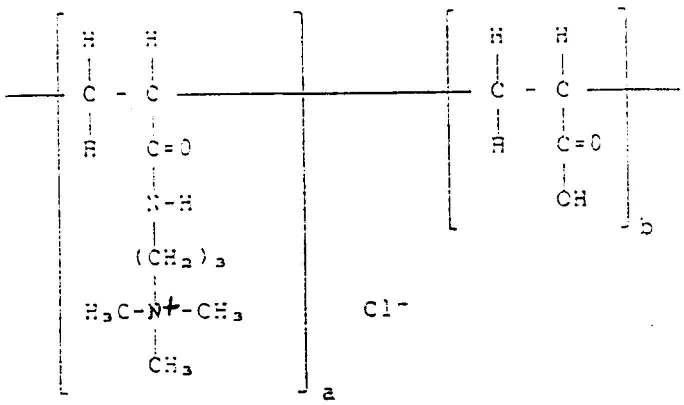

a = 95 Mol-%
b = 5 Mol-%

Grenz-Viskosität (27.3 ml/g)

sowie mit 0,06 Gew.%, bez. auf den trockenen Marmor, mit Natronlauge teilneutralisierter Polyacrylsäure (10 Mol. der Carboxylgruppen neutralisiert) verschiedener spezifischer Viskositäten resp. Molekulargewichten unter starkem Rühren dispergiert (8000 U/min. Rührscheibendurchmesser 50 mm).
Das Ziel dieser Versuchsserie war es, die optimale spezifische Viskosität bzw. das Molekulargewicht des teilneutralisierten anionischen Polyelektrolyten festzustellen.

| spez. Viskosität des teilneutralisierten Polyacrylats | Viskosität der Suspension |
|---|---|
| 0,2 | 2640 mPas |
| 0,35 | 370 mPas |
| 0,54 | 350 mPas |
| 0,71 | 1420 mPas |

Die optimale spez. Viskosität des teilneutralisierten Polyacrylats liegt bei 0,35 - 0,54.

Beispiel 13:

Eine 67 Gew.%-ige wäßrige Aufschlämmung von natürlichem Marmor mit einer Kornverteilung, so daß 60 Gew.% der Teilchen einen äquivalent sphärischen Durchmesser < 2 μm aufweisen (gemessen auf dem Sedigraph 5100), wurden mit 0,33 Gew.%, bez. auf den trockenen Marmor des Copolymers aus Beispiel 12, jedoch mit unterschiedlichen Grenzviskositäten resp. Molekulargewichten, und 0,06 Gew.%, bez. auf den trockenen Marmor, des teilneutralisierten Polyacrylats aus Beispiel 12 mit der spez. Viskosität von 0,35 unter starkem Rühren dispergiert (8000 U/min Rührscheibendurchmesser 50 mm).

| Grenzviskosität des kationischen Copolymers | Viskosität der Suspension 1 Std. nach dem Dispergieren |
|---|---|
| 9,2 ml/g | 730 mPas |
| 12,8 ml/g | 500 mPas |
| 15,5 ml/g | 350 mPas |
| 16,2 ml/g | 156 mPas |
| 31,2 ml/g | 112 mPas |
| 48,5 ml/g | 840 mPas |

Die optimale Grenzviskosität des verwendeten kationischen Copolymers liegt zwischen 15 ml/g und 40 ml/g.

Beispiel 14:

Eine 70 Gew.%-ige wäßrige Aufschlämmung von natürlichem Marmor mit einer Kornverteilung, so daß 60 Gew.% der Teilchen einen äquivalent sphärischen Durchmesser < 2 μm aufweisen (gemessen auf dem Sedigraph 5100), wurden mit 0,33 Gew.%, bez. auf den trockenen Marmor, des Copolymers aus Beispiel 12 und 0,06 Gew.%, bez. auf den trockenen Marmor, Polyacrylsäure (spez. Viskosität 0,35) mit einem verschiedenem Neutralisationsgrad der Carboxylgruppen mit Natronlauge, unter starkem Rühren dispergiert (8000 U/min Rührscheibendurchmesser 50 mm).

| Neutralisationsgrad der Carboxylgruppen der Polyacrylsäure | Viskosität der Suspension | | | |
|---|---|---|---|---|
| | nach 1 Std. | nach 6 Tg. | nach 12 Tg. | nach 18 Tg. |
| 100 Mol.% neutr. | 148 mPas | 640 mPas | 1560 mPas | >3000 mPas |
| 70 Mol.% neutr. | 128 mPas | 350 mPas | 1075 mPas | 1420 mPas |
| 50 Mol.% neutr. | 112 mPas | 176 mPas | 720 mPas | 1075 mPas |
| 30 Mol.% neutr. | 112 mPas | 172 mPas | 460 mPas | 720 mPas |
| 10 Mol.% neutr. | 112 mPas | 128 mPas | 172 mPas | 156 mPas |

Die beste Langzeitstabilität wird mit einer Polyacrylsäure, bei welcher 5 - 10 Mol.% der Carboxylgruppen neutralisiert sind, erreicht.

Beispiel 15:

Eine 70 Gew.%-ige wäßrige Aufschlämmung von natürlichem Marmor mit einer Kornverteilung, so daß 60 Gew.% der Teilchen einen äuqivalent sphärischen Durchmesser < 2 μm aufweisen (gemessen auf dem Sedigraph 5100), wurde mit 0,33 Gew.%, bez. auf den trockenen Marmor, verschiedener prozentualer molarer Zusammensetzungen des Copolymers aus Beispiel 12 und 0,06 Gew.%, bez. auf den trockenen Marmor, des teilneutralisierten Polyacrylats (spez. Viskosität 0,35, aus Beispiel 12) unter starkem Rühren dispergiert (8000 U/min Rührscheibendurchmesser 50 mm).

| Mol.% kationisches Monomer | Mol.% anionisches Monomer | Viskosität der Suspension nach 1 Std. |
|---|---|---|
| 80 Mol.% | 20 Mol.% | 680 mPas |
| 87 Mol.% | 13 Mol.% | 580 mPas |
| 95 Mol.% | 5 Mol.% | 172 mPas |
| 100 Mol.% | 0 Mol.% | 420 mPas |

Die optimale Monomerzusammensetzung des kationischen Polyelektrolyten liegt bei 95 Mol.% kationischer Verbindung und 5 Mol.% anionischer Verbindung.

Beispiel 16:

Eine 67 Gew.%-ige wäßrige Aufschlämmung von natürlichem Marmor mit einem äquivalent sphärischen mittleren Teilchendurchmesser von 12 μm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 60 Gew.% der Teilchen einen äquivalenten sphärischen Durchmesser < 2 μm (gemessen auf dem Sedigraph 5100) aufwiesen, aufgemahlen.

Rezeptur:

5000 g Marmor
15 g amphoteres, kationisches Polymer entsprechend der Formel des Beispiels 12
1,35 g Polyacrylsäure (spez. Viskosität 0,54), 5 Mol.% der Carboxylgruppen neutr. mit NaOH, vor der Mahlung zugegeben
3,15 g Polyacrylsäure (spez. Viskosität 0,54), 5 Mol.% der Carboxylgruppen neutr. mit NaOH, während der Mahlung zugegeben
2472 g Wasser

| Viskosität: | nach 2 Std. | 1 Tag | 5 Tage | 10 Tage | 20 Tage |
|---|---|---|---|---|---|
| | 200 | 116 | 148 | 104 | 104 mPas |

In Beispiel 16 ist deutlich zu erkennen, daß bei der erfindungsgemäßen Art und Kombination von anionischen und kationischen Polyelektrolyten eine sehr tiefe und über Wochen stabile Viskosität auch bei durch Mahlen erzeugten feinteiligen Mineral- und/oder Füllstoff- und/oder Pigment Suspensionen herzustellen ist.

<u>Beispiel 17:</u>

Eine 67 Gew.%-ige wäßrige Aufschlämmung von Champagnekreide mit einem äquivalent sphärischen mittleren Teilchendurchmesser von
12 µm (gemessen auf dem Sedigraph 5100) wurde mit der folgenden Rezeptur in einer Dynomill (0,6 l Mahlbehälter) unter Verwendung
von Mahlkörpern aus Glas (∅ 1 mm) auf eine Kornverteilungskurve, so daß 67 Gew.% der Teilchen einen äquivalenten sphärischen Durchmesser < 2 µm (gemessen auf dem Sedigraph 5100) aufwiesen, aufgemahlen.

Rezeptur:

5000 g Champagnekreide
25 g amphoteres, kationisches Polymer aus Beispiel 12
0,5 g Polyacrylsäure (spez. Viskosität 0,54) 5 Mol.% der Carboxylgruppen neutr. mit NaOH, vor der Mahlung zugegeben
2472 g Wasser

| Viskosität in mPas | | | | |
|---|---|---|---|---|
| nach 1 Std. | 1 Tag | 5 Tage | 10 Tage | 20Tage |
| 2400 | 3900 | >5000 | | |

2,5 gPolyacrylsäure (spez. Viskosität 0,54), 5 Mol.% der Carboxylgruppen neutr. mit NaOH, 5 min nach der Mahlung unter intensivem Rühren zugegeben
(8000 U/min, Rührscheibendurchmesser 50 mm)

| Viskosität in mPas | | | | |
|---|---|---|---|---|
| nach 1 Std. | 1 Tag | 5 Tage | 10 Tage | 20 Tage |
| 235 | 230 | 200 | 200 | 210 |

Im Beispiel 17 ist deutlich zu erkennen, daß auch eine nachträgliche Zugabe des erfindungsgemäßen anionischen Polyelektrolyten eine enorme Viskositätssenkung zur Folge hat und die Viskosität über Wochen stabil bleibt.

Beispiel 18:

Eine 60 Gew.%-ige wäßrige Aufschlämmung von natürlichem Marmor mit einem äquivalent sphärischen mittleren Teilchendurchmesser von 12 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 88 Gew.% der Teilchen einen äquivalent
sphärischen Durchmesser < 2 µm (gemessen auf dem Sedigraph 5100) aufwiesen, aufgemahlen.

Rezeptur:

| | |
|---|---|
| 5000 g | Marmor |
| 40 g | amphoteres, kationisches Polymer aus Beispiel 12 |
| 1,35 g | Polyacrylsäure (spez. Viskosität 0,54), 5 Mol.% der Carboxylgruppen neutr. mit NaOH, vor der Mahlung zugegeben |
| 2,65 g | Polyacrylsäure (spez. Viskosität 0,54), 5 Mol.% der Carboxylgruppen neutr. mit NaOH, während der Mahlung zugegeben |
| 3363 g | Wasser |

| Viskosität: | nach 1 Std. | 1 Tag | 4 Tage | 8 Tage | 11 Tage |
|---|---|---|---|---|---|
| | 500 | 520 | 400 | 400 | 390 mPas |

Auch sehr hohe Feinheiten, wie sie in Streichrezepturen Verwendung finden, sind problemlos durch Mahlen von grob gebrochenem Rohgestein bei hohen Konzentrationen herstellbar.

Beispiel 19:

Eine 67 Gew.%-ige wäßrige Aufschlämmung von natürlicher Campagnekreide mit einem äquivalent sphärischen mittleren Teilchendurchmesser von 18 µm (gemessen auf dem Sedigraph 5100) wurde mit der folgenden Rezeptur in einer Dynomill (0,6 l Mahlbehälter) unter Verwendung von Mahlkörpern aus Glas (∅ 1 mm) auf eine Kornverteilungskurve, so daß 67 Gew.% der Teilchen einen äquivalent sphärischen Durchmesser < 2 µm (gemessen auf dem Sedigraph 5100) aufweisen, aufgemahlen.

Rezeptur:

| | |
|---|---|
| 5000 g | Champagnekreide |
| 37,5 g | amphoteres, kationisches Polymer aus Beispiel 12 |
| 1,35 g | Polyacrylsäure (spez. Viskosität 0,54), 5 Mol.% der Carboxylgruppen neutr. mit NaOH, vor der Mahlung zugegeben |
| 7,65 g | Polyacrylsäure (spez. Viskosität 0,54), 5 Mol.% der Carboxylgruppen neutr. mit NaOH, während der Mahlung zugegeben |
| 2486 g | Wasser |

| Viskosität: | nach 1 Std. | 1 Tag | 4 Tage | 7 Tage |
|---|---|---|---|---|
| | 212 | 170 | 132 | 124 mPas |

Beispiel 20:

Im Pilot-plant-Maßstab wurde der in Beispiel 16 verwendete natürliche Marmor in einer vertikal angeordneten Perlmill (Süssmeier mit 180 l Inhalt) mit Mahlkörpern aus Glas (∅ 1-2 mm) auf eine Kornverteilungskurve, so daß 63 Gew.% der Teilchen einen äquivalent sphärischen Durchmesser < 2 µm (gemessen auf dem Sedigraph 5100) bei einer Konzentration von 67,6 Gew.% Feststoff aufwiesen, vermahlen.

In Batches von ca. 600 kg wurde 50 to dieser Suspension hergestellt.

Rezeptur:

| | |
|---|---|
| 400 kg | Marmor |
| 1,4 kg | amphoteres, kationisches Polymer aus Beispiel 12 |
| 0,12kg | Polyacrylsäure (spez. Viskosität 0,54) 5 Mol.% der Carboxylgruppen neutr. mit NaOH, vor der Mahlung zugegeben |
| 0,24 kg | Polyacrylsäure (spez. Viskosität 0,54) 5 Mol.% der Carboxylgruppen neutr. mit NaOH, während der Mahlung zugegeben |
| 197 kg | Wasser |

Die Stundenleistung der Perlmill betrug 500 l Slurry/Stunde.

| Viskosität: | nach 1 Std. | 1 Tag | 7 Tage | 14 Tage | 21 Tage |
|---|---|---|---|---|---|
| cP | 230 | 230 | 150 | 150 | 160 |

Beispiel 21:

Im Pilot-plant-Maßstab wurde der im Beispiel 16 verwendete Marmor in einer vertikal angeordneten Perlmill (Süssmeier mit 180 l Inhalt) mit Mahlkörpern aus Glas (∅ 1-2 mm) auf eine Kornverteilungskurve, so daß 70 Gew.% der Teilchen einen äquivalent sphärischen Durchmesser < 2 µm (gemessen auf dem Sedigraph 5100) aufwiesen, bei einer Konzentration von 70,6 Gew.% vermahlen. In Batches von ca. 600 kg wurden 4 to dieser Slurry hergestellt.

Rezeptur:

| | |
|---|---|
| 400 kg | Marmor |
| 2,0 kg | amphoteres, kationisches Polymer aus Beispiel 12 |
| 0,12kg | Polyacrylsäure (spez. Viskosität 0,54), 5 Mol.% der Carboxylgruppen neutr. mit NaOH, vor der Mahlung zugegeben |
| 0,36kg | Polyacrylsäure (spez. Viskosität 0,54), 5 Mol.% der Carboxylgruppen neutr. mit NaOH, während der Mahlung zugegeben |
| 168 kg | Wasser |

Die Stundenleistung betrug 500 l Suspension/Std.

| Viskosität: | nach 1 Std. | 1 Tag | 7 Tage | 14 Tage | 21 Tage |
|---|---|---|---|---|---|
| | 450 | 420 | 400 | 400 | 400 |

Beispiel 22 :

Eine 70 Gew.-%ige wäßrige Aufschlämmung von Titandioxyd mit einer Kornverteilung, so daß 94 Gew.-% der Teilchen einen äquivalent sphärischen Durchmesser < 2 µm aufweisen (gemessen auf dem Sedigraph 5100), wurden unter starken Scherkräften dispergiert (8000 U/min, Rührscheiben ∅ 50 mm).

Rezeptur:

| | |
|---|---|
| 1500 g | $TiO_2$ |
| 7 g | amphoteres, kationisches Polymer aus Beispiel 12 |
| 640 g | Wasser zugeben |

| Viskosität in mPas | | | |
|---|---|---|---|
| nach 1 Std. | 1 Tag | 10 Tage | 20 Tage |
| 275 | --- | 300 | 290 |

Beispiel 23 :

Eine 65 Gew.-%ige wäßrige Aufschlämmung von Titandioxyd mit einer Kornverteilung, so daß 94 Gew.-% der Teilchen einen äquivalent sphärischen Durchmesser < 2 µm aufweisen (gemessen auf dem Sedigraph 5100), wurden unter starken Scherkräften dispergiert (8000 U/min, Rührscheiben ∅ 50 mm).

Rezeptur:

1250 g $TiO_2$
2 g amphoteres, kationisches Polymer aus Beispiel 2
675 g Wasser zugeben

| Viskosität in mPas | | | |
|---|---|---|---|
| nach 1 Std. | 1 Tag | 10 Tage | 20 Tage |
| 350 | 370 | 400 | 420 |

Beispiel 24 :

Eine 60 Gew.-%ige wäßrige Aufschlämmung von natürlichem $CaSO_4$ mit einer Kornverteilung, so daß 23 Gew.-% der Teilchen einen äquivalent sphärischen Durchmesser < 2 µm aufweisen (gemessen auf dem Sedigraph 5100 anionisch), wurden unter starken Scherkräften dispergiert (8000 U/min, Rührscheiben ∅ 50 mm).

Rezeptur :

1000 g $CaSO_4$
3,6g amphoteres, kationisches Polymer aus Beispiel 12
670 g Wasser zugeben

| Viskosität in mPas | | | |
|---|---|---|---|
| nach 1 Std. | 1 Tag | 10 Tage | 20 Tage |
| 350 | 390 | 400 | |

Beispiel 25 :

Eine 63 Gew.-%ige wäßrige Aufschlämmung von $CaCO_3$ (74,5 %iger Slurry aus Bespiel 7A) und trockenem Talc wovon 47 Gew.% der Teilchen einen äquivalent sphärischen Durchmesser < 2 µm aufweisen (gemessen auf dem Sedigraph 5100), wurden unter starken Scherkräften dispergiert (8000 U/min, Rührscheiben ∅ 50 mm), so daß eine 1:1 Mischung Talc/$CaCO_3$ entsteht.

Rezeptur :

670 g Slurry 74,5 %ig aus Bespiel 7A

| | |
|---|---|
| 549 g | Talc trocken 91 %ig |
| 2,8 g | amphoteres, kationisches Polymer aus Beispiel 12 |
| 594 g | Wasser zugeben |

| Viskosität in mPas | | |
|---|---|---|
| nach 1 Std. | 1 Tag | 2 Tagen |
| 450 | | 500 |

Anwendungsbeispiel

Die in den Herstellungsbeispielen 20 + 21 hergestellten Marmorslurries wurden im Vergleich zu einem, heute üblicherweise mit anionischen Dispergiermitteln hergestellten Marmorslurry auf ihre Retention bei der Papierherstellung untersucht.

Testbedingungen:

| | | |
|---|---|---|
| Stoff: | 80 % Birkensulfat<br>20 % Kiefersulfat | Mahlgrad 23° SR |
| Retentionshilfsmittel: | | 0,05 % Polyacrylamid<br>(Grenzviskosität 700 ml/g) |

Durchführung der Retentionsuntersuchung nach Britt-Jar Firma Paper Research Material, SYRACUSE, U.S.A.:

1. 275 ml 2%-ige Fasersuspension (atro 3,63 g Fasern) sowie 275 ml des. Wasser ins Britt-Jar Gefäß geben;
2. Britt-Jar Rührer auf 700 U/min;
3. 25,4 ml 5%-ige Mineral- und/oder Füllstoff- und/oder Pigmentsuspension zugeben;
4. nach 20 sec. die entsprechende Menge Retentionsmittel zugeben;
5. nach weiteren 25 sec. Ablaufhahn öffnen und 100 ml Siebwasser ablaufen lassen.
6. Im Siebwasser wird der $CaCO_3$-Anteil komplexometrisch nach aufschließen mit HCl oder mit Flammen-AAS bestimmt. Bei anderen Mineralien und/oder Füllstoffen und/oder Pigmenten wird das Siebwaser über Membranfilter filtriert, verascht bei 600 °C, über einen alkalischen Schmelzaufschluß, z. B. mit NaOH/KOH im Zirkontiegel, in eine wasserlösliche Form gebracht und im angesäuerten Zustand mittels AAS bestimmt. Unter Berücksichtigung der entsprechenden Umrechnungsfaktoren kann auf die jeweiligen Mineralien und/oder Füllstoffe und/oder Pigmente geschlossen werden.
7. Über den eingetragenen Anteil an Mineralien und/oder Füllstoffen und/oder Pigmenten pro 100 ml und den im Siebwasser bestimmten Anteil an Mineralien und/oder Füllstoffen und/oder Pigmenten pro 100 ml läßt sich die Füllstoffretention berechnen.

Resultate:

| Produkte: | Füllstoff-First-Pass-Retention |
|---|---|
| Anionisch stabilisierte Slurry mit 60 % < 2 µm (0,15 % Natriumpolyacrylat spez. Visk. 0,35) | 41,1 % |
| Anionisch stabilisierte Slurry mit 70 % < 2 µm (0,3% Natriumpolyacrylat spez. Vis. 0,54) | 35,3 % |
| Slurry aus Herstellungsbeispiel 19 | 61,9 % |
| Slurry aus Herstellungsbeispiel 20 | 67,8 % |

Durch den Einsatz eines mit dem erfindungsgemäßen, neuen Herstellungsverfahren produzierten Marmorslurry ist eine starke Steigerung der Füllstoffretention möglich ohne die Papierformation negativ zu beeinträchtigen, was einen enormen Sprung in der Entwicklung darstellt.

Die erfindungsgemäßen wäßrigen Suspensionen sowie das erfindungsgemäße Verfahren zu ihrer Herstellung weisen u.a. folgende Vorteile auf:

- Im Gegensatz zu den bis heute bekannten Verfahren ist es möglich, hochkonzentrierte (≥ 60 Gew.%) Mineral- und/oder Füllstoff- und/oder Pigment-Suspension durch Naßvermahlung aus grob gebrochenem Rohstein herzustellen.
- Die Suspensionen weisen eine ausgezeichnete Lagerstabilität bei tiefen Viskositäten auf.
- In der Anwendung ergeben sich z.B. große Vorteile bezüglich der Füllstoffretention in der Papiererzeugung.
- Die Mahlung und Dispergierung ist unter hohen Mahlkräften und bei Siedetemperatur des Wassers möglich.

**Patentansprüche**

1. Wäßrige Suspension aus Mineralien und/oder Füllstoffen und/oder Pigmenten mit einem Feststoffgehalt ≥ 60 Gew.%, bezogen auf das trockne Mineral bzw. den trocknen Füllstoff bzw. das trockne Pigment, wobei das Mineral bzw. der Füllstoff bzw. das Pigment mit einem Dispergiermittel dispergiert ist,
**dadurch gekennzeichnet**,

daß das Dispergiermittel
einen oder mehrere amphotere Polyelektrolyten, bei welchen die Anzahl der negativen Ladungen in den anionischen Monomereinheiten gleich der Anzahl der positiven Ladungen in den kationischen Monomereinheiten ist, oder die zusätzlich neutrale Monomereinheiten enthalten können,
und/oder
einen oder mehrere amphotere kationische Polyelektrolyten, bei welchen die nicht neutralen Monomereinheiten überwiegend positive Ladungen tragen,
und/oder
einen oder mehrere amphotere anionische Polyelektrolyten, bei welchen die nicht neutralen Monomereinheiten überwiegend negative Ladungen tragen
und/oder
einen oder mehrere teilneutralisierte anionische Polyelektrolyten
und/oder
einen oder mehrere teilneutralisierte amphotere anionische Polyelektrolyten, bei welchen die nicht neutralen Monomereinheiten überwiegend negative Ladungen tragen, enthält, wobei die amphoteren, die amphoter kationischen und die amphoter anionischen Polyelektrolyten quaternäre Ammoniumgruppen enthalten, und wobei die Füllstoff- und/oder Pigment- und/oder Mineralteilchen eine gegen außen neutrale oder positive Ladung tragen müssen.

2. Wäßrige Suspension nach Anspruch 1,
**dadurch gekennzeichnet**,

daß die Suspension zusätzlich einen oder mehrere kationische Polyelektrolyten enthält.

3. Wäßrige Suspension nach Anspruch 1 oder Anspruch 2,
dadurch gekennzeichnet,

daß das Dispergiermittel eine Mischung aus

(a) einem oder mehreren kationischen Polyelektrolyten und/oder einem oder mehreren amphoteren kationischen Polyelektrolyten, bei welchen die nichtneutralen Monomereinheiten überwiegend positive Ladungen tragen, und
(b) einem oder mehreren teilneutralisierten anionischen Polyelektrolyten und/oder einem oder mehreren teilneutralisierten amphoteren anionischen Polyelektrolyten, bei welchen die nichtneutralen Monomereinheiten überwiegend negative Ladungen tragen, ist, wobei der kationische Polyelektrolyt und/oder der amphotere kationische Polyelektrolyt in einer Menge vorliegt, daß die dispergierten Mineral- bzw. Füllstoff- bzw. Pigment-Partikeln positive Ladungen tragen.

**4.** Wäßrige Suspension nach einem oder mehreren der vorhergehenden Ansprüche,
dadurch gekennzeichnet,

daß der amphotere anionische und der amphotere kationische und der kationische Polyelektrolyt die die positive Ladungen erzeugende funktionelle Gruppe in einem Substituenten der ethylenischen Hauptkette trägt, und quaternäre Ammoniumgruppen, Carboxylgruppen und/oder Sulfonsäuregruppen und/oder saure phosphorsäure-esterhaltige Gruppen enthält und der die kationische Ladung tragende Substituent über

```
 O H         oder        O
 ‖ |                     ‖
-C-N-                   -C-O-
```

an der Hauptkette gebunden ist.

**5.** Wäßrige Suspension nach einem oder mehreren der vorhergehenden Ansprüche,
dadurch gekennzeichnet,

daß der anionische teilneutralisierte und der amphotere anionische teilneutralisierte Polyelektrolyt je Carboxylgruppen tragen und der anionische teilneutralisierte Polyelektrolyt ein homo- und/oder copolymerer Polyelektrolyt ist.

**6.** Wäßrige Suspension nach einem oder mehreren der vorhergehenden Ansprüche,
dadurch gekennzeichnet,

daß der amphotere anionische und der amphotere und der amphotere kationische Polyelektrolyt eine oder mehrere Verbindungen aus der Gruppe der folgenden Verbindungen gemäß der folgenden allgemeinen Formel ist:

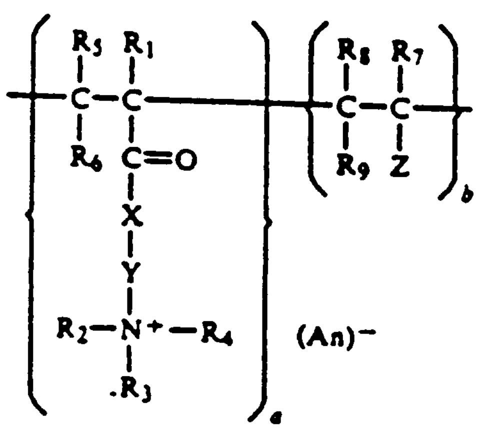

wobei $R_1$, $R_5$, $R_6$ und $R_7$ H ist,
und/oder $R_1$ bis $R_7$

- ein Alkyl und/oder
- Aryl ist,

$R_8$ und $R_9$

- H und/oder
- Alkyl und/oder
- Aryl ist, oder

$R_8$ oder $R_9$ - C00H
sein kann,
wenn

Z = -C00H ist,
X = O und/oder N-H,
Y = $-CH_2-$ bis - $C_5H_{10}$-

Z = 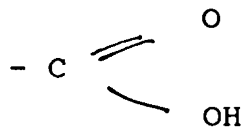 und/oder

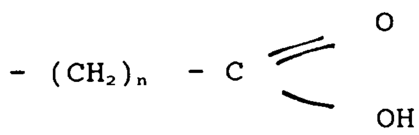 und/oder

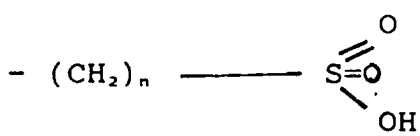 und/oder

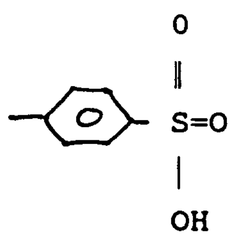 und/oder

eine saure Phosphorsäureester-Gruppe ist und
n = 1-18 ist und
a + b die relativen Mengen der jeweiligen Monomere in der Formel im Bereich von 5 : 95 bis 99 : 1 darstellen,

und $(An)^-$ = Chlorid und/oder Bromid und/oder Jodid und/oder $HS0_4^-$ und/oder $CH_3SO_4^-$ und/oder Nitrit ist.

**7.** Wäßrige Suspension nach Anspruch 6,
dadurch gekennzeichnet,

daß die erfindungsgemäßen Polyelektrolyten Verbindungen gemäß der folgenden Formel sind:

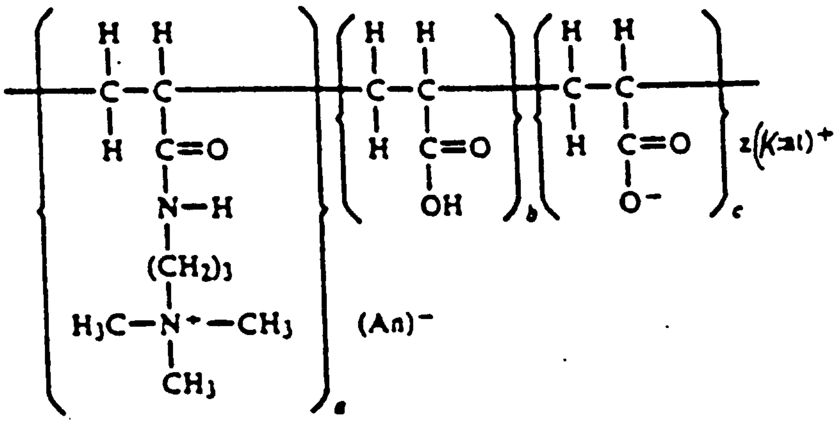

$$z : \frac{c}{\text{Wertigkeit von } (Kat)^{+}},$$

und wenn c = 0, dann z = 0,
und wobei

$(Kat)^+$ = Alkali- und/oder Erdalkali- und/oder Erdmetallkationen und/oder Amine und/oder Alkanolamine und/oder quaternäre Ammonium-Kationen,

$(An)^-$ = Chlorid, Bromid, Jodid, $HS0_4^-$, $CH_3SO_4^-$ und/oder Nitrit, und wobei a + b + c in einem der nachfolgenden Verhältnisse vorliegt:

| amphoter anionisch | amphoter | amphoter kationisch |
|---|---|---|
| a = 49-47 Mol.% | a = 50 Mol.% | a = 51-80 Mol.% |
| b + c = 51-53 Mol.% | b+c = 50 Mol.% | b + c = 49-20 Mol.% |

**8.** Wäßrige Suspension nach einem oder mehreren der vorhergehenden Ansprüche, dadurch gekennzeichnet,

daß der Substituent Z durch Alkali- und/oder Erdalkalimetallkationen teilneutralisiert ist, wobei der Neutralisationsgrad im Bereich von 1 - 99 Mol.% liegt.

**9.** Wäßrige Suspension nach einem oder mehreren der vorhergehenden Ansprüche, dadurch gekennzeichnet,

daß der Neutralisationsgrad von Z mit Alkalimetallkationen im Bereich von 1 - 25 Mol.% liegt.

**10.** Wäßrige Suspension nach einem oder mehreren der vorhergehenden Ansprüche, dadurch gekennzeichnet,

daß Z vollneutralisiert ist, wenn das Kation aus 2-wertigen Kationen, 3-wertigen Kationen, $NH_4^+$, primären, sekundären oder tertiären Aminen oder quartären Ammoniumionen ausgewählt wird.

**11.** Wäßrige Suspension nach einem oder mehreren der vorhergehenden Ansprüche, dadurch gekennzeichnet,

daß der Substiuent Z nicht neutralisiert vorliegt.

**12.** Wäßrige Suspension nach einem oder mehreren der vorhergehenden Ansprüche,
dadurch gekennzeichnet,

daß das Molverhältnis der anionischen Ladung zur kationischen Ladung im Bereich von 55 : 45 bis 51 : 49 liegt.

**13.** Wäßrige Suspension nach einem oder mehreren der vorhergehenden Ansprüche,
dadurch gekennzeichnet,

daß der Neutralisationsgrad der anionischen Komponente aller Polyelektrolyten mit Ausnahme der rein kationischen bei Neutralisation mit Erdalkalikationen 0,1 - 100 Mol.% beträgt.

**14.** Wäßrige Suspension nach einem oder mehreren der vorhergehenden Ansprüche,
dadurch gekennzeichnet,

daß der kationische Polyelektrolyt eine oder mehrere Verbindungen aus der Gruppe der folgenden Verbindungen gemäß der folgenden allgemeinen Formel ist

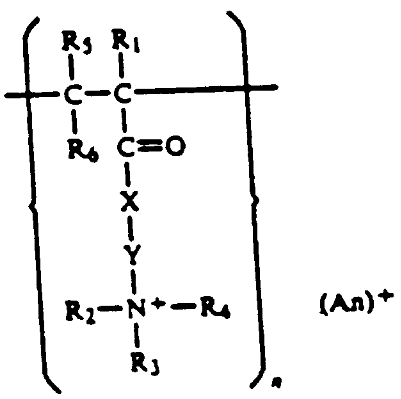

wobei $R_1$ , $R_5$ und $R_6$ =

- H und/oder $R_1$ bis $R_6$ = Alkyl und/oder
- Aryl,

wobei $R_5$ auch

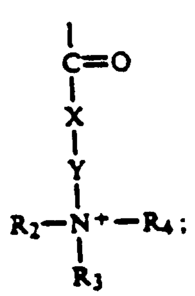

X = 0 und/oder N-H

Y = $-CH_2-$ bis - $C_5H_{10}$.
n = 20 bis 3000

und $(An)^-$ = Chlorid und/oder Bromid und/oder Jodid und/oder $HSO_4^-$ und/oder $CH_3SO_4^-$ und/oder Nitrit ist.

**15.** Wäßrige Suspension nach einem oder mehreren der vorhergehenden Ansprüche, dadurch gekennzeichnet,

daß der anionische teilneutralisierte Polyelektrolyt eine oder mehrere der Verbindungen aus der Gruppe der folgenden Verbindungen gemäß der folgenden allgemeinen Formel ist

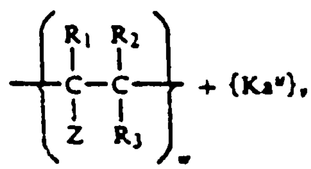

Z =
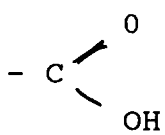
und/oder
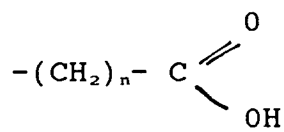
und/oder

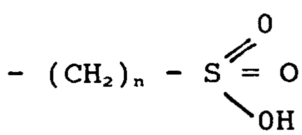
und/oder

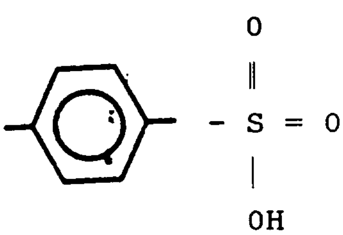

eine saure Phosphorsäureester-Gruppe

$R_1$ = - H oder - $CH_3$

$R_2$ und $R_3$ =
- H und/oder
- Alkyl und/oder
- Aryl

wobei $R_2$ oder $R_3$ auch Z sein kann, wenn

Z = - 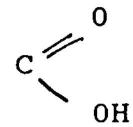

u = +I und/oder +II und/oder +III
Ka = Alkali- und/oder Erdalkali und/oder Erdmetallion w = 59 bis 95 Mol.% pro Anzahl Z im Monomer
v = 5 bis 41 Mol.% geteilt durch u
n = 1 - 12 ist.

**16.** Wäßrige Suspension nach einem oder mehreren der vorhergehenden Ansprüche,
dadurch gekennzeichnet,

daß beim anionischen Polyelektrolyten und/oder beim amphoteren anionischen Polyelektrolyten 1 bis 70 Mol.% der Säuregruppen neutralisiert sind, wobei die spezifische Viskosität "Eta" des teilneutralisierten anionischen Polyelektrolyten und/oder des amphoteren anionischen Polyelektrolyten in der Mischung mit dem kationischen und/oder dem amphoteren kationischen Polyelektrolyten, gemessen in der vollen Salzform zwischen 0,2 und 1,0 liegt und
der Polymerisationsgrad des kationischen Polyelektrolyten und/oder des amphoteren kationischen Polyelektrolyten in der Mischung mit dem teilneutralisierten anionischen Polyelektrolyten und/oder dem amphoteren anionischen teilneutralisierten Polyelektrolyten, gemessen über die Grenzviskosität, im Bereich von 5 ml/g bis 50 ml/g liegt und die Grenzviskosität der in der wäßrigen Suspension verwendeten kationischen und/oder amphoteren kationischen Polyelektrolyten im Bereich zwischen 9,2 ml/g und 48,5 ml/g liegt.

**17.** Wäßrige Suspension nach einem oder mehreren der vorhergehenden Ansprüche,
dadurch gekennzeichnet,

daß der Polymerisationsgrad der amphoteren anionischen und der amphoteren neutralen und der amphoteren kationischen Polyelektrolyten, gemessen über die Viskosität, im Bereich von 5 mPa.s bis 150 mPa.s liegt.

**18.** Wäßrige Suspension nach einem oder mehreren der vorhergehenden Ansprüche,
dadurch gekennzeichnet,

daß die wäßrige Suspension aus 97,0 Gew.% bis 99,97 Gew.% Mineralien und/oder Füllstoffen und/oder Pigmenten und Wasser und 0,03 Gew.% - 3,0 Gew.% der erfindungsgemäßen amphoteren Polyelektrolyten bei einem Feststoffgehalt von 60 - 80 Gew.%, bezogen auf das trockene Mineral bzw. den trockenen Füllstoff bzw. das trockene Pigment, besteht.

**19.** Wäßrige Suspension nach einem oder mehreren der vorhergehenden Ansprüche,
dadurch gekennzeichnet,

daß sie besteht aus 97,0 Gew.% bis 99,89 Gew.% Mineralien und/oder Füllstoffen und/oder Pigmenten und Wasser und O,11 Gew.% - 3,0 Gew.% einer Mischung aus kationischem und/oder amphoterem kationischen und teilneutralisiertem anionischen und/oder teilneutralisiertem amphoteren anionischen Polyelektrolyt, bei einem Feststoffgehalt von 60 - 80 Gew.%, bezogen auf daß trockene Mineral bzw. den trockenen Füllstoff bzw. das trockene Pigment.

**20.** Wäßrige Suspension nach einem oder mehreren der vorhergehenden Ansprüche,
dadurch gekennzeichnet,

daß die Mineralien und/oder Füllstoffe und/oder Pigmente Elemente der zweiten und/oder der dritten Hauptgruppe und/oder der vierten Nebengruppe des Periodensystems der Elemente umfassen.

**21.** Wäßrige Suspension nach einem oder mehreren der vorhergehenden Ansprüche,

dadurch gekennzeichnet,

daß die Mineralien und/oder Füllstoffe und/oder Pigmente natürliches Calciumcarbonat, präzipitiertes Calciumcarbonat, Marmor, Kreide, Dolomit und/oder dolomithaltiges Calciumcarbonat sind.

**22.** Verfahren zur Herstellung einer wäßrigen Suspension nach einem oder mehreren der vorhergehenden Ansprüche, gekennzeichnet durch folgende Verfahrensschritte, ausgewählt aus der Gruppe von 1 :

a) eine wäßrige Suspension von Mineralien und/oder Füllstof fen und/oder Pigmenten wird zusammen mit einer Dispergier- und Mahlhilfsmittelmischung nach einem oder mehreren der vorhergehenden Ansprüche naßvermahlen, wobei

b) die erfindungsgemäßen amphoteren Polyelektrolyten vollständig vor der Vermahlung oder

c) ein Teil der erfindungsgemäßen amphoteren Polyelektrolyten vor der Vermahlung und

d) ein Teil der erfindungsgemäßen amphoteren Polyelektkrolyten während der Vermahlung und/oder

e) ein Teil der erfindungsgemäßen amphoteren Polyelektrolyten nach der Vermahlung hinzugegeben werden,

oder 2:

a) eine wäßrige Suspension von Mineralien und/oder Füllstof fen und/oder Pigmenten wird zusammen mit einer Dispergier- und Mahlhilfsmittelmischung nach einem oder mehreren der vorhergehenden Ansprüche naßvermahlen, wobei

b) ein Teil des teilneutralisierten anionischen und/oder teilneutralisierten amphoteren anionischen Polyelektrolyten vor der Vermahlung und

c) ein Teil des teilneutralisierten anionischen und/oder teilneutralisierten amphoteren anionischen Polyelektrolyten während der Vermahlung und/oder

d) ein Teil des teilneutralisierten anionischen und/oder teilneutralisierten amphoteren anionischen Polyelektrolyten nach der Vermahlung,

e) und der kationische und/oder amphotere kationische Polyelektrolyt vollständig vor der Vermahlung oder nur

f) ein Teil des kationischen und/oder amphoteren kationischen Polyelektrolyten vor der Vermahlung und

g) ein Teil des kationischen und/oder amphoteren kationischen Polyelektrolyten während der Vermahlung und/oder

h) ein Teil des kationischen und/oder amphoteren kationischen Polyelektrolyten nach der Vermahlung hinzugegeben werden.

**23.** Verwendung der wäßrigen Suspension von Mineralien und/oder Füllstoffen und/oder Pigmenten nach einem oder mehreren der vorhergehenden Ansprüche bei der Papierherstellung bzw. in der Papiererzeugung, zur Oberflächenbehandlung, Pigmentierung der Papieroberfläche in der Leimpresse der Papiermaschine und in der Papierstreicherei, im Vorstrich bzw. im Deckstrich bei der Papierstreicherei, im Holzschliff zur Störstoffbekämpfung, im Streichereiausschuß zur Störstoffbekämpfung, zur Pitchkontrolle, im Kreislaufwasser der Papiermaschine zur Erniedrigung des chemischen Sauerstoffbedarfs, in der Kläranlage zur Abwasserbehandlung, zur Vorflockung anionisch stabilisierter Pigment- und/oder Mineral- und/oder Füllstoff-Suspensionen in der Papiererzeugung oder zur Vorflockung, Immobilisierung von Streichfarben in der Streicherei.

## Claims

**1.** Aqueous suspension of minerals and/or fillers and/or pigments having a solids content $\geq$ 60 % by weight with respect to the dry mineral or the dry filler or the dry pigment, the mineral or the filler or the pigment being dispersed

with one or more dispersing agents,
characterized in that the dispersing agent contains

one or more amphoteric polyelectrolytes in which the number of the negative charges in the anionic monomer units is equal to the number of the positive charges in the cationic monomer units and which may optionally additionally contain neutral monomer units,
and/or
one or more amphoteric cationic polyelectrolytes in which the non-neutral monomer units carry predominantly positive charges,
and/or
one or more amphoteric anionic polyelectrolytes in which the non-neutral monomer units carry predominantly negative charges,
and/or
one or more partially neutralized anionic polyelectrolytes,
and/or
one or more partially neutralized amphoteric anionic polyelectrolytes in which the non-neutral monomer units carry predominantly negative charges,
wherein the amphoteric, the amphoteric cationic and the amphoteric anionic polyelectrolytes contain quaternary ammonium groups, and
wherein the filler and/or pigment and/or mineral particles carry a charge neutral or positive to the outside.

2. Aqueous suspension according to claim 1 characterized in that the suspension contains additionally one or more cationic polyelectrolytes.

3. Aqueous suspension according to claim 1 or 2, characterized in that the dispersing agent is a mixture of

(a) one or more cationic polyelectrolytes and/or one or more amphoteric cationic polyelectrolytes in which the non-neutral monomer units carry predominantly positive charges, and
(b) one or more partially neutralized anionic polyelectrolytes and/or one or more partially neutralized amphoteric anionic polyelectrolytes in which the non-neutral monomer units carry predominantly negative charges, the cationic polyelectrolyte and/or the amphoteric cationic polyelectrolyte being present in an amount such that the dispersed mineral or filler or pigment particles carry positive charges.

4. Aqueous suspension according to one or more of the preceding claims, characterized in that

the amphoteric anionic and the cationic and the amphoteric cationic polyelectrolyte carry the functional group generating the positive charge in a substituent of the ethylenic main chain and contains quaternary ammonium groups, carboxyl groups and/or sulfonic acid groups and/or acidic phosphoric-acid-ester-containing groups and the substituent carrying the cationic charge is bound to the main chain via

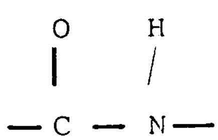

or

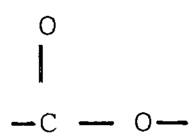

5. Aqueous suspension according to one or more of the preceding claims, characterized in that

the anionic partially neutralized and the amphoteric anionic partially neutralized polyelectrolyte carry each carboxyl groups and the anionic partially neutralized polyelectrolyte being a homopolymeric and/or a copolymeric electrolyte.

6. Aqueous suspension according to one or more of the preceding claims, characterized in that

the amphoteric anionic and the amphoteric and the amphoteric cationic polyelectrolyte is one or more compounds from the group of the following compounds according to the following general formula:

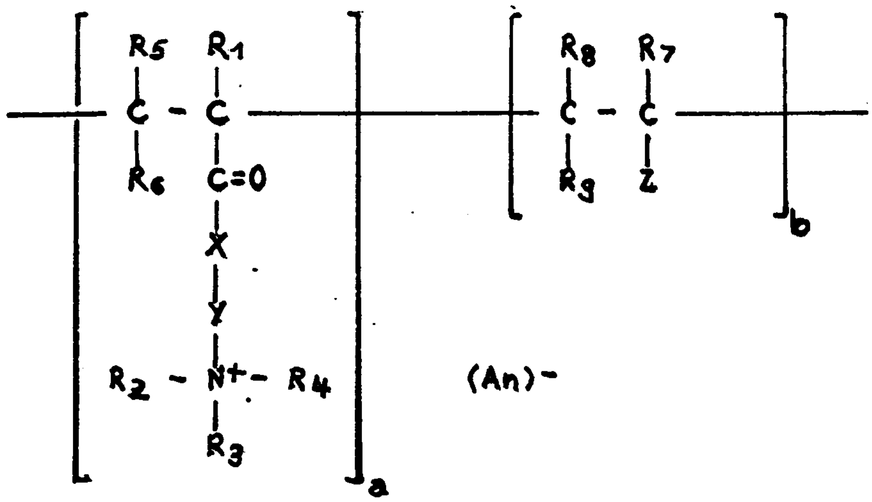

wherein $R_1$, $R_5$, $R_6$ and $R_7$ is H, and/or $R_1$ to $R_7$ may be an alkyl and/or

- acryl and/or
- aryl,
- $R_8$ and $R_9$ = H and/or
- alkyl and/or
- aryl or

$R_8$ or $R_9$ may also be -COOH
if

Z = -COOH
X= O and/or N-H,
Y= $-CH_2-$ to $C_5H_{10}-$;

Z =

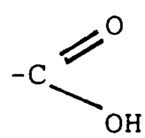

and/or

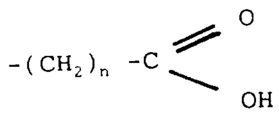 and /or

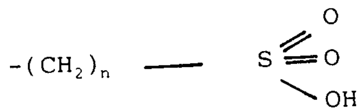 and/or

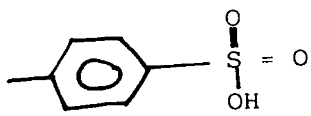 and/or

an acidic phosphoric acid ester group and n=1-18,

a + b are the relative amounts of the monomers in the formula in the range of 5 : 95 to 99 : 1, and

$(An)^-$ may be = chloride and/or bromide and/or iodide and/or $HSO_4^-$ and or/or $CH_3SO_4^-$ and/or nitrite.

**7.** Aqueous suspension according to claim 6 characterized in that

the polyelectrolytes according to the invention are compounds of the following formula:

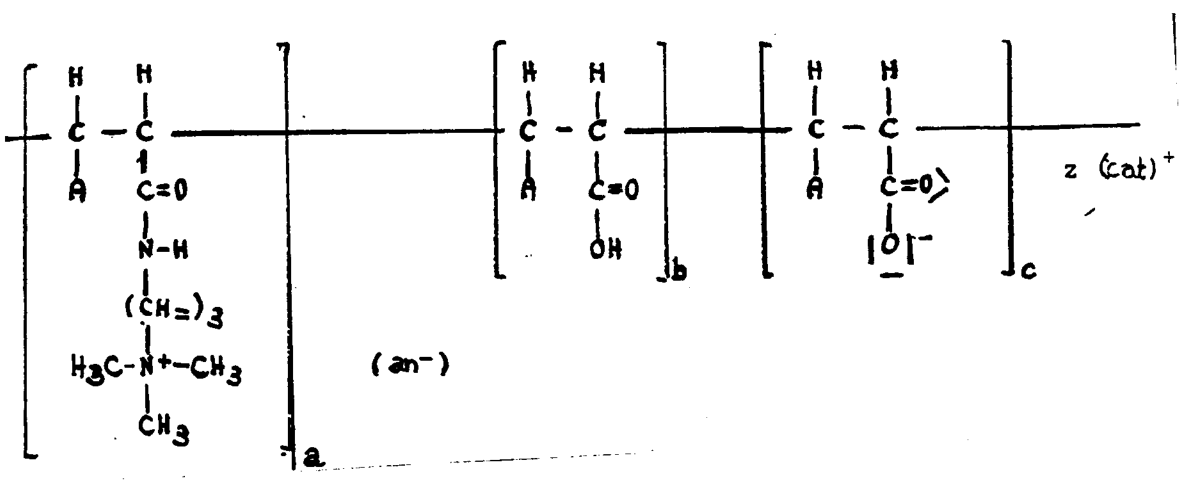

$$Z = \frac{C}{\text{valency of (cat)}^+}$$

and if c = 0, then z = 0,
and

$(cat)^+$ = alkalines and/or alkaline earth and/or earth metal cations and/or amines and/or alkanol amines and/or quaternary ammonium cations,

$(an)^-$ = chloride, bromide, iodide, $HSO_4^-$, $CH_3SO_4^-$and/or nitrite,

and in the polyelectrolytes according to the invention a and b and c are present in the following ratios:

| amphoteric anionic | amphoteric | amphoteric cationic |
|---|---|---|
| a = 49-47 mole%<br>b+c = 51-53 mole% | a= 50 mole%<br>b+c= 50 mole% | a=51-80 mole%<br>b+c=49-20 mole% |

**8.** Aqueous suspension according to one or more of the preceding claims characterized in that

substituent Z is partially neutralized by alkaline and/or alkaline earth metal cations, wherein the neutralization degree is in a range from 1-99 mole%.

**9.** Aqueous suspension according to one or more of the preceding claims characterized in that

the neutralization degree of Z with alkaline metal cations is in a range of 1-25 mole%.

**10.** Aqueous suspension according to one or more of the preceding claims characterized in that

Z is completely neutralized provided the cation is selected from 2 valent cations, 3 valent cations, $NH_4^+$, primary, secondary or tertiary amines or quaternary ammonium ions.

**11.** Aqueous suspension according to one or more of the preceding claims characterized in that

substituent Z is not neutralized.

**12.** Aqueous suspension according to one or more of the preceding claims characterized in that

the molar ration of the anionic charge with respect to the cationic charge is in a range of 55 : 45 to 51 : 49.

**13.** Aqueous suspension according to one or more of the preceding claims characterized in that

the neutralization degree of the anionic component of all polyelectrolytes with the exception of the pure cationic polyelectrolytes is 0.1 - 100 mole% when neutralized with alkaline earth cations.

**14.** Aqueous suspension according to one or more of the preceding claims characterized in that

the cationic polyelectrolyte is one or more compounds of the group of the following compounds according to the following general formula:

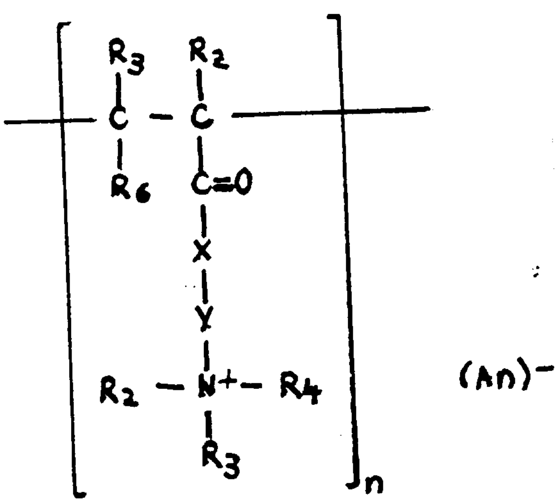

where $R_1$, $R_5$ and $R_6$ =

- H and/or $R_1$ to $R_6$ = alkyl and/or
- aryl,

and $R_5$ may also be

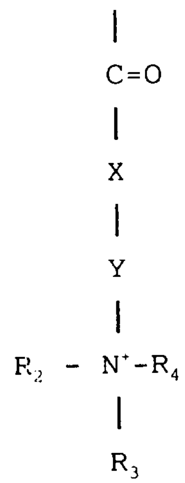

X = 0 and/or N-H
Y = $-CH_2-$ to $-C_5H_{10-}$
n = 20 to 3000

and $(An)_-$ = chloride and/or bromide and/or iodide and/or $HSO_4^-$ and/or $CH_3SO_4^-$ and/or nitrite.

**15.** Aqueous suspension according to one or more of the

preceding claims characterized in that the anionic partially neutralized polyelectrolyte is one or more of the compounds of the group of the following compounds according to the following general formula

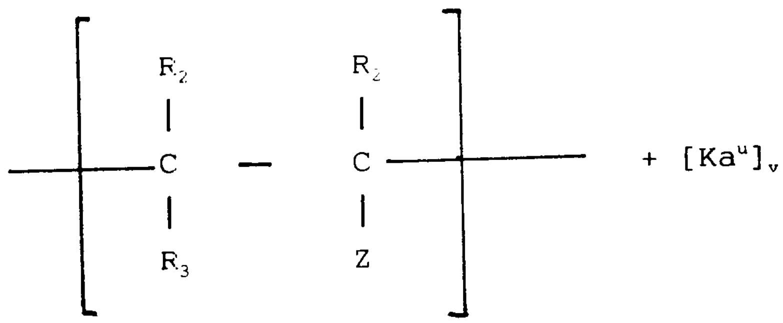

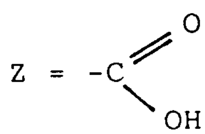

and/or

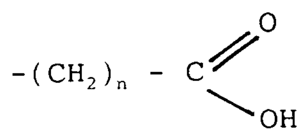

and/or

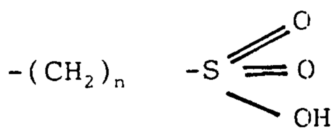

and/or

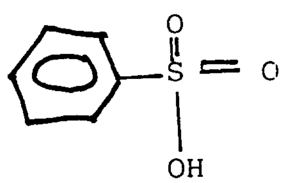

and/or

an acidic phosphoric acid ester group

$R_1$ = -H or $-CH_3$

$R_2$ and $R_3$ =

- H and/or
- alkyl and/or
- aryl

and where $R_2$ or $R_3$ may also be Z when

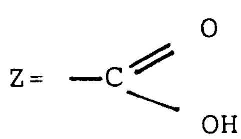

u= +I and/or +II and/or +III
Ka = alkaline and/or alkaline earth and/or earth metal ion
w = 59 to 95 mole% per number Z in monomer
v = 5 to 41 mole% divided by u
n = 1-12.

**16.** Aqueous suspension according to one or more of the preceding claims characterized in that

in the anionic polyelectrolyte and/or in the amphoteric anionic polyelectrolyte 1 to 70 mole% of the acid groups are neutralized, the specific viscosity "Eta" of the partially neutralized anionic polyelectrolyte and/or of the amphoteric anionic polyelectrolyte in the mixture with the cationic and/or the amphoteric cationic polyelectrolyte, measured in the full salt form, lying between 0.2 and 1.0 and the polymerization degree of the cationic polyelectrolyte and/or the amphoteric cationic polyelectrolyte in mixture with the partially neutralized anionic polyelectrolyte and/or the amphoteric anionic partially neutralized polyelectrolyte, measured via the limiting viscosity, lies in the range of 5 ml/g to 50 ml/g and the limiting viscosity of the cationic and/or amphoteric cationic polyelectrolytes used in the aqueous suspension lies in the range between 9.2 ml/g and 48.5 ml/g.

**17.** Aqueous suspension according to one or more of the preceding claims characterized in that

the polymerization degree of the amphoteric anionic and the amphoteric neutral and the amphoteric cationic polyelectrolytes, measured via the viscosity, is in a range of 5 mPa.s to 150 mPa.s.

**18.** Aqueous suspension according to one or more of the preceding claims characterized in that

the aqueous suspension contains 97.0 percent per weight to 99.97 percent per weight of minerals and/or fillers and/or pigments or water and 0.03 percent per weight to 3.0 percent per weight of the amphoteric polyelectrolytes according to the invention with a solids content of 60-80 percent per weight, with respect to the dry mineral and the dry filler and the dry pigment, respectively.

**19.** Aqueous suspension according to one or more of the preceding claims characterized in that

the aqueous suspension contains 97.0 percent per weight to 99.89 percent per weight of minerals and/or fillers and/or pigments and water and 0.11 percent per weight to 3.0 percent per weight of a mixture of cationic and/or amphoteric cationic and partially neutralized anionic and/or partially neutralized amphoteric anionic polyelectrolytes with a solids content of 60-80 percent per weight, with respect to the dry mineral and the dry filler and the dry pigment, respectively.

**20.** Aqueous suspension according to one or more of the preceding claims characterized in that

the minerals and/or fillers and/or pigments comprise elements of the second and/or third main group and/or of the forth subgroup of the periodic system of the elements.

**21.** Aqueous suspension according to one or more of the preceding claims characterized in that

the minerals and/or fillers and/of pigments are natural calcium carbonate, precipitated calcium carbonate and/or marble, chalk, dolomite and/or dolomite-containing calcium carbonate.

**22.** A method for obtaining an aqueous suspension according to or more of the preceding claims characterized by the following method steps, selected from group 1:

(a) an aqeous suspension of minerals and/or fillers and/or pigments is wet ground together with a dispersing and grinding agent mixture according to one or more of the preceding claims, wherein

(b) the amphoteric polyelectrolytes according to the invention being added completely before the grinding or

(c) a part of the amphoteric polyelectrolytes according to the invention being added before the grinding and

(d) a part of the amphoteric polyelectrolytes according to the invention being added during the grinding and/or

(e) a part of the amphoteric polyelectrolytes according to the invention being added after the grinding;

or 2:

(a) an aqueous suspension of minerals and/or fillers and/or pigments is wet ground together with a dispersing

and grinding agent mixture according to one or more of the preceding claims, wherein

(b) a part of the partially neutralized anionic and/or partially neutralized amphoteric anionic polyelectrolytes is added before grinding and

(c) a part of the partially neutralized anionic and/or partially neutralized amphoteric anionic polyelectrolytes is added during grinding and/or

(d) a part of the partially neutralized anionic and/or partially neutralized amphoteric anionic polyelectrolytes is added after grinding

(e) and the cationic and/or amphoteric cationic polyelectrolytes are completely added before grinding or only

(f) a part of the cationic and/or amphoteric cationic polyelectrolytes is added before grinding and

(g) a part of the cationic and/or amphoteric cationic polyelectrolytes is added during grinding and/or

(h) a part of the cationic and/or amphoteric cationic polyelectrolytes is added after grinding.

**23.** Use of the aqueous suspension of minerals and/or fillers and/or pigments according to one or more of the preceding claims in paper manufacture or paper production, for surface treatment, pigmenting of the paper surface in the size press of the papermaking machine and in the paper coating, in the preliminary coat or in the top coat in the paper coating, in the wood pulp for impurity control for pitch control, in the circulation water of the papermaking machine for chemical oxygen demand reduction, in the purification plant for waste water treatment, for prefloocu-lating anionically stabilized pigment and/or mineral and/or filler suspensions in paper production or for pre-occulat-ing, immobilization of coating compounds in the coating apparatus.

## Revendications

**1.** Suspension aqueuse de minéraux et/ou charges et/ou pigments avec une teneur en matières solides ≥ 60 % en poids, rapportés au minéral sec et/ou à la charge sèche et/ou au pigment sec, le minéral et/ou la charge et/ou le pigment étant dispersés avec un dispersant, **caractérisée en ce** que le dispersant contient

un ou plusieurs polyampholytes dans les quels le nombre des charges négatives dans les motifs monomères anioniques est égal au nombre des charges positives dans les motifs monomères cationiques, ou qui peuvent contenir en supplément des motifs monomères neutres
et/ou
un ou plusieurs polyampholytes cationiques dans les quels les motifs monomères non neutres portent essentiellement des charges positives
et/ou un ou plusieurs polyampholyte anioniques dans lesquels les motifs monomères non neutres portent essentiellement des charges négatives
et/ou
un ou plusieurs polyélectrolytes anioniques partiellement neutralisés,
et/ou
un ou plusieurs polyampholytes anioniques partiellement neutralisés dans les quels les motifs monomères non neutres portent essentiellement des charges négatives, les polyampholytes, les polyampholytes cationiques et les polyampholytes anioniques contenant des groupes d'ammonium quaternaire, et
les particules de charges et/ou de pigments et/ou de minéraux devant porter une charge neutre ou positive vis-à-vis de l'extérieur.

**2.** Suspension aqueuse selon la revendication 1, caractérisée en ce que la suspension contient, en supplément, un ou plusieurs polyélectrolytes cationiques.

**3.** Suspension aqueuse selon la revendication 1 ou la revendication 2, caractérisée en ce que le dispersant est un mélange

(a) d'un ou de plusieurs polyélectrolytes cationiques et/ou d'un ou de plusieurs polyampholytes cationiques dans les quels les motifs monomères non neutres portent essentiellement des charges positives, et

(b) d'un ou de plusieurs polyélectrolytes anioniques partiellement neutralisés et/ou d'un ou de plusieurs polyampholytes anioniques partiellement neutralisés dans les quels les motifs monomères non neutres portent essentiellement des charges négatives, le polyélectrolyte cationique et/ou le polyampholyte cationique étant présents en une quantité telle que les particules de minéraux et/ou charges et/ou pigments dispersées portent des charges positives.

**4.** Suspension aqueuse selon l'une ou plusieurs des revendications précédentes, caractérisée en ce que le polyampholyte anionique et le polyampholyte cationique et le polyélectrolyte cationique portent le groupe fonctionnel générateur des charges positives dans un substituant de la chaîne principale éthylénique, qu'ils contiennent des groupes d'ammonium quaternaire, des groupes carboxyle et/ou des groupes d'acide sulfonique et/ou des groupes acides contenant de l'ester phosphorique, et que le substituant portant la charge cationique est lié à la chaîne principale par l'intermédiaire de

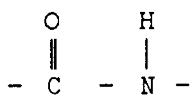 ou 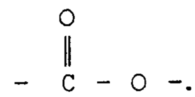

**5.** Suspension aqueuse selon l'une ou plusieurs des revendications précédentes, caractérisée en ce que le polyélectrolyte anionique partiellement neutralisé et le polyampholyte anionique partiellement neutralisé portent respectivement des groupes carboxyle et que le polyélectrolyte anionique partiellement neutralisé est un polyélectrolyte homo- et/ou copolymère.

**6.** Suspension aqueuse selon l'une ou plusieurs des revendications précédentes, caractérisée en ce que le polyampholyte anionique et le polyélectrolyte amphotère et le polyampholyte cationique sont constitués par un ou plusieurs composés appartenant au groupe des composés suivants d'après la formule générale ci-après :

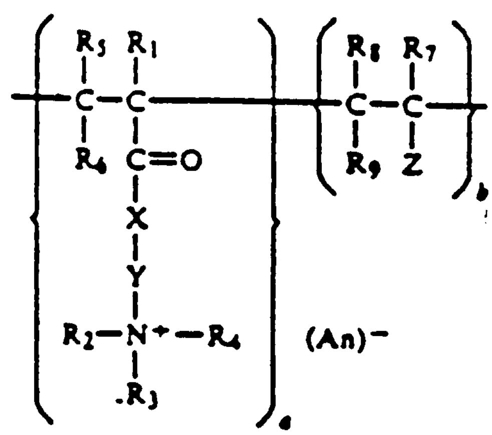

où $R_1$, $R_5$, $R_6$ et $R_7$ est H
et/ou $R_1$ à $R_7$ est

- un alkyle et/ou
- un aryle,

$R_8$ et $R_9$ est

- H et/ou
- alkyle et/ou
- aryle, ou

$R_8$ ou $R_9$ peut être - COOH

si

Z = -COOH,
X = O et/ou N-H,
Y = $-CH_2-$ à $-C_5H_{10}-$

Z = 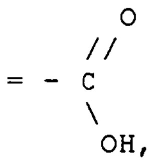 et/ou

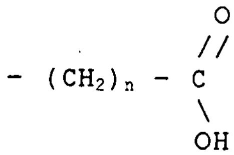 et/ou

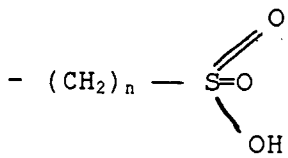 et/ou

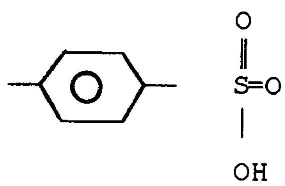 et/ou

un groupe acide d'ester phosphorique et
n = 1 - 18, et
a + b représentent les quantités relatives des monomères respectifs dans la formule dans la plage de 5 : 95 à 99 : 1,

et $(An)^-$ = Chlorure et/ou bromure et/ou iodure et/ou $HSO_4^-$ et/ou $CH_3SO_4^-$ et/ou nitrite.

7. Suspension aqueuse selon la revendication 6, caractérisée en ce que les polyélectrolytes selon l'invention sont des composés de la formule suivante :

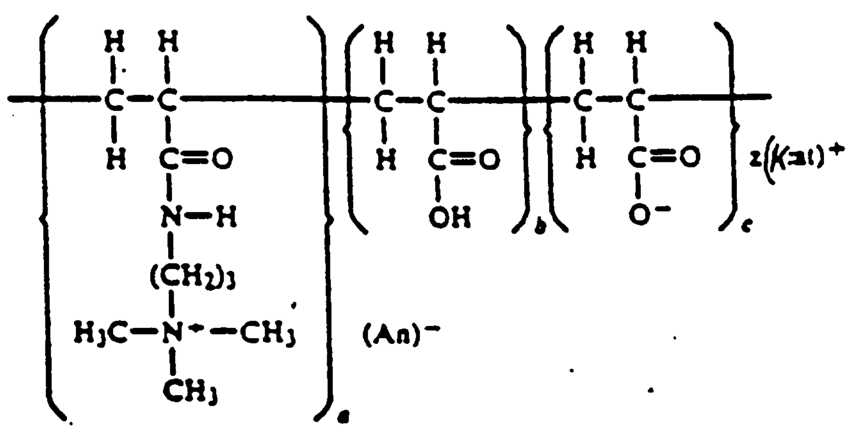

$$Z : \frac{c}{\text{Valence de (Kat)}^{+}},$$

et si c = 0, z = 0 et où

$(Kat)^+$ = Cations alcalins et/ou alcalino-terreux et/ou de métaux terreux et/ou amines et/ou amino-alcools et/ou cations d'ammonium quaternaire,

$(An)^-$ = Chlorure, bromure, iodure, $HSO_4^-$, $CH_3SO_4^-$ et/ou nitrite, et où a + b + c se présente dans l'un des rapports suivants :

| amphotère anionique | amphotère | amphotère cationique |
|---|---|---|
| a = 49 à 47 % mol.<br>b+c = 51 à 53 % mol. | a = 50 % mol.<br>c+b = 50 % mol. | a = 51 à 80 % mol.<br>b+c = 49 à 20 % mol. |

**8.** Suspension aqueuse selon l'une ou plusieurs des revendications précédentes, caractérisée en ce que le substituant Z est partiellement neutralisé par des cations alcalins et/ou alcalino-terreux, le degré de neutralisation étant compris entre 1 et 99 % mol..

**9.** Suspension aqueuse selon l'une ou plusieurs des revendications précédentes, caractérisée en ce que le degré de neutralisation de Z avec des cations de métaux alcalins est compris entre 1 et 25 % mol..

**10.** Suspension aqueuse selon l'une ou plusieurs des revendications précédentes, caractérisée en ce que Z est complètement neutralisé si le cation est sélectionné parmi des cations bivalents, des cations trivalents, $NH_4^+$, des amines primaires, secondaires ou tertiaires ou des ions d'ammonium quaternaires.

**11.** Suspension aqueuse selon l'une ou plusieurs des revendications précédentes, caractérisée en ce que le substituant Z n'est pas neutralisé.

**12.** Suspension aqueuse selon l'une ou plusieurs des revendications précédentes, caractérisée en ce que le rapport molaire entre la charge anionique et la charge cationique est compris entre 55 : 45 et 51 : 49.

**13.** Suspension aqueuse selon l'une ou plusieurs des revendications précédentes, caractérisée en ce que le degré de neutralisation de la composante anionique de tous les polyélectrolytes, à l'exception des polyélectrolytes purement cationiques avec neutralisation avec des ions alcalino-terreux, est de 0,1 à 100 % mol.

**14.** Suspension aqueuse selon l'une ou plusieurs des revendications précédentes, caractérisée en ce que le polyélectrolyte cationique est constitué par un ou plusieurs composés appartenant au groupe des composés suivants d'après la formule générale ci-après :

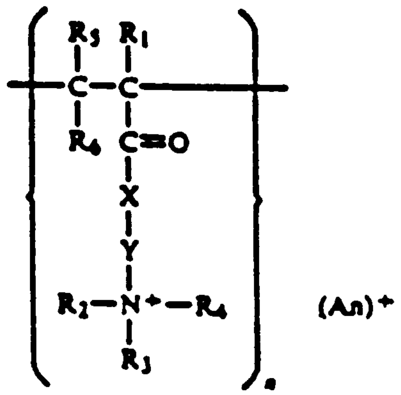

où $R_1$, $R_5$ et $R_6$ =

- H et/ou $R_1$ à $R_6$ = alkyle et/ou
- aryle,

où $R_5$ est également

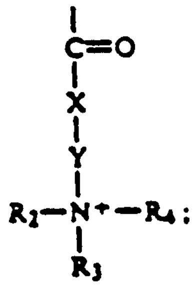

X = O et/ou N-H
Y = $-CH_2$ à $-CH_5H_{10}-$
n = 20 à 3000

et $(An)^-$ = Chlorure et/ou bromure et/ou iodure et/ou $HSO_4^-$ et/ou $CH_3SO_4^-$ et/ou nitrite.

**15.** Suspension aqueuse selon l'une ou plusieurs des revendications précédentes, caractérisée en ce que le polyélectrolyte anionique partiellement neutralisé est constitué par un ou plusieurs composés appartenant au groupe des composés suivants d'après la formule générale ci-après :

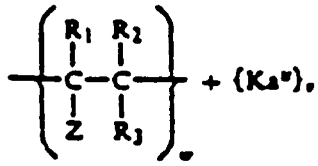

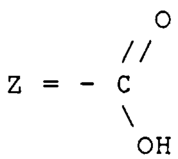 et/ou 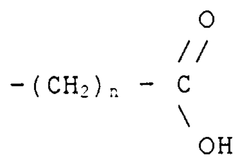 et/ou

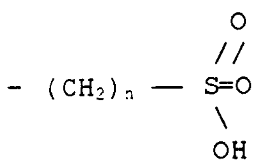 et/ou

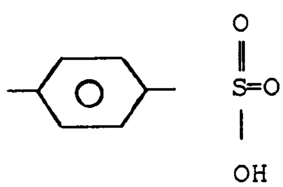 et/ou

un groupe acide d'ester phosphorique

$R_1$ = - H ou - $CH_3$

$R_2$ et $R_3$ =

- H et/ou
- alkyle et/ou
- aryle

où $R_2$ ou $R_3$ peut également être Z si

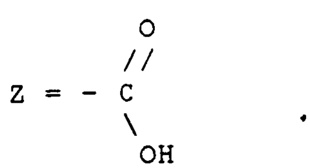

u = +I et/ou +II et/ou +III
Ka = ion alcalin et/ou alcalino-terreux et/ou de métal terreux w = 59 à 95 % mol. par nombre de Z dans le monomère
v = 5 à 41 % mol. divisé par u
n = 1 à 12.

**16.** Suspension aqueuse selon l'une ou plusieurs des revendications précédentes, caractérisée en ce que dans le

polyélectrolyte anionique et/ou dans le polyampholyte anionique, 1 à 70 % mol. des groupes d'acide sont neutralisés, la viscosité spécifique « êta » du polyélectrolyte anionique partiellement neutralisé et/ou du polyampholyte anionique en mélange avec le polyélectrolyte cationique et/ou le polyampholyte cationique, mesurée sous la forme entièrement saline se situe entre 0,2 et 1,0, et que le degré de polymérisation du polyélectrolyte cationique et/ou du polyampholyte cationique en mélange avec le polyélectrolyte anionique partiellement neutralisé et/ou le polyampholyte anionique partiellement neutralisé, mesuré par la viscosité limite, est compris entre 5 ml/g et 50 ml/g, et que la viscosité limite du polyélectrolyte cationique et/ou du polyampholyte cationique utilisés dans la suspension aqueuse est comprise entre 9,2 ml/g et 48,5 ml/g.

**17.** Suspension aqueuse selon l'une ou plusieurs des revendications précédentes, caractérisée en ce que le degré de polymérisation des polyampholytes anioniques et des polyampholytes et des polyampholytes cationiques, mesuré par la viscosité, est compris entre 5 mPa.s et 150 mPa.s.

**18.** Suspension aqueuse selon l'une ou plusieurs des revendications précédentes, caractérisée en ce que la suspension aqueuse se compose de 97,0 % en poids à 99,97 % en poids de minéraux et/ou charges et/ou pigments et d'eau et de 0,03 % en poids à 3,0 % en poids des polyampholytes selon l'invention, avec une teneur en matières solides de 60 à 80 % en poids, rapportée au minéral sec et/ou à la charge sèche et/ou au pigment sec.

**19.** Suspension aqueuse selon l'une ou plusieurs des revendications précédentes, caractérisée en ce qu'elle se compose de 97,0 % en poids à 99,89 % en poids de minéraux et/ou charges et/ou pigments et d'eau et de 0,11 % en poids à 3,0 % en poids d'un mélange de polyélectrolyte cationique et/ou de polyampholyte cationique et de polyélectrolyte anionique partiellement neutralisé et/ou de polyampholyte anionique partiellement neutralisé, avec une teneur en matières solides de 60 à 80 % en poids, rapportée au minéral sec et/ou à la charge sèche et/ou au pigment sec.

**20.** Suspension aqueuse selon l'une ou plusieurs des revendications précédentes, caractérisée en ce que les minéraux et/ou charges et/ou pigments comprennent des éléments du deuxième et/ou troisième groupes principaux et/ou du quatrième groupe secondaire de la classification périodique des éléments.

**21.** Suspension aqueuse selon l'une ou plusieurs des revendications précédentes, caractérisée en ce que les minéraux et/ou charges et/ou pigments sont du carbonate de calcium naturel, du carbonate de calcium précipité, du marbre, de la craie, de la dolomite et/ou du carbonate de calcium contenant de la dolomite.

**22.** Procédé de fabrication d'une suspension aqueuse selon l'une ou plusieurs des revendications précédentes, caractérisé par les opérations de base suivantes sélectionnées dans le groupe de 1 :

a) broyer à l'état humide une suspension aqueuse de minéraux et/ou charges et/ou pigments conjointement avec un mélange de dispersants et d'agents de broyage selon l'une ou plusieurs des revendications précédentes, en ajoutant
b) la totalité des polyampholytes selon l'invention avant le broyage ou
c) une partie des polyampholytes selon l'invention avant le broyage et
d) une partie des polyampholytes selon l'invention pendant le broyage et/ou
e) une partie des polyampholytes selon l'invention après le broyage,

ou 2 :

a) broyer à l'état humide une suspension aqueuse de minéraux et/ou charges et/ou pigments conjointement avec un mélange de dispersants et d'agents de broyage selon l'une ou plusieurs des revendications précédentes, en ajoutant
b) une partie du polyélectrolyte anionique partiellement neutralisé et/ou du polyampholyte anionique partiellement neutralisé avant le broyage et
c) une partie du polyélectrolyte anionique partiellement neutralisé et/ou du polyampholyte anionique partiellement neutralisé pendant le broyage et/ou
d) une partie du polyélectrolyte anionique partiellement neutralisé et/ou du polyampholyte anionique partiellement neutralisé après le broyage,
e) et la totalité du polyélectrolyte cationique et/ou du polyampholyte cationique avant le broyage ou seulement
f) une partie du polyélectrolyte cationique et/ou du polyampholyte cationique avant le broyage et
g) une partie du polyélectrolyte cationique et/ou du polyampholyte cationique pendant le broyage et

h) une partie du polyélectrolyte cationique et/ou du polyampholyte cationique après le broyage.

**23.** Utilisation de la suspension aqueuse de minéraux et/ou charges et/ou pigments selon l'une ou plusieurs des revendications précédentes lors de la fabrication de papier ou dans la production de papier, pour le traitement de surface, la pigmentation de la surface du papier dans la presse encolleuse de la machine à papier et dans l'atelier d'enduction du papier, dans la pâte mécanique pour la lutte contre des substances perturbatrices, dans le rebut d'enduction pour la lutte contre les substances perturbatrices, pour le contrôle pitch, dans l'eau de recyclage de la machine à papier pour la réduction des besoins chimiques en oxygène, dans l'installation de décantation pour le traitement des eaux usées, pour la floculation préliminaire de suspensions de pigments et/ou minéraux et/ou charges à stabilisation anionique dans la fabrication de papier ou pour la floculation préliminaire, l'immobilisation de couleurs d'enduction dans l'installation de couchage.